# EUROPEAN PATENT SPECIFICATION

(11) **EP 1 575 509 B1**
(45) Date of publication and mention of the grant of the patent: **26.10.2011**
(21) Application number: 03784738.1
(22) Date of filing: 12.05.2003
(51) Int. Cl.: C07K 16/24, C07K 16/30, C12N 15/12, C12N 15/63, C12N 5/10, G01N 33/53, G01N 33/574, A61K 39/395, C12P 21/08, C07K 16/00, A61P 35/00, A61P 35/04, A61K 31/00, C07K 16/28, A61K 47/48, C12N 15/13, C12N 5/20, G01N 33/577, G01N 33/68

(54) **EPHA2 AGONISTIC MONOCLONAL ANTIBODIES AND METHODS OF USE THEREOF**
EPHA2 AGONISTISCHE MONOKLONALE ANTIKÖRPER UND DEREN ANWENDUNGSVERFAHREN
ANTICORPS MONOCLONAUX AGONISTES DE LA EPHA2 ET UTILISATIONS DE CEUX-CI

(30) Priority: 10.05.2002 US 379368 P; 14.10.2002 US 418204 P; 03.04.2003 US 460358 P
(43) Date of publication of application: 21.09.2005
(73) Proprietor: Purdue Research Foundation, West Lafayette, IN 47906 (US); MEDIMMUNE, INC., Gaithersburg, MD 20878 (US)
(72) Inventor: KINCH, Michael, S., Laytonsville, MD 20882 (US); CARLES-KINCH, Kelly, Laytonsville, MD 20882 (US); STEWART, Jane, C., West Lafayette, IN 47906 (US)
(74) Representative: Chapman, Paul Gilmour
(86) International application number: PCT/US2003/015046
(87) International publication number: WO 2004/014292

(56) References cited:
- WO-A-02/30465
- WO-A-03/094859
- WO-A-2005/051307
- WO-A1-01/12172
- WO-A2-01/12840
- US-A- 5 747 033
- US-A- 5 766 886
- US-A- 5 795 734
- US-A- 5 955 291
- US-A- 6 063 903
- US-B1- 6 555 321
- US-B1- 6 927 203
- US-B2- 6 696 550
- ZELINSKI D P ET AL: "EPHA2 OVEREXPRESSION CAUSES TUMORIGENESIS OF MAMMARY EPITHELIAL CELLS" CANCER RESEARCH, AMERICAN ASSOCIATION FOR CANCER RESEARCH, BALTIMORE, MD, US, vol. 61, no. 5, 1 March 2001 (2001-03-01), pages 2301-2306, XP001182247 ISSN: 0008-5472
- LANDEN CHARLES N JR ET AL: "Efficacy and antivascular effects of EphA2 reduction with an agonistic antibody in ovarian cancer." JOURNAL OF THE NATIONAL CANCER INSTITUTE 1 NOV 2006, vol. 98, no. 21, 1 November 2006 (2006-11-01), pages 1558-1570, XP002421774 ISSN: 1460-2105
- BRANTLEY D M ET AL: "Soluble Eph A receptors inhibit tumor angiogenesis and progression in vivo" ONCOGENE, BASINGSTOKE, HANTS, GB, vol. 21, no. 45, 10 October 2002 (2002-10-10), pages 7011-7026, XP002992332 ISSN: 0950-9232
- ZANTEK N D ET AL: "E-CADHERIN REGULATES THE FUNCTION OF THE EPHA2 RECEPTOR TYROSINE KINASE" CELL GROWTH AND DIFFERENTIATION, THE ASSOCIATION, PHILADELPHIA, PA, US, vol. 10, September 1999 (1999-09), pages 629-638, XP000978524 ISSN: 1044-9523
- EWERT S ET AL: "Stability improvement of antibodies for extracellular and intracellular applications: CDR grafting to stable frameworks and structure-based framework engineering" METHODS : A COMPANION TO METHODS IN ENZYMOLOGY, ACADEMIC PRESS INC., NEW YORK, NY, US, vol. 34, no. 2, October 2004 (2004-10), pages 184-199, XP004526805 ISSN: 1046-2023
- TILLMAN D. ET AL.: 'Both IgM and IgG Ati-DNA Antibodies Are the Products of Clonally Selective B Cell Stimulation in (NZB x NZW)FI Mice.' JOURNAL OF EXPERIMENTAL MEDICINE. vol. 176, September 1992, pages 761 - 779, XP002930986
- CATON A. ET AL.: 'Structural and Functional Implicaties of a restricted antibody response to a defined antigenic region on the influenza virus hemagglutinin.' THE EMBO JOURNAL vol. 5, no. 7, 1986, pages 1577 - 1587, XP002963047
- OGAWA K. ET AL.: 'The ephrin-A1 ligand and its receptor, EphA2, are expressed during tumorneovascularization.' ONCEGENE. vol. 19, October 2000, pages 6043 - 6052, XP001024674
- ZANTEK N D ET AL: "E-CADHERIN REGULATES THE FUNCTION OF THE EPHA2 RECEPTOR TYROSINE KINASE", CELL GROWTH AND DIFFERENTIATION, THE ASSOCIATION, PHILADELPHIA, PA, US, vol. 10, 1 September 1999 (1999-09-01), pages 629-638, XP000978524, ISSN: 1044-9523

## Description

### 1. FIELD OF THE INVENTION

The present invention relates to methods and compositions designed for the treatment, management, or prevention of cancer. The methods of the invention comprise the administration of an effective amount of one or more antibodies specific for EphA2, preferably monoclonal antibodies, that are EphA2 agonists and preferentially bind epitopes on EphA2 that are selectively exposed or increased on cancer cells relative to non-cancer cells. The invention also provides pharmaceutical compositions comprising one or more monoclonal antibodies of the invention either alone or in combination with one or more other agents useful for cancer therapy. Diagnostic methods and methods for screening for therapeutically useful anti-EphA2 antibodies are also provided.

### 2. BACKGROUND OF THE INVENTION

### Cancer

A neoplasm, or tumor, is a neoplastic mass resulting from abnormal uncontrolled cell growth which can be benign or malignant. Benign tumors generally remain localized. Malignant tumors are collectively termed cancers. The term "malignant" generally means that the tumor can invade and destroy neighboring body structures and spread to distant sites to cause death (for review, see Robbins and Angell, 1976, Basic Pathology, 2d Ed., W.B. Saunders Co., Philadelphia, pp. 68-122). Cancer can arise in many sites of the body and behave differently depending upon its origin. Cancerous cells destroy the part of the body in which they originate and then spread to other part(s) of the body where they start new growth and cause more destruction.

More than 1.2 million Americans develop cancer each year. Cancer is the second leading case of death in the United States and, if current trends continue, cancer is expected to be the leading cause of the death by the year 2010. Lung and prostate cancer are the top cancer killers for men in the United States. Lung and breast cancer are the top cancer killers for women in the United States. One in two men in the United States will be diagnosed with cancer at some time during his lifetime. One in three women in the United States will be diagnosed with cancer at some time during her lifetime.

A cure for cancer has yet to be found. Current treatment options, such as surgery, chemotherapy and radiation treatment, are often either ineffective or present serious side effects.

### Metastasis

The most life-threatening forms of cancer often arise when a population of tumor cells gains the ability to colonize distant and foreign sites in the body. These metastatic cells survive by overriding restrictions that normally constrain cell colonization into dissimilar tissues. For example, typical mammary epithelial cells will generally not grow or survive if transplanted to the lung, yet lung metastases are a major cause of breast cancer morbidity and mortality. Recent evidence suggests that dissemination of metastatic cells through the body can occur long before clinical presentation of the primary tumor. These micrometastatic cells may remain dormant for many months or years following the detection and removal of the primary tumor. Thus, a better understanding of the mechanisms that allow for the growth and survival of metastatic cells in a foreign microenvironment is critical for the improvement of therapeutics designed to fight metastatic cancer and diagnostics for the early detection and localization of metastases.

### Cancer Cell Signaling

Cancer is a disease of aberrant signal transduction. Aberrant cell signaling overrides anchorage-dependent constraints on cell growth and survival (Rhim, et al., Critical Reviews in Oncogenesis 8:305, 1997; Patarca, Critical Reviews in Oncogenesis 7:343, 1996; Malik, et al., Biochimica et Biophysica Acta 1287:73, 1996; Cance, et al., Breast Cancer Res Treat 35:105, 1995). Tyrosine kinase activity is induced by ECM anchorage and indeed, the expression or function of tyrosine kinases is usually increased in malignant cells (Rhim, et al., Critical Reviews in Oncogenesis 8:305,1997; Cance, et al., Breast Cancer Res Treat 35:105, 1995; Hunter, Cell 88:333, 1997). Based on evidence that tyrosine kinase activity is necessary for malignant cell growth, tyrosine kinases have been targeted with new therapeutics (Levitzki, et al., Science 267:1782, 1995; Kondapaka, et al., Molecular & Cellular Endocrinology 117:53, 1996; Fry, et al., Current Opinion in BioTechnology 6: 662, 1995). Unfortunately, obstacles associated with specific targeting to tumor cells often limit the application of these drugs. In particular, tyrosine kinase activity is often vital for the function and survival of benign tissues (Levitzki, et al., Science 267:1782, 1995). To minimize collateral toxicity, it is critical to identify and then target tyrosine kinases that are selectively overexpressed in tumor cells.

### EphA2

EphA2 is a 130 kDa receptor tyrosine kinase that is expressed in adult epithelia, where it is found at low levels and is enriched within sites of cell-cell adhesion (Zantek, et al, Cell Growth & Differentiation 10:629, 1999; Lindberg, et al., Molecular & Cellular Biology 10: 6316, 1990). This subcellular localization is important because EphA2 binds ligands (known as EphrinsA1 to A5) that are anchored to the cell membrane (Eph Nomenclature Committee, 1997, Cell 90:403; Gale, et al., 1997, Cell & Tissue Research 290: 227). The primary consequence of ligand binding is EphA2 autophosphorylation (Lindberg, et al., 1990, *supra*)*.* However, unlike other receptor tyrosine kinases, EphA2 retains enzymatic activity in the absence of ligand binding or phosphotyrosine content (Zantek, et al., 1999, *supra*)*.* EphA2 is upregulated on a large number of aggressive carcinoma cells.

### Cancer Therapy

One barrier to the development of anti-metastasis agents has been the assay systems that are used to design and evaluate these drugs. Most conventional cancer therapies target rapidly growing cells. However, cancer cells do not necessarily grow more rapidly but instead survive and grow under conditions that are non-permissive to normal cells (Lawrence and Steeg, 1996, World J. Urol. 14:124-130). These fundamental differences between the behaviors of normal and malignant cells provide opportunities for therapeutic targeting. The paradigm that micrometastatic tumors have already disseminated throughout the body emphasizes the need to evaluate potential chemotherapeutic drugs in the context of a foreign and three-dimensional microenvironment. Many standard cancer drug assays measure tumor cell growth or survival under typical cell culture conditions (*i.e.,* monolayer growth). However, cell behavior in two-dimensional assays often does not reliably predict tumor cell behavior *in vivo.*

Currently, cancer therapy may involve surgery, chemotherapy, hormonal therapy and/or radiation treatment to eradicate neoplastic cells in a patient (see, for example, Stockdale, 1998, "Principles of Cancer Patient Management," in Scientific American: Medicine, vol. 3, Rubenstein and Federman, eds., Chapter 12, Section IV). Recently, cancer therapy may also involve biological therapy or immunotherapy. All of these approaches can pose significant drawbacks for the patient. Surgery, for example, may be contraindicated due to the health of the patient or may be unacceptable to the patient. Additionally, surgery may not completely remove the neoplastic tissue. Radiation therapy is only effective when the neoplastic tissue exhibits a higher sensitivity to radiation than normal tissue, and radiation therapy can also often elicit serious side effects. Hormonal therapy is rarely given as a single agent and, although it can be effective, is often used to prevent or delay recurrence of cancer after other treatments have removed the majority of the cancer cells. Biological therapies/immunotherapies are limited in number and each therapy is generally effective for a very specific type of cancer.

With respect to chemotherapy, there are a variety of chemotherapeutic agents available for treatment of cancer. A significant majority of cancer chemotherapeutics act by inhibiting DNA synthesis, either directly, or indirectly by inhibiting the biosynthesis of the deoxyribonucleotide triphosphate precursors, to prevent DNA replication and concomitant cell division (see, for example, Gilman et al., Goodman and Gilman's: The Pharmacological Basis of Therapeutics, Eighth Ed. (Pergamom Press, New York, 1990)). These agents, which include alkylating agents, such as nitrosourea, anti-metabolites, such as methotrexate and hydroxyurea, and other agents, such as etoposides, campathecins, bleomycin, doxorubicin, daunorubicin, etc., although not necessarily cell cycle specific, kill cells during S phase because of their effect on DNA replication. Other agents, specifically colchicine and the vinca alkaloids, such as vinblastine and vincristine, interfere with microtubule assembly resulting in mitotic arrest. Chemotherapy protocols generally involve administration of a combination of chemotherapeutic agents to increase the efficacy of treatment.

Despite the availability of a variety of chemotherapeutic agents, chemotherapy has many drawbacks (see, for example, Stockdale, 1998, "Principles Of Cancer Patient Management" in Scientific American Medicine, vol. 3, Rubenstein and Federman, eds., ch. 12, sect. 10). Almost all chemotherapeutic agents are toxic, and chemotherapy causes significant, and often dangerous, side effects, including severe nausea, bone marrow depression, immunosuppression, etc. Additionally, even with administration of combinations of chemotherapeutic agents, many tumor cells are resistant or develop resistance to the chemotherapeutic agents. In fact, those cells resistant to the particular chemotherapeutic agents used in the treatment protocol often prove to be resistant to other drugs, even those agents that act by mechanisms different from the mechanisms of action of the drugs used in the specific treatment; this phenomenon is termed pleiotropic drug or multidrug resistance. Thus, because of drug resistance, many cancers prove refractory to standard chemotherapeutic treatment protocols.

There is a significant need for alternative cancer treatments, particularly for treatment of cancer that has proved refractory to standard cancer treatments, such as surgery, radiation therapy, chemotherapy, and hormonal therapy. Further, it is uncommon for cancer to be treated by only one method. Thus, there is a need for development of new therapeutic agents for the treatment of cancer and new, more effective, therapy combinations for the treatment of cancer. WOO1/12172 descibes cancer treatment using anti EphA2 agonistic antibodies.

### 3. SUMMARY OF THE INVENTION

EphA2 is overexpressed and functionally altered in a large number of malignant carcinomas. EphA2 is an oncoprotein and is sufficient to confer metastatic potential to cancer cells. EphA2 that is overexpressed on malignant cells exhibits kinase activity independent from ligand binding. The present inventors have found that a decrease in EphA2 levels decrease metastatic behavior of a cell. In particular, the present invention inventors' have discovered that, surprisingly, antibodies that agonize EphA2, *i.e.,* elicit EphA2 signaling, actually decrease EphA2 expression and inhibit tumor cell growth and/or metastasis. Although not intending to be bound by any mechanism of action, agonistic antibodies may repress malignant cell behavior by inducing EphA2 autophosphorylation, thereby causing subsequent EphA2 degradation to down-regulate expression. Thus the EphA2 antibodies of the invention agonize EphA2 signaling and increase phosphorylation of EphA2 ("EphA2 agonistic antibodies").

Differences in the subcellular localization, ligand binding properties or protein organization (*e.g*., structure, orientation in the cell membrane) can further distinguish the EphA2 that is present on cancer cells from EphA2 on non-cancer cells. In non-cancer cells, EphA2 is expressed at low levels and is localized to sites of cell-cell contact, where it can engage its membrane-anchored ligands. However, cancer cells generally display decreased cell-cell contacts and this can decrease EphA2-ligand binding. Furthermore, the overexpression of EphA2 can cause an excess of EphA2 relative to ligand that increases the amount of non-ligand bound EphA2. Consequently, changes in the subcellular distribution or membrane orientation of EphA2 can cause EphA2 to localize to sites in a cancer cell where it is inaccessible to ligand. Additionally, EphA2 may have altered ligand binding properties (*e.g*., due to an altered conformation) in cancer cells such that it is incapable of stable interactions with its ligand whether or not it is localized to the cell-cell junction. In each case, these changes can expose certain epitopes on the EphA2 in cancer cells that are not exposed in non-cancer cells. Accordingly, the invention also provides antibodies that specifically bind EphA2 but preferably bind an EphA2 epitope exposed on cancer cells but not on non-cancer cells ("exposed EphA2 epitope antibodies"). Exposing cancer cells to such EphA2 antibodies that preferentially bind epitopes on BphA2 that are selectively exposed or increased on cancer cells but not non-cancer cells targets the therapeutic/prophylactic antibody to cancer cells and prevents or decreases the cells' ability to proliferate while sparing non-cancer cells.

The present invention provides for the screening and identification of antibodies that bind to and agonize EphA2 and preferentially bind epitopes on EpbA2 that are selectively exposed or increased on cancer cells but not non-cancer cells, preferably monoclonal antibodies. In particular, the antibodies of the invention bind to the extracellular domain of EphA2 and, preferably, elicit EphA2 signaling and EphA22 autophosphorylation. In another particular embodiment, the antibodies of the invention bind to the extracellular domain of EphA2 and, preferably, bind an EphA2 epitope exposed on cancer cells but not non-cancer cells.
In a preferred embodiment, the antibodies of the invention are human or humanized.

In order, to identify antibodies that preferentially bind an EphA2 epitope exposed on cancer cells but not non-cancer cells, antibodies may be screened for the ability to preferentially bind EphA2 not bound to ligand, *e.g*., Ephrin A1, and that is not localized to cell-cell contacts. Any method known in the art to determine antibody binding/localization on a cell can be used to screen candidate antibodies for desirable binding properties. In a specific embodiment, immunofluorescence microscopy or flow cytometry is used to determine the binding characteristics of an antibody. In this embodiment, antibodies that bind poorly to EphA2 when it is bound to its ligand and localized to cell-cell contacts but bind well to free EphA2 on a cell are encompassed by the invention. In another specific embodiment, BphA2 antibodies are selected for their ability to compete with ligands (*e.g*., cell-anchored or purified ligands) for binding to EphA2 using cell-based or ELISA assays.

The antibodies of the invention may be human or humanized.

Accordingly, the present invention relates to pharmaceutical compositions and prophylactic and therapeutic regimens designed to prevent, treat, or manage cancer, particularly metastatic cancer, in a subject comprising administering one or more antibodies that specifically bind to and agonize EphA2 and preferentially bind epitopes on EphA2 that are selectively exposed or increased on cancer cells but not non-cancer cells. In one embodiment, the cancer is of an epithelial cell origin. In another embodiment, the cancer is a cancer of the skin, lung, colon, breast, prostate, bladder, kidney, or pancreas. In another embodiment, the cancer cells in the cancer to be prevented, treated, or managed overexpress BphA2. In a preferred embodiment, some EphA2 is not bound to ligand, either as a result of decreased cell-cell contacts, altered subcellular localization, or increases in amount of EphA22 relative to ligand. In a preferred embodiment, the methods of the invention are used to prevent, treat, or manage metastasis of tumors. The antibodies of the invention can be administered in combination with one or more other cancer therapies. In particular, the present invention provides antibodies for preventing, treating, or managing cancer in a subject which may be administered to said subject in a therapeutically or prophylactically effective amount of one or more EphA2 antibodies of the invention in combination with the administration of a therapeutically or prophylactically effective amount of one or more chemotherapies, hormonal therapies, biological therapies/immunotherapies and/or radiation therapies other than the administration of an EphA2 antibody of the invention or in combination with surgery.

The compositions of the invention are useful not only in untreated patients but are also useful in the treatment of patients partially or completely refractory to current standard and experimental cancer therapies, including but not limited to chemotherapies, hormonal therapies, biological therapies, radiation therapies, and/or surgery as well as to improve the efficacy of such treatments. Accordingly, in a preferred embodiment, the invention provides therapeutic and prophylactic antibodies for the treatment or prevention of cancer that has been shown to be or may be refractory or non-responsive to therapies other than those comprising administration of EphA2 antibodies of the invention. In a specific embodiment, one or more EphA2 antibodies of the invention are administered to a patient refractory or non-responsive to a non-EphA2-based treatment to render the patient non-refractory or responsive. The treatment to which the patient had previously been refractory or non-responsive can then be administered with therapeutic effect.

In addition, the present invention provides methods of screening for EphA2 antibodies of the invention. In particular, antibodies may be screened for binding to EphA2, particularly the extracellular domain of EphA2, using routine immunological techniques. In one embodiment, to identify agonistic EphA2 antibodies, EphA2 antibodies may be screened for the ability to elicit EphA2 signaling, i.e. increase EphA2 phosphorylation.

In another embodiment, to identify antibodies that preferentially bind an EphA2 epitope exposed on cancer cells but not non-cancer cells, antibodies may be screened for the ability to preferentially bind EphA2 that is not bound to ligand, *e.g*., Ephrin A1, and that is not localized to cell-cell contacts. Any method known in the art to determine antibody binding/localization on a cell can be used to screen candidate antibodies for desirable binding properties. In a specific embodiment, immunofluorescence microscopy or flow cytometry is used to determine the binding characteristics of an antibody. In this embodiment, antibodies that bind poorly to EphA2 when it is bound to its ligand and localized to cell-cell contacts but bind well to free EphA2 on a cell are encompassed by the invention. In another specific embodiment, EphA2 antibodies are selected for their ability to compete with ligands (*e.g*., cell-anchored or purified ligands) for binding to EphA2 using cell-based or ELISA assays.

The invention further provides diagnostic methods using the EphA2 antibodies of the invention to evaluate the efficacy of cancer treatment, either EphA2-based or not EphA2-based. In general, increased EphA2 expression is associated with increasingly invasive and metastatic cancers. Accordingly, a reduction in EphA2 expression with a particular treatment indicates that the treatment is reducing the invasiveness and/or metastatic potential of cancer. In particular embodiments, the diagnostic methods of the invention provide methods of imaging and localizing metastases and methods of diagnosis and prognosis using tissues and fluids distal to the primary tumor site (as well as methods using tissues and fluids of the primary tumor), for example, whole blood, sputum, urine, serum, fine needle aspirates (*i.e*., biopsies). In other embodiments, the diagnostic methods of the invention provide methods of imaging and localizing metastases and methods of diagnosis and prognosis *in vivo*. In such embodiments, primary metastatic tumors are detected using an antibody of the invention, preferably an exposed EphA2 epitope antibody. The antibodies of the invention may also be used for immunohistochemical analyses of frozen or fixed cells or tissue assays.

In another embodiment, kits comprising the pharmaceutical compositions or diagnostic reagents of the invention are provided.

### 3.1 DEFINITIONS

As used herein, the term "agonist" refers to a antibody, or antibody fragment, that increases the activity, activation or function of another molecule. EphA2 agonists cause increased phosphorylation and may cause degradation of EphA2 protein. EphA2 antibodies that agonize EphA2 may or may not preferentially bind an EphA2 epitope that is exposed in a cancer cell relative to a non-cancer cell.

The term "antibodies or fragments thereof that immunospecifically bind to EphA2" as used herein refers to antibodies or fragments thereof that specifically bind to an EphA2 polypeptide or a fragment of an EphA2 polypeptide and do not specifically bind to other non-EphA2 polypeptides. Preferably, antibodies or fragments that immunospecifically bind to an EphA2 polypeptide or fragment thereof do not non-specifically cross-react with other antigens (*e.g*., binding cannot be competed away with a non-EphA2 protein, *e.g*., BSA in an appropriate immunoassay). Antibodies or fragments that immunospecifically bind to an EphA2 polypeptide can be identified, for example, by immunoassays or other techniques known to those of skill in the art. Antibodies of the invention include, but are not limited to, synthetic monoclonal antibodies, multispecific antibodies (including bi-specific antibodies), human antibodies, humanized antibodies, chimeric antibodies, synthetic antibodies, single-chain Fvs (scFv) (including bi-specific scFvs), single chain antibodies, Fab fragments, F(ab') fragments, disulfide-linked Fvs (sdFv), and anti-idiotypic (anti-Id) antibodies, and epitope-binding fragments of any of the above. In particular, antibodies of the present invention include immunoglobulin molecules and immunologically active portions of immunoglobulin molecules, *i.e.,* molecules that contain an antigen binding site that immunospecifically binds to an EphA2 antigen (*e.g*., one or more complementarity determining regions (CDRs) of an anti-EphA2 antibody). Preferably agonistic antibodies or fragments that immunospecifically bind to an EphA2 polypeptide or fragment thereof only agonize EphA2 and do not significantly agonize other activities.

As used herein, the term "cancer" refers to a disease involving cells that have the potential to metastasize to distal sites and exhibit phenotypic traits that differ from those of non-cancer cells, for example, formation of colonies in a three-dimensional substrate such as soft agar or the formation of tubular networks or weblike matrices in a three-dimensional basement membrane or extracellular matrix preparation, such as MATRIGEL™. Non-cancer cells do not form colonies in soft agar and form distinct sphere-like structures in three-dimensional basement membrane or extracellular matrix preparations. Cancer cells acquire a characteristic set of functional capabilities during their development, albeit through various mechanisms. Such capabilities include evading apoptosis, self-sufficiency in growth signals, insensitivity to anti-growth signals, tissue invasion/metastasis, limitless replicative potential, and sustained angiogenesis. The term "cancer cell" is meant to encompass both pre-malignant and malignant cancer cells.

The term "derivative" as used herein refers to a polypeptide that comprises an amino acid sequence of an EphA2 polypeptide, a fragment of an EphA2 polypeptide, an antibody that immunospecifically binds to an EphA2 polypeptide, or an antibody fragment that immunospecifically binds to an EphA2 polypeptide which has been altered by the introduction of amino acid residue substitutions, deletions or additions (*i.e.,* mutations). In some embodiments, an antibody derivative or fragment thereof comprises amino acid residue substitutions, deletions or additions in one or more CDRs. The antibody derivative may have substantially the same binding, better binding, or worse binding when compared to a non-derivative antibody. In specific embodiments, one, two, three, four, or five amino acid residues of the CDR have been substituted, deleted or added (*i.e.*, mutated). The term "derivative" as used herein also refers to an EphA2 polypeptide, a fragment of an EphA2 polypeptide, an antibody that immunospecifically binds to an EphA2 polypeptide, or an antibody fragment that immunospecifically binds to an EphA2 polypeptide which has been modified, *i.e,* by the covalent attachment of any type of molecule to the polypeptide. For example, but not by way of limitation, an EphA2 polypeptide, a fragment of an EphA2 polypeptide, an antibody, or antibody fragment may be modified, *e.g.,* by glycosylation, acetylation, pegylation, phosphorylation, amidation, derivatization by known protecting/blocking groups, proteolytic cleavage, linkage to a cellular ligand or other protein, etc. A derivative of an EphA2 polypeptide, a fragment of an EphA2 polypeptide, an antibody, or antibody fragment may be modified by chemical modifications using techniques known to those of skill in the art, including, but not limited to specific chemical cleavage, acetylation, formylation, metabolic synthesis of tunicamycin, etc. Further, a derivative of an EphA2 polypeptide, a fragment of an EphA2 polypeptide, an antibody, or antibody fragment may contain one or more non-classical amino acids. In one embodiment, a polypeptide derivative possesses a similar or identical function as an EphA2 polypeptide, a fragment of an EphA2 polypeptide, an antibody, or antibody fragment described herein. In another embodiment, a derivative of EphA2 polypeptide, a fragment of an EphA2 polypeptide, an antibody, or antibody fragment has an altered activity when compared to an unaltered polypeptide. For example, a derivative antibody or fragment thereof can bind to its epitope more tightly or be more resistant to proteolysis.

The term "epitopes" as used herein refers to a portion of an EphA2 polypeptide having antigenic or immunogenic activity in an animal, preferably in a mammal, and most preferably in a mouse or a human. An epitope having immunogenic activity is a portion of an EphA2 polypeptide that elicits an antibody response in an animal. An epitope having antigenic activity is a portion of an EphA2 polypeptide to which an antibody immunospecifically binds as determined by any method well known in the art, for example, by immunoassays. Antigenic epitopes need not necessarily be immunogenic.

The "fragments" described herein include a peptide or polypeptide comprising an amino acid sequence of at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least contiguous 80 amino acid residues, at least contiguous 90 amino acid residues, at least contiguous 100 amino acid residues, at least contiguous 125 amino acid residues, at least 150 contiguous amino acid residues, at least contiguous 175 amino acid residues, at least contiguous 200 amino acid residues, or at least contiguous 250 amino acid residues of the amino acid sequence of an EphA2 polypeptide or an antibody that immunospecifically binds to an EphA2 polypeptide. Preferably, antibody fragments are epitope-binding fragments.

As used herein, the term "humanized antibody" refers to forms of non-human (*e.g*., murine) antibodies that are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or non-human primate having the desired specificity, affinity, and capacity. In some instances, Framework Region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin, that immunospecifically binds to an EphA2 polypeptide, that has been altered by the introduction of amino acid residue substitutions, deletions or additions (*i.e*., mutations). In some embodiments, a humanized antibody is a derivative. Such a humanized antibody comprises amino acid residue substitutions, deletions or additions in one or more non-human CDRs. The humanized antibody derivative may have substantially the same binding, better binding, or worse binding when compared to a non-derivative humanized antibody. In specific embodiments, one, two, three, four, or five amino acid residues of the CDR have been substituted, deleted or added (*i.e.*, mutated). For further details in humanizing antibodies, see European Patent Nos. EP 239,400, EP 592,106, and EP 519,596; International Publication Nos. WO 91/09967 and WO 93/17105; U.S. Patent Nos. 5,225,539, 5,530,101, 5,565,332, 5,585,089, 5,766,886, and 6,407,213; and Padlan, 1991, Molecular Immunology 28(4/5):489-498; Studnicka et al., 1994, Protein Engineering 7(6):805-814; Roguska et al., 1994, PNAS 91:969-973; Tan et al., 2002, J. Immunol. 169:1119-25; Caldas et al., 2000, Protein Eng. 13:353-60; Morea et al., 2000, Methods 20:267-79; Baca et al., 1997, J. Biol. Chem. 272:10678-84; Roguska et al., 1996, Protein Eng. 9:895-904; Couto et al., 1995, Cancer Res. 55 (23 Supp):5973s-5977s; Couto et al., 1995, Cancer Res. 55:1717-22; Sandhu, 1994, Gene 150:409-10; Pedersen et al., 1994, J. Mol. Biol. 235:959-73; Jones et al., 1986, Nature 321:522-525; Reichmann et al., 1988, Nature 332:323-329; and Presta, 1992, Curr. Op. Struct. Biol. 2:593-596.

As used herein, the term "hypervariable region" refers to the amino acid residues of an antibody which are responsible for antigen binding. The hypervariable region comprises amino acid residues from a "Complementarity Determining Region" or "CDR" (*i.e*. residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*i.e*. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk, 1987, J. Mol. Biol. 196:901-917). CDR residues for EA2 are listed in Table 1. "Framework Region" or "FR" residues are those variable domain residues other than the hypervariable region residues as herein defined.

As used herein, the term "in combination" refers to the use of more than one prophylactic and/or therapeutic agents. The use of the term "in combination" does not restrict the order in which prophylactic and/or therapeutic agents are administered to a subject with a hyperproliferative cell disorder, especially cancer. A first prophylactic or therapeutic agent can be administered prior to (*e.g*., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (*e.*g., 1 minute, 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second prophylactic or therapeutic agent to a subject which had, has, or is susceptible to a hyperproliferative cell disorder, especially cancer. The prophylactic or therapeutic agents are administered to a subject in a sequence and within a time interval such that the agent of the invention can act together with the other agent to provide an increased benefit than if they were administered otherwise. Any additional prophylactic or therapeutic agent can be administered in any order with the other additional prophylactic or therapeutic agents.

As used herein, the phrase "low tolerance" refers to a state in which the patient suffers from side effects from treatment so that the patient does not benefit from and/or will not continue therapy because of the adverse effects and/or the harm from the side effects outweighs the benefit of the treatment.

As used herein, the terms "manage," "managing" and "management" refer to the beneficial effects that a subject derives from administration of a prophylactic or therapeutic agent, which does not result in a cure of the disease. In certain embodiments, a subject is administered one or more prophylactic or therapeutic agents to "manage" a disease so as to prevent the progression or worsening of the disease.

As used herein, the phrase "non-responsive/ refractory" is used to describe patients treated with one or more currently available therapies (*e.g*., cancer therapies) such as chemotherapy, radiation therapy, surgery, hormonal therapy and/or biological therapy/immunotherapy, particularly a standard therapeutic regimen for the particular cancer, wherein the therapy is not clinically adequate to treat the patients such that these patients need additional effective therapy, *e.g*., remain unsusceptible to therapy. The phrase can also describe patients who respond to therapy yet suffer from side effects, relapse, develop resistance, etc. In various embodiments, "non-responsive/refractory" means that at least some significant portion of the cancer cells are not killed or their cell division arrested. The determination of whether the cancer cells are "non-responsive/refractory" can be made either *in vivo* or *in vitro* by any method known in the art for assaying the effectiveness of treatment on cancer cells, using the art-accepted meanings of "refractory" in such a context. In various embodiments, a cancer is "non-responsive/refractory" where the number of cancer cells has not been significantly reduced, or has increased during the treatment.

As used herein, the term "potentiate" refers to an improvement in the efficacy of a therapeutic agent at its common or approved dose.

As used herein, the terms "prevent," "preventing" and "prevention" refer to the prevention of the recurrence or spread of a disease in a subject resulting from the administration of a prophylactic or therapeutic agent

As used herein, the terms "prophylactic agent" and "prophylactic agents" refer to any agent(s) that can be used in the prevention of the onset, recurrence or spread of a disorder associated with EphA2 overexpression, particularly cancer. The term "prophylactic agent" refers to an BphA2 agonistic antibody and optionally an exposed EphA2 epitope antibody. In certain other embodiments, the terms "prophylactic agent" and "prophylactic agents" refer to cancer chemotherapeutics, radiation therapy, hormonal therapy, biological therapy (*e.g.*, immunotherapy), and/or EphA2 antibodies of the invention. In other embodiments, more than one prophylactic agent may be administered in combination.

As used herein, a "prophylactically effective amount" refers to that amount of the prophylactic agent sufficient to result in the prevention of the recurrence or spread of cancer. A prophylactically effective amount may refer to the amount of prophylactic agent sufficient to prevent the recurrence or spread of cancer or the occurrence of cancer in a patient, including but not limited to those predisposed to cancer or previously exposed to carcinogens. A prophylactically effective amount may also refer to the amount of the prophylactic agent that provides a prophylactic benefit in the prevention of cancer. Further, a prophylactically effective amount with respect to a prophylactic agent of the invention means that amount of prophylactic agent alone, or in combination with other agents, that provides a prophylactic benefit in the prevention of cancer. Used in connection with an amount of an EphA2 antibody of the invention, the term can encompass an amount that improves overall prophylaxis or enhances the prophylactic efficacy of or synergies with another prophylactic agent

A used herein, a "protocol" includes dosing schedules and dosing regimens.

As used herein, the phrase "side effects" encompasses unwanted and adverse effects of a prophylactic or therapeutic agent. Adverse effects are always unwanted, but unwanted effects are not necessarily adverse. An adverse effect from a prophylactic or therapeutic agent might be harmful or uncomfortable or risky. Side effects from chemotherapy include, but are not limited to, gastrointestinal toxicity such as, but not limited to, early and late-forming diarrhea and flatulence, nausea, vomiting, anorexia, leukopenia, anemia, neutropenia, asthenia, abdominal cramping, fever, pain, loss of body weight, dehydration, alopecia, dyspnea, insomnia, dizziness, mucositis, xerostomia, and kidney failure, as well as constipation, nerve and muscle effects, temporary or permanent damage to kidneys and bladder, flu-like symptoms, fluid retention, and temporary or permanent infertility. Side effects from radiation therapy include but are not limited to fatigue, dry mouth, and loss of appetite. Side effects from biological therapies/immunotherapies include but are not limited to rashes or swellings at the site of administration, flu-like symptoms such as fever, chills and fatigue, digestive tract problems and allergic reactions. Side effects from hormonal therapies include but are not limited to nausea, fertility problems, depression, loss of appetite, eye problems, headache, and weight fluctuation. Additional undesired effects typically experienced by patients are numerous and known in the art. Many are described in the Physicians' Desk Reference (56th ed., 2002).

As used herein, the terms "single-chain Fv" or "scFv" refer to antibody fragments comprise the VH and VL domains of antibody, wherein these domains are present in a single polypeptide chain. Generally, the Fv polypeptide further comprises a polypeptide linker between the VH and VL domains which enables the scFv to form the desired structure for antigen binding. For a review of sFv see Pluckthun in The Pharmacology of Monoclonal Antibodies, vol. 113, Rosenburg and Moore eds. Springer-Verlag, New York, pp. 269-315 (1994). In specific embodiments, scFvs include bispecific scFvs and humanized scFvs.

As used herein, the terms "subject" and "patient" are used interchangeably. As used herein, a subject is preferably a mammal such as a non-primate (*e.g.,* cows, pigs, horses, cats, dogs, rats etc.) and a primate (*e.g*., monkey and human), most preferably a human.

As used herein, the terms "treat," "treating" and "treatment" refer to the eradication, reduction or amelioration of symptoms of a disease or disorder, particularly, the eradication, removal, modification, or control of primary, regional, or metastatic cancer tissue that results from the administration of one or more therapeutic agents. In certain embodiments, such terms refer to the minimizing or delaying the spread of cancer resulting from the administration of one or more therapeutic agents to a subject with such a disease.

As used herein, the terms "therapeutic agent" and "therapeutic agents" refer to any agent(s) that can be used in the prevention, treatment, or management of a disorder associated with the overexpression of EphA2, particularly cancer. In certain embodiments, the term "therapeutic agent" refers to an EpbA2 agonistic antibody and optionally an exposed EphA2 epitope antibody. In certain other embodiments, the terms "therapeutic agent" and "therapeutic agents" refer to cancer chemotherapeutics, radiation therapy, hormonal therapy, biological therapy/immunotherapy, in combination with EphA2 antibody of the invention. In other embodiments, more than one therapeutic agent may be administered in combination.

As used herein, a "therapeutically effective amount" refers to that amount of the therapeutic agent sufficient to destroy, modify, control or remove primary, regional or metastatic cancer tissue. A therapeutically effective amount may refer to the amount of therapeutic agent sufficient to delay or minimize the spread of cancer. A therapeutically effective amount may also refer to the amount of the therapeutic agent that provides a therapeutic benefit in the treatment or management of cancer. Further, a therapeutically effective amount with respect to a therapeutic agent of the invention means that amount of therapeutic agent alone, or in combination with other therapies, that provides a therapeutic benefit in the treatment or management of cancer. Used in connection with an amount of an EphA2 antibody of the invention, the term can encompass an amount that improves overall therapy, reduces or avoids unwanted effects, or enhances the therapeutic efficacy of or synergies with another therapeutic agent.

### 4. DESCRIPTION OF THE FIGURES

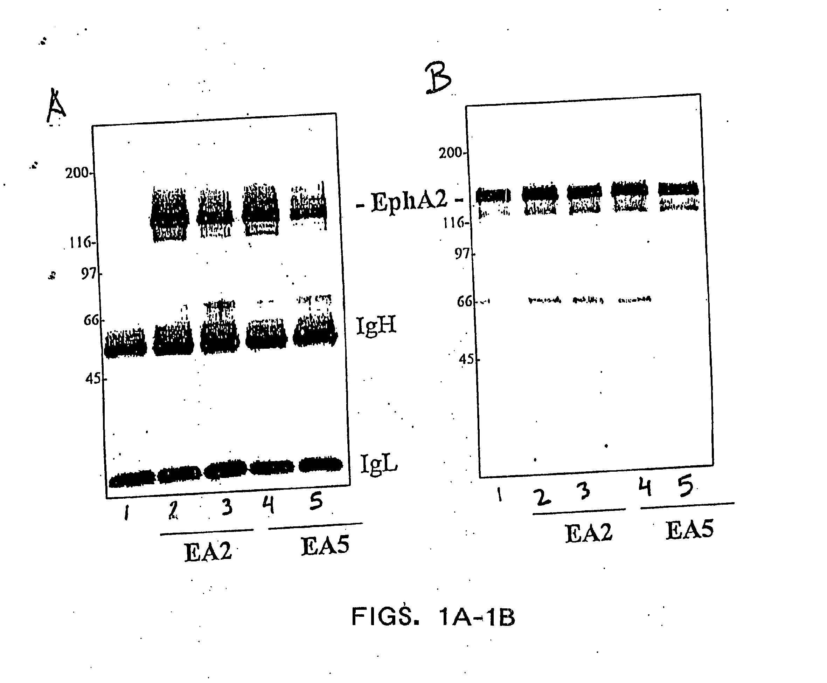

**FIGS. 1A-1C****:** EphA2 antibodies promote tyrosine phosphorylation and degradation of EphA2 in MDA-MB-231 cells. **(A, B)** Monolayers of MDA-MB-231 cells were incubated in the presence of EA2 or EA5 or control for 8 minutes at 37°C. Cell lysates were then immunoprecipitated with an EphA2-specific antibody, resolved by SDS-PAGE and subjected to western blot analysis with a phosphotyrosine-specific antibody **(A).** The membranes were stripped and re-probed with the EphA2-specific antibody used in the immunoprecipitation as a loading control **(B).** Levels of EphA2 phosphorylation increase with antibody incubation. **(C)** Monolayers of MDA-MB-231 cells were incubated in the presence of presence of 30 µg/ml EA2 or EA5 or a control for 24 hours at 37°C. Cell lysates were then resolved by SDS PAGE and subjected to western blot analysis with an EphA2-specific antibody. EphA2 protein levels decrease with antibody incubation. The relative mobility of molecular mass standards is shown on the left of each blot. Antibody heavy (IgH) and light (IgL) chains are indicated.

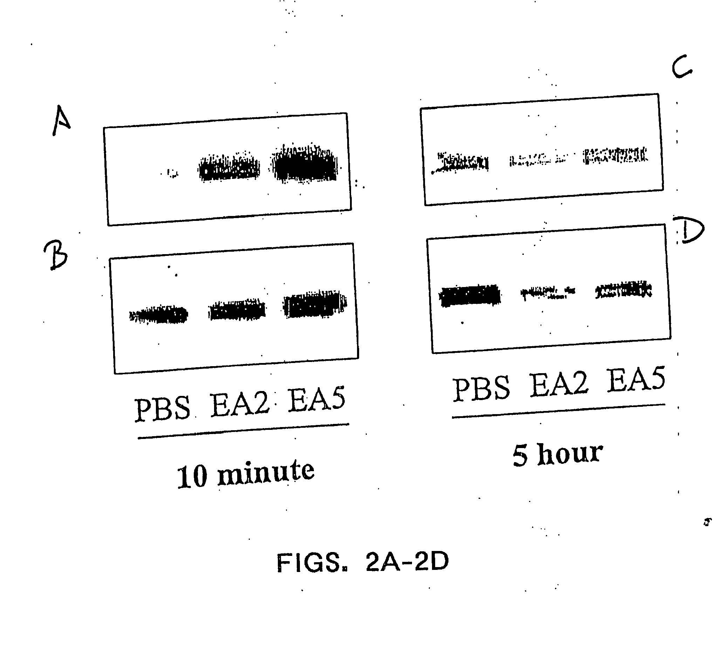

**FIGS. 2A-2D****:** EphA2 antibodies promote tyrosine phosphorylation and degradation of EphA2 in A549 cells. Monolayers of A549 cells were incubated at 37°C in the presence of EA2 or EA5 or control (PBS) for either **(A, B)** 10 minutes or **(C, D)** 5 hours. Cell lysates were then immunoprecipitated with an EphA2-specific antibody D7, resolved by SDS-PAGE and subjected to western blot analysis with a phosphotyrosine-specific antibody **(A, C).** The membranes were stripped and re-probed with the EphA2-specific antibody used in the immunoprecipitation as a loading control **(B, D).**

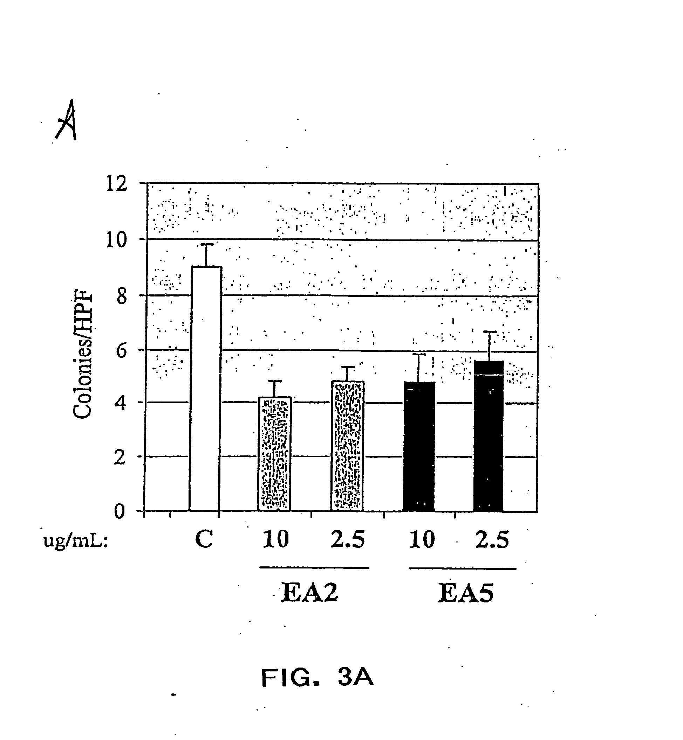

**FIGS. 3A-3B****:** EphA2 antibodies inhibit malignant tumor cell growth in vitro. Purified EphA2 antibodies were incubated with both malignant and benign tumor cells for 7 days at 37°C in soft agar. **(A)** A549 malignant lung cancer cells were incubated with either 10 µg/ml or 2.5 µg/ml of EA2 or EA5 monoclonal antibodies or a control (PBS). All amounts of antibodies used inhibited cell growth in soft agar. **(B)** Benign MCF-7 breast epithelial tumor cells were converted to malignant cells by the overexpression of EphA2 (MCF-7^{EphA2}). Both tumor cell types were incubated with either EA2 monoclonal antibodies or a control (PBS). EA2 inhibits the ability of MCF-7^{EphA2} cells to grow in soft agar. Results are reported as colonies per high-powered field (HPF).

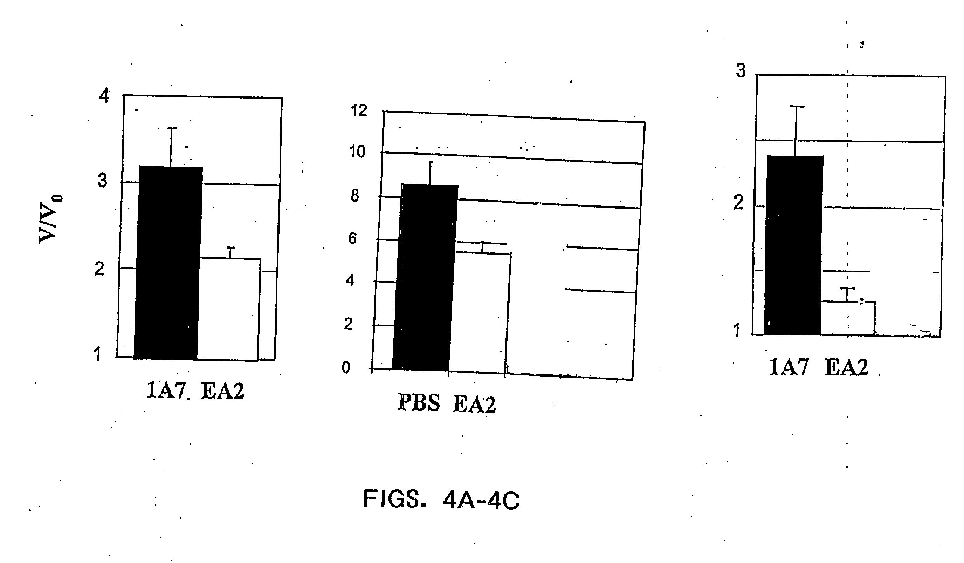

**FIGS. 4A-4D****:** The EphA2 antibody EA2 inhibits tumor cell growth in vivo. MDA-MB-231 breast cancer cells were implanted **(A)** orthotopically or **(B)** subcutaneously into athymic mice. **(C)** A549 lung cancer cells were implanted subcutaneously into athymic mice. After the tumors had grown to an average volume of 100mm³, mice were administered 6 mg/kg of the indicated antibody or negative control (PBS or 1A7 antibody) intraperitoneally twice a week for 3 weeks. Tumor growth was assessed and expressed as a ratio of the tumor volume divided by initial tumor volume (100 mm³). **(D)** MDA-MB-231 breast cancer cells were implanted subcutaneously into athymic mice. After the tumors had grown to an average volume of 100mm³, mice were administered 6 mg/kg of the indicated antibody or negative control intraperitoneally twice a week for 3 weeks. Total tumor volume was determined after sacrifice. Negative control is black and EA2 is white.

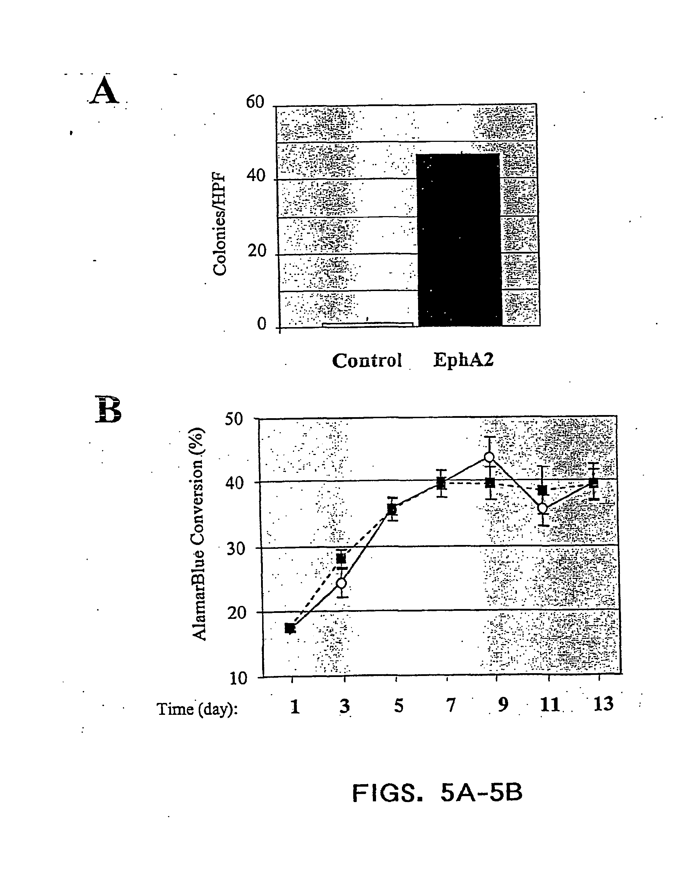

**FIGS. 5A-5B****:** EphA2 overexpression selectively increases malignant cell growth. (A) 1x10⁵ control (white bar) or MCF-7^{EPhA2} cells (black bar) were suspended in soft agar in the presence of 1 mg/ml 17β-estradiol for 14 days prior to microscopic evaluation. EphA2-transfected cells formed more colonies (47 colonies/high powered field (HPF)) than matched controls (1 colony/HPF; P<0.01). **(B)** Monolayer growth assays did not distinguish between the growth of control (white circles) and MCF-7^{EPhA2} cells (black squares).

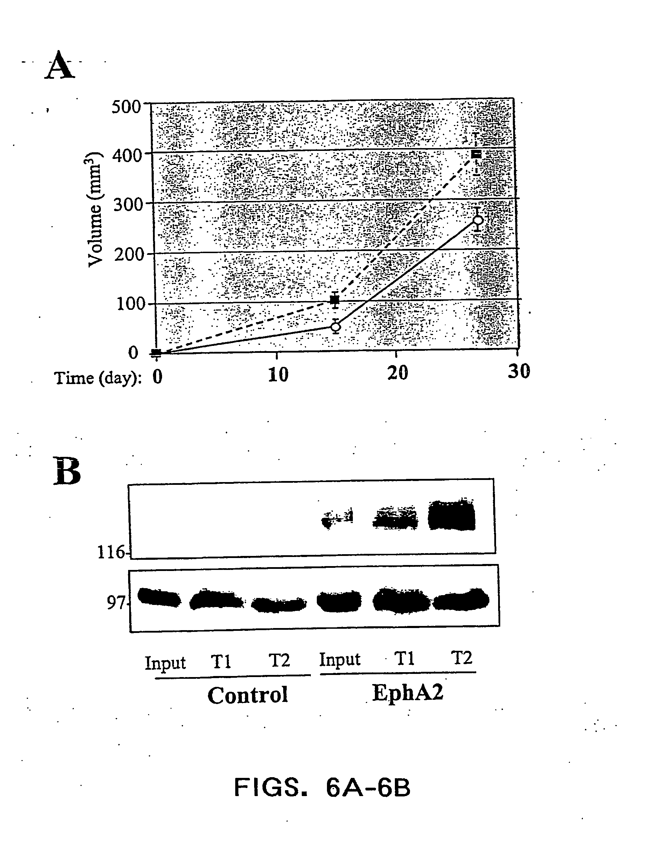

**FIGS. 6A-6B****:** EphA2 overexpression increases tumorigenic potential. **(A)** 1x10⁶ control (white circle) or MCF-7^{EPhA2} cells (black square) were implanted into the mammary fatpad of athymic mice (n=20 mice per group) in the presence of supplemental estrogen (1 µM 17β-estradiol). The tumors formed by MCF-7^{EphA2} cells were significantly larger than tumors formed by matched controls (P = 0.027). **(B)** Equal amounts of protein lysate, isolated from input cells or resected tumors (T) were evaluated by western blot analyses with an EphA2 antibody (D7). The membranes were stripped and re-probed with a β-catenin-specific antibody as a loading control.

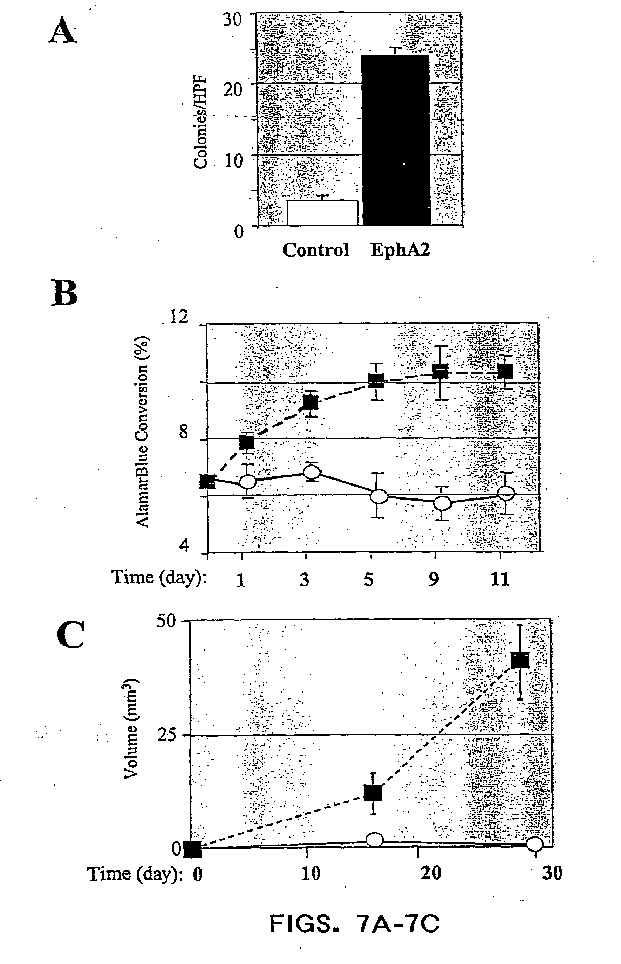

**FIGS. 7A-7C****:** EphA2 overexpression decreases estrogen dependence. **(A)** 1x10⁵ control (white bar) or MCF-7^{EphA2} cells (black bar) were suspended in soft agar in the absence of exogenous estrogen and colony formation was evaluated microscopically after 14 days. The monolayer growth **(B)** and tumorigenic potential **(C)** of MCF-7^{EphA2} (black square) cells were increased relative to matched controls (white circle) in the absence of supplemental estrogen (P<0.01 and P<0.004, respectively).

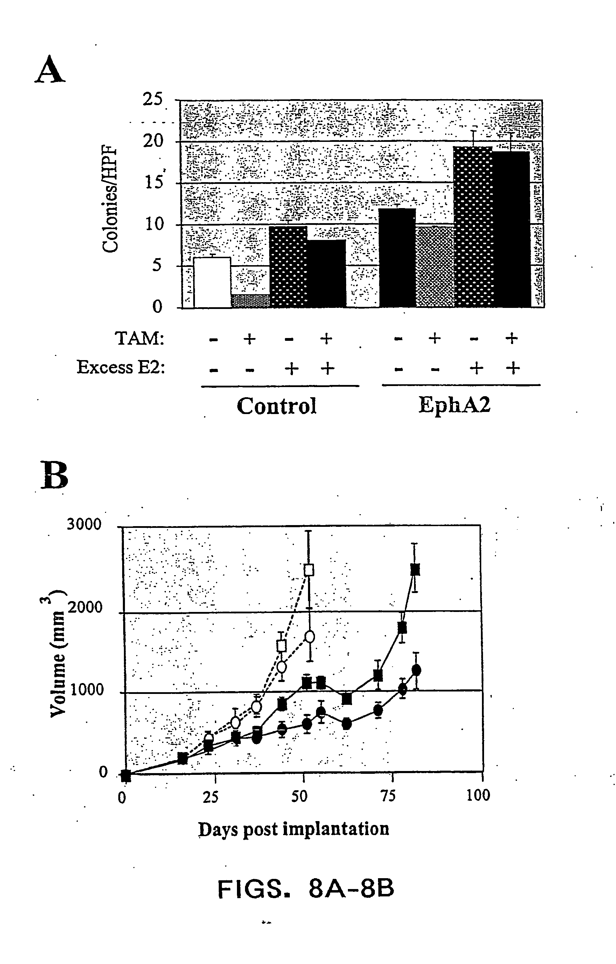

**FIGS. 8A-8B****:** EphA2 overexpression decreases tamoxifen sensitivity. **(A)** 1x10⁵ MCF-7 or MCF-7^{EPhA2} cells were suspended in soft agar in the presence of ₁µM tamoxifen (TAM) and or 1µM 17β-estradiol and colony formation was evaluated microscopically after 14 days. **(B)** MCF-7 (circles) or MCF-7^{EphA2} cells (squares) were implanted into the mammary fatpad (n=15 mice per group) in the presence of supplemental estrogen. Tamoxifen treatment was initiated 17 days post-implantation. Tumor volume of tamoxifen treated (black circles and squares) and saline treated (white circles and squares) animals was measured at the indicated time. Note the lower inhibitory effects of tamoxifen on MCF-7^{EphA2} relative to control cells (P=0.01).

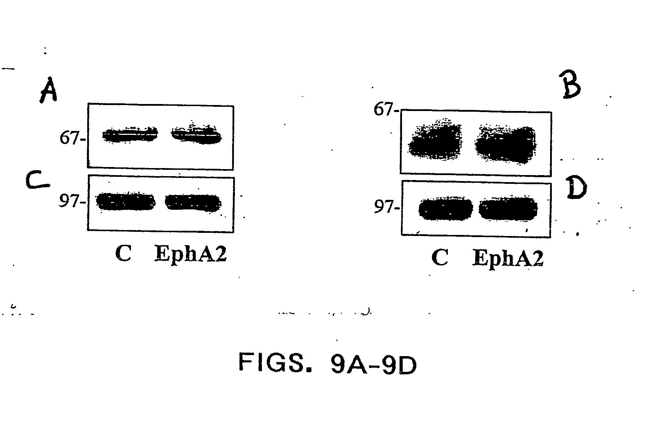

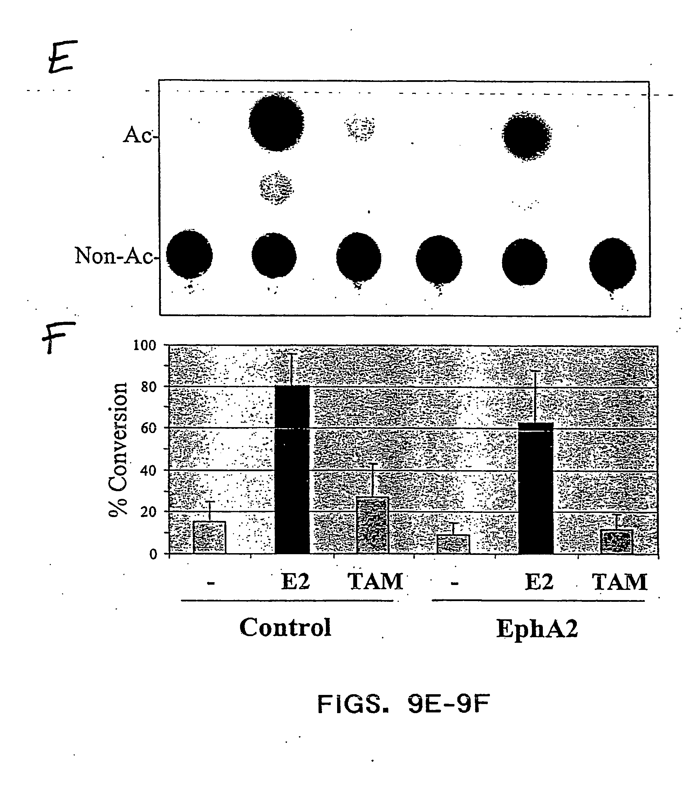

**FIGS. 9A-9F****:** Estrogen receptor is expressed but functionally altered in MCF-7^{EphA2} cells. **(A)** ERa and **(B)** ERβ levels were evaluated in MCF-7^{neo} control cells and MCF-7^{EphA2} cells by western blot analyses with an EphA2-specific antibody (D7). **(C, D)** The membranes were stripped and re-probed with a β-catenin-specific antibody as a loading control. **(E, F)** Estrogen receptor activity was measured using a CAT reporter system, revealing comparable estrogen receptor activity in control and MCF-7^{EphA2} cells. The average results from three experiments are graphed in **(F).** E2 indicates estrogen treatment; TAM indicates tamoxifen treatment; % conversion indicates the amount of substrate converted from non-acetylated substrate (non-AC) to acetylated substrate (AC) by CAT enzyme.

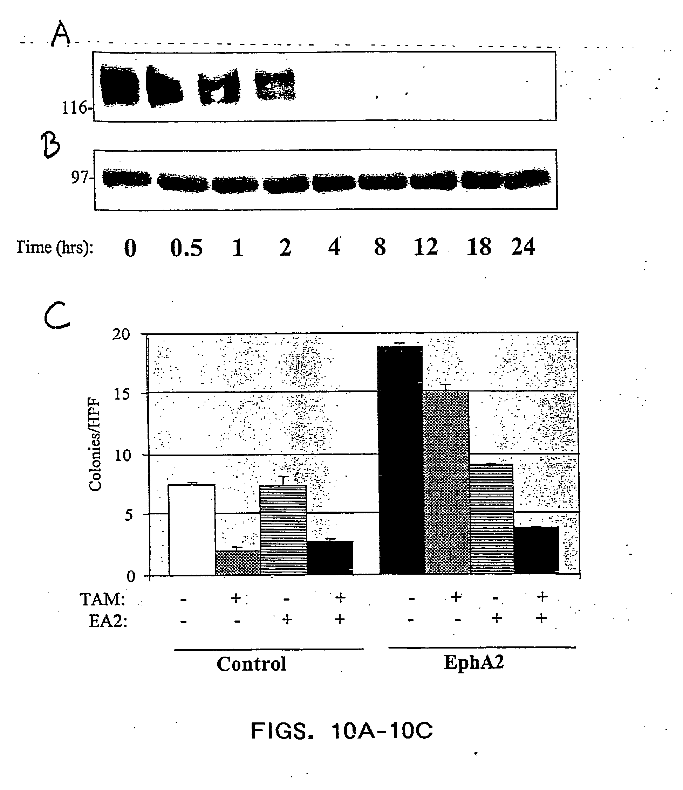

**FIGS. 10A-10C****:** EphA2 agonistic antibody EA2 decreases malignant growth. MCF-7^{EPhA2} cells were incubated in the presence of 3 µg/ml of EA2 for the time indicated prior to sample extraction and western blot analyses with an EphA2-specific antibody (D7). **(B)** The membrane was stripped and re-probed with a β-catenin-specific antibody as a loading control. **(C)** 1x10⁵ control or MCF-7^{EPhA2} cells were suspended in soft agar in the presence or absence of tamoxifen (TAM, 1µM) and EphA2 agonistic antibody (EA2, 10µg/ml). Note that EA2 increased the sensitivity of MCF-7^{EphA2} cells to tamoxifen.

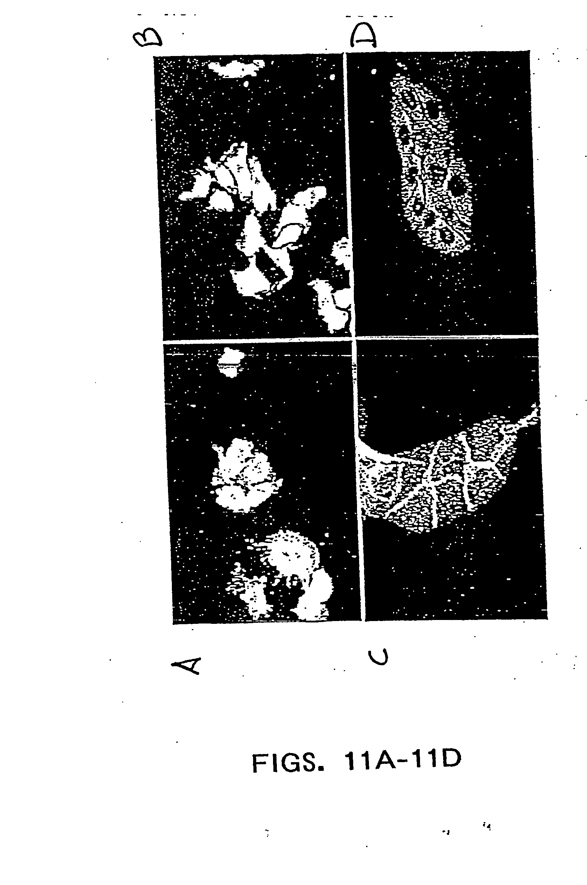

**FIGS.11A-11D****:** EA2 and EA5 selectively bind to malignant cells. The anti-EphA2 monoclonal antibodies EA2 **(A, C)** and EA5 **(B, D)** bind malignant MDA-MB-231 breast epithelial tumor cells **(A, B)** more strongly than benign MCF-10A breast epithelial tumor cells **(C, D)** as shown by immunofluorescent staining.

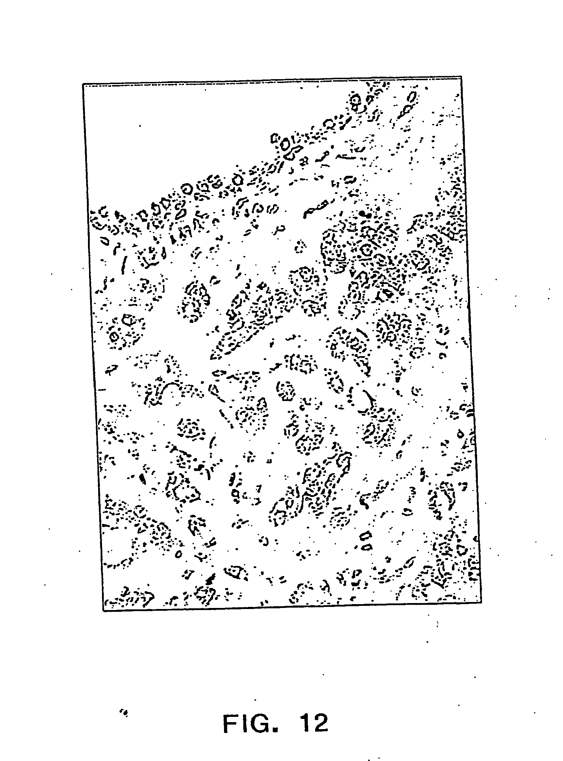

**FIG.12****:** EA2 was immunoreactive against malignant prostate cells. The anti-EphA2 monoclonal antibody EA2 identified malignant prostate cancer cells in formalin-fixed, paraffin-embedded archival clinical specimens.

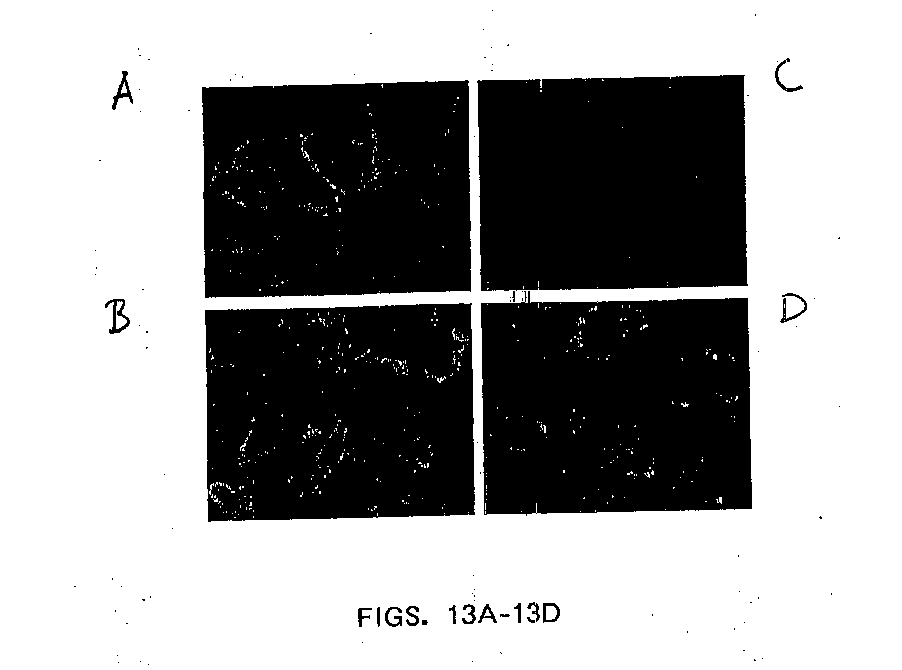

**FIGS. 13A-13D****.:** EphA2 EA2 antibody preferentially binds cancer cells. Non-transformed MCF-10A **(A, C)** or transformed MDA-MB-231 **(B, D)** cells were incubated with 10 µg/ml **(A, B)** Eph099B-233.152 or **(C, D)** EA2 at 4°C prior to fixation and immunolabeling with fluorophore-conjugated anti-mouse IgG.

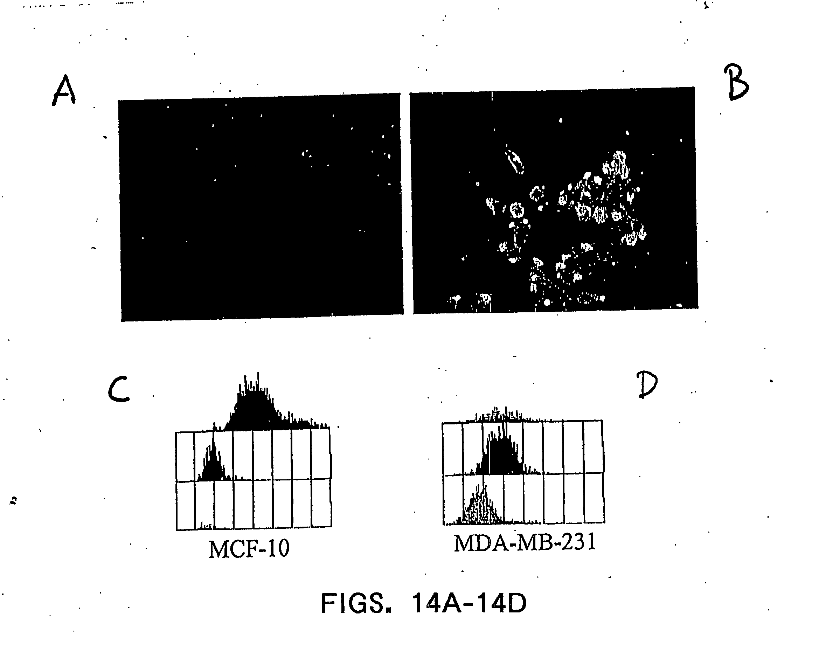

**FIGS. 14A-14D****:** EphA2 EA2 antibody preferentially binds EphA2 epitopes exposed by decreasing cell-cell contacts. **(A, B)** Non-transformed MCF-10A cells were labeled with EA2 at 4°C either before **(A)** or after **(B)** treatment with EGTA and prior to fixation and immunolabeling with fluorophore-conjugated anti-mouse IgG. **(C, D)** Non-transformed MCF-10A **(C)** or transformed MDA-MB-231 **(D)** cells were labeled with EA2 either before (middle) or after (top) treatment with EGTA. Control cells were incubated with secondary antibody alone (bottom). The amount of EA2-EphA2 binding was measured using flow cytometry.

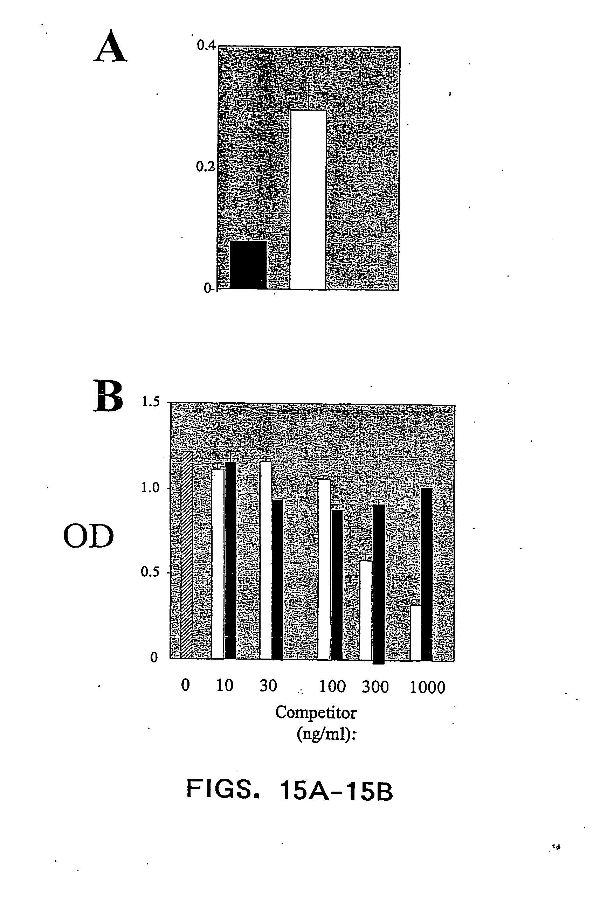

**FIGS. 15A-15B****:** EphA2 EA2 epitope is distinct from ligand binding site. (A) EphA2- Fₒ was incubated with and bound to immobilized Ephrin A1-F_{c}. Labeled Ephrin A1-Fₒ (black) or EA2 (white) was incubated with the EphA2-Ephrin A1-F_{c} complex and amount of binding was measured. **(B)** BphA2-F_{c} was incubated with and bound to immobilized Ephrin A1-F_{c}. Labeled EA2 was then incubated with the EphA2-Ephrin A1 complex. Unlabeled competitor was incubated with EphA2-Ephrin A1-EA2 complex in the indicated amount. Competitors were Ephrin A1-F_{c} (black) or EA2 (white).

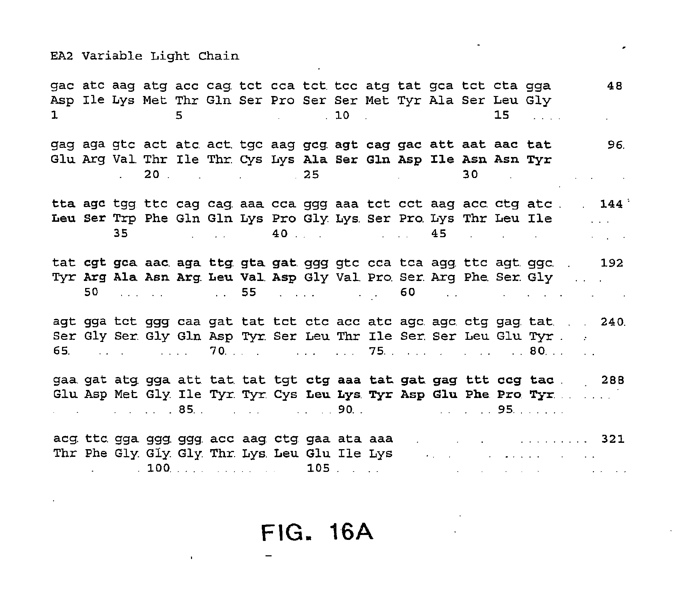

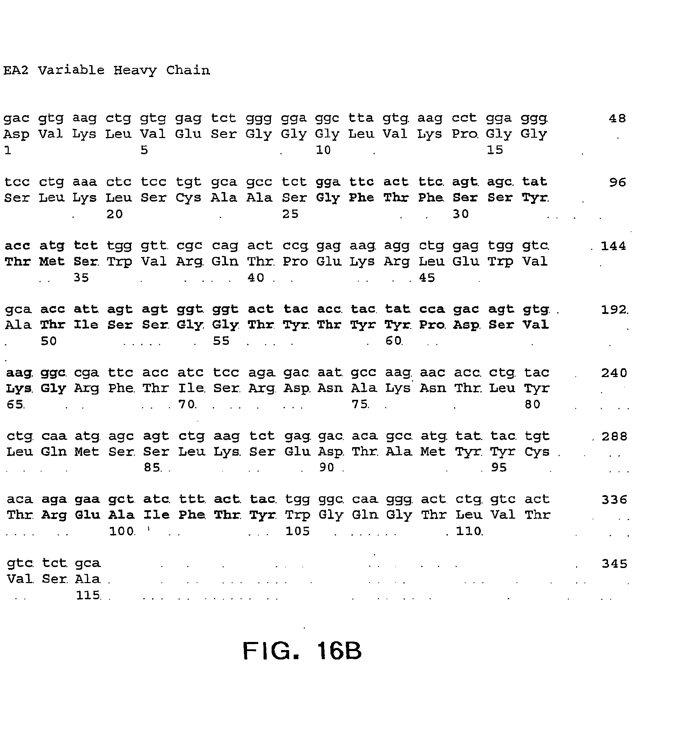

**FIG.16****:** Sequences of VL and VH of EA2. Amino acid and nucleic acid sequences of EA2 **(A)** VL (SEQ ID NOs:1 and 9, respectively) and **(B)** VH (SEQ ID NOs:5 and 13, respectively) are shown. Sequences of the CDRs are indicated.

### 5. DETAILED DESCRIPTION OF THE INVENTION

The present invention is based, in part, on the inventors' discovery that EphA2 monoclonal antibodies can inhibit cancer cell phenotypes. Decreased BphA2 activity selectively inhibits malignant cancer cell growth. Decreased EphA22 activity can be achieved with EphA2 agonistic monoclonal antibodies. Although not intending to be bound by any mechanism of action, this inhibition of malignant call growth is achieved by stimulating (*i.e.*, agonizing) BphA2 signaling thereby causing EphA2 phosphorylation which leads to its degradation. Malignant cell growth is decreased due to the decreased EphA2 levels and, therefore, ligand-independent EphA2 signaling.

Accordingly, the present invention relates to compositions that provide for the treatment, inhibition, and management of cancer, particularly metastatic cancer. A particular aspect of the invention relates to compositions containing compounds that inhibit cancer cell proliferation and invasion, particularly those cancer cells that overexpress EphA2. The present invention further relates to compositions for the treatment, inhibition, or management of metastases of cancers of epithelial cell origin, especially human cancers of the breast, lung, skin, and prostate. Further compositions of the invention include other types of active ingredients in combination with the EphA2 antibodies of the invention.

The present invention also relates to compositions for the treatment, inhibition, and management of cancer that has become partially or completely refractory to current or standard cancer treatment, such as chemotherapy, radiation therapy, hormonal therapy, and biological therapy.

The invention further provides diagnostic methods using the EphA2 antibodies of the invention, particularly the exposed EphA2 epitope antibodies, to evaluate the efficacy of cancer treatment, either EphA2-based or not EphA2-based. The diagnostic methods of the invention can also be used to prognose or predict cancer progression. In particular embodiments, the diagnostic methods of the invention provide antibodies for imaging and localizing metastases and for diagnosis and prognosis using tissues and fluids distal to the primary tumor site (as well as methods using tissues and fluids of the primary tumor In other embodiments, the diagnostic methods of the invention provide antibodies for imaging and localizing metastases and for diagnosis and prognosis *in vivo*.

### 5.1 Antibodies

As discussed above, the invention encompasses administration of antibodies (preferably monoclonal antibodies) or fragments thereof that immunospecifically bind to and agonize EphA2 signaling ("EphA2 agonistic antibodies") and preferentially bind epitopes on EphA2 that are selectively exposed or increased on cancer cells but not non-cancer cells ("exposed EphA2 epitope antibodies"). In one embodiment, the antibody binds to the extracellular domain of EphA2 and, also agonizes EphA2, *e.g.*, increases EphA2 phosphorylation. In another embodiment, the antibody binds to the extracellular domain of EphA2 and, preferably, also binds an epitope on EphA2 that is selectively exposed or increased on cancer cells but not non-cancer cells. In other embodiments, the antibody of the invention immunospecifically binds to and agonizes EphA2 signaling and preferentially binds an epitope on BphA2 that is selectively exposed or increased on cancer cells but not non-cancer cells and may or may not compete for binding with an EphA2 ligand, *e.g.*, Ephrin A1.

Hybridomas producing antibodies EA2 (strain EA2.31) and EA5 (strain EA5.12) of the invention have been deposited with the American Type Culture Collection (ATCC, P.O. Box 1549, Manassas, VA 20108) on May 22, 2002 under the provisions of the Budapest Treaty on the International Recognition of the Deposit of Microorganisms for the Purposes of Patent Procedures, and assigned accession numbers PTA-4380 and PTA-4381, The amino acid sequence of VL and VH of a preferred antibody is shown in FIGS. 16A-16B. The sequences of the preferred antibodies CDRs are indicated in Table 1. In a most preferred embodiment, the antibody is human or has been humanized.

Antibodies used in the methods of the invention include, but are not limited to, monoclonal antibodies, synthetic antibodies, multispecific antibodies (including bi-specific antibodies), human antibodies, humanized antibodies, chimeric antibodies, single-chain Fvs (scFv) (including bi-specific scfvs), single chain antibodies, Fab fragments, F(ab') fragments, disulfide-linked Fvs (sdFv), and epitope-binding fragments of any of the above. In particular, antibodies used in the methods of the present invention include immunoglobulin molecules and immunologically active portions of immunoglobulin molecules, *i.e.*, molecules that contain an antigen binding site that immunospecifically binds to EphA2 and is an agonist of EphA2 and/or preferentially binds an EphA2 epitope exposed on cancer cells but not non-cancer cells. The immunoglobulin molecules of the invention can be of any type (*e.g.*, IgG, IgE, IgM, IgD, IgA and IgY), class (*e.g.*, IgG₁, IgG₂, IgG₃, IgG₄, IgA₁ and IgA₂) or subclass of immunoglobulin molecule.

The antibodies used in the methods of the invention may be from any animal origin including birds and mammals (*e.g.*, human, murine, donkey, sheep, rabbit, goat, guinea pig, camel horse, or chicken). Preferably, the antibodies are human or humanized monoclonal antibodies. As used herein, "human" antibodies include antibodies having the amino acid sequence of a human immunoglobulin and include antibodies isolated from human immunoglobulin libraries or from mice or other animal that express antibodies from human genes.

The antibodies used in the methods of the present invention may be monospecific, bispecific, trispecific or of greater multispecificity. Multispecific antibodies may immunospecifically bind to different epitopes of an BphA2 polypeptide or may immunospecifically bind to both an EphA2 polypeptide as well a heterologous epitope, such as a heterologous polypeptide or solid support material. See, *e.g.*, International Publication Nos. WO 93/17715, WO 92/08802, WO 91/00360, and WO 92/05793; Tutt, et al., 1991, J. Immunol. 147:60-69; U.S. Patent Nos. 4,474,893, 4,714,681, 4,925,648, 5,573,920, and 5,601,819; and Kostelny et al.,1992, J. Immunol. 148:1547-1553.

In a specific embodiment, an antibody used the present invention is an antibody or an antigen-binding fragment thereof (*e.g.*, one or more complementarity determining regions (CDRs) of the afore-mentioned antibodies of the invention *e.g.*, see Table 1). In another embodiment, an agonistic antibody used in the present invention binds to the same epitope as a disclosed antibody
or competes with a disclosed antibody for binding to EphA2, *e.g.*, in an ELISA assay.

The present invention also encompasses antibodies or fragments thereof that immunospecifically bind to EphA2 and agonize EphA2 and/or preferentially bind an EphA2 epitope exposed in cancer cells.
The present invention also encompasses the use of antibodies that immunospecifically bind to BphA2 and agonize EphA2 and/or preferentially bind an BphA2 epitope exposed in cancer cells. The present invention also encompasses the use of antibodies that immunospecifically bind to EphA2 and agonize EphA2 and preferentially bind an EphA2 epitope exposed in cancer cells, said antibodies comprising one or more VH CDRs and one or more VL CDRs of PTA-4380 or PTA-4381. In particular, the invention encompasses the use of antibodies that immunospecifically bind to EphA2 and agonize EphA2 and/or preferentially bind an EphA2 epitope exposed in cancer cells, said antibodies comprising a VH CDR1 and a VL CDR1; a VH CDR1 and a VL CDR2; a VH CDR1 and a VL CDR3; a VH CDR2 and a VL CDR1; VH CDR2 and VL CDR2; a VH CDR2 and a VL CDR3; a VH CDR3 and a VL CDR1; a VH CDR3 and a VL CDR2; a VH CDR3 and a VL CDR3; a VH1 CDR1, a VH CDR2 and a VL CDR1; a VH CDR1, a VH CDR2 and a VL CDR2; a VH CDR1, a VH CDR2 and a VL CDR3; a VH CDR2, a VH CDR3 and a VL CDR1, a VH CDR2, a VH CDR3 and a VL CDR2; a VH CDR2, a VH CDR3 and a VL CDR3; a VH1 CDR1, a VH CDR3 and a VL CDR1; a VH CDR1, a VH CDR3 and a VL CDR2; a VH CDR1, a VH CDR3 and a VL CDR3; a VH CDR1, a VL CDR1 and a VL CDR2; a VH CDR1, a VL CDR1 and a VL CDR3; a VH CDR1, a VL CDR2 and a VL CDR3; a VH CDR2, a VL CDR1 and a VL CDR2; a VH CDR2, a VL CDR1 and a VL CDR3; a VH CDR2, a VL CDR2 and a VL CDR3; a VH CDR3, a VL CDR1 and a VL CDR2; a VH CDR3, a VL CDR1 and a VL CDR3; a VH CDR3, a VL CDR2 and a VL CDR3; a VH CDR1, a VH CDR2, a VH CDR3 and a VL CDR1; a VH CDR1, a VH CDR2, a VH CDR3 and a VL CDR2; a VH CDR1, a VH CDR2, a VH CDR3 and a VL CDR3; a VH CDR1, a VL CDR1, a VL CDR2 and a VL CDR3; a VH CDR2, a VL CDR1, a VL CDR2 and a VL CDR3; a VH CDR3, a VL CDR1, a VL CDR2 and a VL CDR3; a VH CDR1, a VH CDR2, a VL CDR1 and a VL CDR2; a VH CDR1, a VH CDR2, a VL CDR1 and a VL CDR3; a VH CDR1, a VH CDR2, a VL CDR2 and a VL CDR3; a VH CDR1, a VH CDR3, a VL CDR1 and a VL CDR2; a VH CDR1, a VH CDR3, a VL CDR1 and a VL CDR3; ; a VH CDR1, a VH CDR3, a VL CDR2 and a VL CDR3; a VH CDR2, a VH CDR3, a VL CDR1 and a VL CDR2; a VH CDR2, a VH CDR3, a VL CDR1 and a VL CDR3; a VH CDR2, a VH CDR3, a VL CDR2 and a VL CDR3; a VH CDR1, a VH CDR2, a VH CDR3, a VL CDR1 and a VL CDR2; a VH CDR1, a VH CDR2, a VH CDR3, a VL CDR1 and a VL CDR3; a VH CDR1, a VH CDR2, a VH CDR3, a VL CDR2 and a VL CDR3; a VH CDR1, a VH CDR2, a VL CDR1, a VL CDR2, and a VL CDR3; a VH CDR1, a VH CDR3, a VL CDR1, a VL CDR2, and a VL CDR3; a VH CDR2, a VH CDR3, a VL CDR1, a VL CDR2, and a VL CDR3; a VH CDR1, a VH CDR2, a VH CDR3, a VL CDR1, a VL CDR2, and a VL CDR3 or any combination thereof of the VH CDRs and VL CDRs of EA2, EA3, EA4, or EA5. In specific embodiments, the VH CDR1 is SEQ ID NO:6; the VH CDR2 is SEQ ID NO:7; the VH CDR3 is SEQ ID NO:8; the VL CDR1 is SEQ ID NO:2; the VL CDR2 is SEQ ID NO:3; and the VL CDR3 is SEQ ID NO:4 (see, *e.g.*, Table 1). The invention also encompasses any of the foregoing with one, two, three, four, or five amino acid substitutions, additions, or deletions that bind EphA2.

In one embodiment, an antibody that immunospecifically binds to EphA2 and agonizes EphA2 and/or preferentially binds an EphA2 epitope exposed in cancer cells comprises a VH CDR1 having the amino acid sequence of SEQ ID NO:6 and a VL CDR1 having the amino acid sequence of SEQ ID NO:2. In another embodiment, an antibody that immunospecifically binds to EphA2 and agonizes EphA2 and/or preferentially binds an EphA2 epitope exposed in cancer cells comprises a VH CDR1 having the amino acid sequence of SEQ ID NO:6 and a VL CDR2 having the amino acid sequence of SEQ ID NO:3. In another embodiment, an antibody that immunospecifically binds to EphA2 and agonizes EphA2 and/or preferentially binds an EphA2 epitope exposed in cancer cells comprises a VH CDR1 having the amino acid sequence of SEQ ID NO:6 and a VL CDR3 having the amino acid sequence of SEQ ID NO:4.

In another embodiment, an antibody that immunospecifically binds to EphA2 and agonizes EphA2 and/or preferentially binds an EphA2 epitope exposed in cancer cells comprises a VH CDR2 having the amino acid sequence of SEQ ID NO:7 and a VL CDR1 having the amino acid sequence of SEQ ID NO:2. In another embodiment, an antibody that immunospecifically binds to EphA2 and agonizes EphA2 and/or preferentially binds an EphA2 epitope exposed in cancer cells comprises a VH CDR2 having the amino acid sequence of SEQ ID NO:7 and a VL CDR2 having the amino acid sequence of SEQ ID NO:3. In another embodiment, an antibody that immunospecifically binds to EphA2 and agonizes EphA2 and/or preferentially binds an EphA2 epitope exposed in cancer cells comprises a VH CDR2 having the amino acid sequence of SEQ ID NO:7 and a VL CDR3 having the amino acid sequence of SEQ ID NO:4.

In another embodiment, an antibody that immunospecifically binds to EphA2 and agonizes EphA2 and preferentially binds an EphA2 epitope exposed in cancer cells comprises a VH CDR3 having the amino acid sequence of SEQ ID NO:8 and a VL CDR1 having the amino acid sequence of SEQ ID NO:2. In another embodiment, an antibody that immunospecifically binds to EphA2 and agonizes EphA2 and preferentially binds an EphA2 epitope exposed in cancer cells comprises a VH CDR3 having the amino acid sequence of SEQ ID NO:8 and a VL CDR2 having the amino acid sequence of SEQ ID NO:3. In another embodiment, an antibody that immunospecifically binds to EphA2 and agonizes EphA2 and preferentially binds an EphA2 epitope exposed in cancer cells comprises a VH CDR3 having the amino acid sequence of SEQ ID NO:8 and a VL CDR3 having the amino acid sequence of SEQ ID NO:4.

The antibodies used in the invention include derivatives that are modified, *i.e.*, by the covalent attachment of any type of molecule to the antibody. For example, but not by way of limitation, the antibody derivatives include antibodies that have been modified, *e.g.*, by glycosylation, acetylation, pegylation, phosphorylation, amidation, derivatization by known protecting/blocking groups, proteolytic cleavage, linkage to a cellular ligand or other protein, etc. Any of numerous chemical modifications may be carried out by known techniques, including, but not limited to, specific chemical cleavage, acetylation, formylation, metabolic synthesis of tunicamycin, etc. Additionally, the derivative may contain one or more non-classical amino acids.

The present invention also provides antibodies of the invention or fragments thereof that comprise a framework region known to those of skill in the art. Preferably, the antibody of the invention or fragment thereof is human or humanized.

The present invention encompasses single domain antibodies, including camelized single domain antibodies (see *e.g.*, Muyldermans et al., 2001, Trends Biochem. Sci. 26:230; Nuttall et al., 2000, Cur. Pharm. Biotech. 1:253; Reichmann and Muyldermans, 1999, J: Immunol. Meth. 231:25; International Publication Nos. WO 94/04678 and WO 94/25591; U.S. Patent No. 6,005,079;
In one embodiment, the present invention provides single domain antibodies comprising two VH domains of an EphA2 agonistic antibody or preferably EphA2 antibody that preferentially binds an BphA2 epitope exposed on cancer cells but not non-cancer cells) with modifications such that single domain antibodies are formed.

The present invention also encompass the use of antibodies or fragments thereof that have half-lives (*e.g*., serum half-lives) in a mammal, preferably a human, of greater than 15 days, preferably greater than 20 days, greater than 25 days, greater than 30 days, greater than 35 days, greater than 40 days, greater than 45 days, greater than 2 months, greater than 3 months, greater than 4 months, or greater than 5 months. The increased half-lives of the antibodies of the present invention or fragments thereof in a mammal, preferably a human, result in a higher serum titer of said antibodies or antibody fragments in the mammal, and thus, reduce the frequency of the administration of said antibodies or antibody fragments and/or reduces the concentration of said antibodies or antibody fragments to be (administered. Antibodies or fragments thereof having increased *in vivo* half-lives can be generated by techniques known to those of skill in the art. For example, antibodies or fragments thereof with increased *in vivo* half-lives can be generated by modifying (e.g., substituting, deleting or adding) amino acid residues identified as involved in the interaction between the Fc domain and the FcRn receptor (see, *e.g.,* International Publication Nos. WO 97/34631 and WO 02/060919, or fragments thereof with increased *in vivo* half-lives can be generated by attaching to said antibodies or antibody fragments polymer molecules such as high molecular weight polyethyleneglycol (PEG). PEG can be attached to said antibodies or antibody fragments with or without a multifunctional linker either through site-specific conjugation of the PEG to the N- or C- terminus of said antibodies or antibody fragments or via epsilon-amino groups present on lysine residues. Linear or branched polymer derivatization that results in minimal loss of biological activity will be used. The degree of conjugation will be closely monitored by SDS-PAGE and mass spectrometry to ensure proper conjugation of PEG molecules to the antibodies. Unreacted PEG can be separated from antibody-PEG conjugates by, *e.g.*, size exclusion or ionexchange chromatography.

The present invention also encompasses the use of antibodies or antibody fragments comprising the amino acid sequence of one or both variable domains of EA2, EA3, EA4, or EA5 with mutations (*e.g.*, one or more amino acid substitutions) in the framework or variable regions. Preferably, mutations in these antibodies maintain or enhance the avidity and/or affinity of the antibodies for the particular antigen(s) to which they immunospecifically bind. Standard techniques known to those skilled in the art (*e.g.*, immunoassays) can be used to assay the affinity of an antibody for a particular antigen.

Standard techniques known to those skilled in the art can be used to introduce mutations in the nucleotide sequence encoding an antibody, or fragment thereof, including, e.g., site-directed mutagenesis and PCR-mediated mutagenosis, which results in amino acid substitutions. Preferably, the derivatives include less than 15 amino acid substitutions, less than 10 amino acid substitutions, less than 5 amino acid substitutions, less than 4 amino acid substitutions, less than 3 amino acid substitutions, or less than 2 amino acid substitutions relative to the original antibody or fragment thereof. In a preferred embodiment, the derivatives have conservative amino acid substitutions made at one or more predicted non-essential amino acid residues.

The present invention also encompasses antibodies or fragments thereof that immunospecifically bind to EphA2 and agonize EphA2 and/or preferentially bind an EphA2 epitope exposed in cancer cells, said antibodies or antibody fragments comprising an amino acid sequence of a variable light chain and/or variable heavy chain that is at least 45%, at least 50%, at least 55%, at least 60%, at least 65%, at least 70%, at least 75%, at least 80%, at least 85%, at least 90%, at least 95%, or at least 99% identical to the amino acid sequence of the variable light chain and/or heavy chain of PTA-4380 or PTA-4381. In some embodiments, antibodies or antibody fragments of the invention immunospecifically bind to EphA2 and comprise an amino acid sequence of a variable light chain that is at least 45%, at least 50%, at least 55%, at least 60%, at least 65%, at least 70%, at least 75%, at least 80%, at least 85%, at least 90%, at least 95%, or at least 99% identical to SEQ ID NO:1. In other embodiments, antibodies or antibody fragments of the invention immunospecifically bind to EphA2 and comprise an amino acid sequence of a variable heavy chain that is at least 45%, at least 50%, at least 55%, at least 60%, at least 65%, at least 70%, at least 75%, at least 80%, at least 85%, at least 90%, at least 95%, or at least 99% identical to SEQ ID NO:5. In other embodiments, antibodies or antibody fragments of the invention immunospecifically bind to EphA2 and comprise an amino acid sequence of a variable light chain that is at least 45%, at least 50%, at least 55%, at least 60%, at least 65%, at least 70%, at least 75%, at least 80%, at least 85%, at least 90%, at least 95%, or at least 99% identical to SEQ ID NO:1 and a variable heavy chain that is at least 45%, at least 50%, at least 55%, at least 60%, at least 65%, at least 70%, at least 75%, at least 80%, at least 85%, at least 90%, at least 95%, or at least 99% identical to SEQ ID NO:5.

The determination of percent identity of two amino acid sequences can be determined by any method known to one skilled in the art, including BLAST protein searches.

The present invention further encompasses antibodies or fragments thereof that immunospecifically bind to EphA2 and agonize EphA2 and/or preferentially bind an EphA2 epitope exposed in cancer cells, said antibodies or antibody fragments comprising an amino acid sequence of 6 CDRs comprising amino acid residue substitutions, deletions or additions as compared to SEQ ID NO: 2, 3, 4, 6, 7, or 8. The antibody comprising the one or more CDRs comprising amino acid residue substitutions, deletions or additions may have substantially the same binding, better binding, or worse binding when compared to an antibody comprising one or more CDRs without amino acid residue substitutions, deletions or additions. In specific embodimemts, one, two a three, amino acid residues of the CDR have been substituted, deleted or added (*i.e.*, mutated).

The present invention also encompasses antibodies or the use of antibodies or antibody, fragments that immunospecifically bind to EphA2 and agonize EphA2 and preferentially bind epitopes on EphA2 that are selectively exposed or increased on cancer cells but not non-cancer cells, where said antibodies or antibody fragments are encoded by a nucleotide sequence that hybridizes to the nucleotide sequence of PTA-4380 or PTA-4381 under stringent conditions. In one embodiment, the invention provides antibodies or fragments thereof that immunospecifically bind to EphA2 and agonize EphA2 and/or preferentially bind an epitope on EphA2 that is selectively exposed or increased on cancer cells but not non-cancer cells, said antibodies or antibody fragments comprising a variable light chain encoded by a nucleotide sequence that hybridizes under stringent conditions to the nucleotide sequence of the variable light chain of PTA-4380 or PTA-4381.
In a preferred embodiments, the antibodies or fragments of the invention immunospecifically bind to EphA2 and comprise a CDR encoded by a nucleotide sequence that hybridizes under stringent conditions the nucleotide sequence of SEQ ID NO:10, 11, or 12,
and comprise a CDR encoded by a nucleotide sequence that hybridizes under stringent conditions the nucleotide sequence of SEQ ID NO:14, 15, or 16.

Stringent hybridization conditions include, but are not limited to, hybridization to filter-bound DNA in 6X sodium chloride/sodium citrate (SSC) at about 45°C followed by one or more washes in 0.2X SSC/0.1% SDS at about 50-65°C, highly stringent conditions such as hybridization to filter-bound DNA in 6X SSC at about 45°C followed by one or more washes in 0.1X SSC/0.2% SDS at about 60°C, or any other stringent hybridization conditions known to those skilled in the art (see, for example, Ausubel, F.M. et al., eds. 1989 Current Protocols in Molecular Biology, vol. 1, Green Publishing Associates, Inc. and John Wiley and Sons, Inc., NY at pages 6.3.1 to 6.3.6 and 2.10.3).

The present invention further encompasses antibodies or fragments thereof that immunospecifically bind to EphA2 and agonize EpbA2 and preferentially bind an EphA2 epitope exposed in cancer cells, said antibodies or antibody fragments said antibodies or antibody fragments comprising one or more CDRs encoded by a nucleotide sequence of one or more CDRs comprising nucleic acid residue substitutions, deletions or additions as compared to SEQ ID NO:10, 11, 12, 14, 15, or 16. The antibody comprising the one or more CDRs comprising nucleic acid residue substitutions, deletions or additions may have substantially the same binding, better binding, or worse binding when compared to an antibody comprising one or more CDRs without nucleic acid residue substitutions, deletions or additions. In specific embodiments, one, two, three, four, five, six, seven, eight, nine, ten, eleven, twelve, thirteen, fourteen, or fifteen nucleic acid residues of the CDR have been substituted, deleted or added (i.e., mutated). The nucleic acid substitutions may or may not change the amino acid sequence of the mutated CDR.

**TABLE 1**

| **Antibody** | **V chain** | **CDR** | **SEQ ID NO. (amino acids)** | **SEQ ID NO. (nucleic acids)** | **ATCC Deposit No.** |
|---|---|---|---|---|---|
| EA2 | | | | | PTA-4380 |
| | VL | | 1 | 9 | |
| | | VL1 | 2 | 10 | |
| | | VL2 | 3 | 11 | |
| | | VL3 | 4 | 12 | |
| | VH | | 5 | 13 | |
| | | VH1 | 6 | 14 | |
| | | VH2 | 7 | 15 | |
| | | VH3 | 8 | 16 | |

### 5.1.1 Antibody conjugates

The present invention encompasses the use of antibodies or fragments thereof recombinantly fused or chemically conjugated (including both covalent and noncovalent conjugations) to a heterologous polypeptide (or portion thereof, preferably to a polypeptide of at least 10, at least 20, at least 30, at least 40, at least 50, at least 60, at least 70, at least 80, at least 90 or at least 100 amino acids) to generate fusion proteins. The fusion does not necessarily need to be direct, but may occur through linker sequences. For example, antibodies may be used to target heterologous polypeptides to particular cell types, either *in vitro* or *in vivo,* by fusing or conjugating the antibodies to antibodies specific for particular cell surface receptors. Antibodies fused or conjugated to heterologous polypeptides may also be used *in in vitro* immunoassays and purification methods using methods known in the art. See e.g., International Publication WO 93/21232; EP 439,095; Naramura et al., 1994, Immunol. Lett. 39:91-99; U.S. Patent 5,474,981; Gillies et al., 1992, PNAS 89:1428-1432; and Fell et al., 1991, J. Immunol. 146:2446-2452,
In some embodiments, the disorder to be detected, treated, managed, or monitored is malignant cancer that overexpresses EphA2. In other embodiments, the disorder to be detected, treated, managed, or monitored is a pre-cancerous condition associated with cells that overexpress EphA2. In a specific embodiments, the pre-cancerous condition is high-grade prostatic intraepithelial neoplasia (PIN), fibroadenoma of the breast, fibrocystic disease, or compound nevi.

The present invention further includes compositions comprising heterologous polypeptides fused or conjugated to antibody fragments. For example, the heterologous polypeptides may be fused or conjugated to a Fab fragment, Fd fragment, Fv fragment, F(ab)₂ fragment, or portion thereof. Methods for fusing or conjugating polypeptides to antibody portions are known in the art. See, e.g., U.S. Patent Nos. 5,336,603. 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337- 11341

Additional fusion proteins, may be generated through the techniques of gene-shuffling, motif-shuffling, exon-shuffling, and/or codon-shuffling (collectively referred to as "DNA shuffling"). DNA shuffling may be employed to alter the activities of antibodies of the invention or fragments thereof (e.g., antibodies or fragments thereof with higher affinities and lower dissociation rates). See, generally, U.S. Patent Nos. 5,605,793; 5,811,238; 5,830,721; 5,834,252; and 5,837,458, and Patten et al., 1997, Curr. Opinion Biotechnol. 8:724-33; Harayama, 1998. Trends Btoiechnol. 16:76; Hansson, et al., 1999, J. Mol. Biol. 287:265; and Lorenzo and Blasco, 1998, BioTechniques 24:308 Antibodies or fragments thereof, or the encoded antibodies or fragments thereof, may be altered by being subjected to random mutagenesis by error-prone PCR, random nucleotide insertion or other methods prior to recombination. One or more portions of a polynucleotide encoding an antibody or antibody fragment, which portions immunospecifically bind to EphA2 may be recombined with one or more components, motifs, sections, parts, domains, fragments, etc. of one or more heterologous molecules.

Moreover, the antibodies or fragments thereof can be fused to marker sequences, such as a peptide to facilitate purification. In preferred embodiments, the marker amino acid sequence is a hexa-histidine peptide, such as the tag provided in a pQB vector (QIAGEN, Inc., 9259 Eton Avenue, Chatsworth, CA, 91311), among others, many of which are commercially available. As described in Gentz et al.,1989, *PNAS* 86:821, for instance, haxa-histidine provides for convenient purification of the fusion protein. Other peptide tags useful for purification include, but are not limited to, the hemagglutinin "HA" tag, which corresponds to an epitope derived from the influenza hemagglutinin protein (Wilson et al., 1984, Cell 37:767) and the "flag" tag.

In other embodiments, antibodies of the present invention or fragments or variants thereof are conjugated to a diagnostic or detectable agent Such antibodies can be useful for monitoring or prognosing the development or progression of a cancer as part of a clinical testing procedure, such as determining the efficacy of a particular therapy. Additionally, such antibodies can be useful for monitoring or prognosing the development or progression of a pre-cancarous condition associated with cells that overexpress EphA2 (e.g., high-grade prostatic intraepithelial neoplasia (PIN), fibroadenoma of the breast, fibrocystic disease ,or compound nevi). In one embodiment, an exposed EphA2 epitope antibody is conjugated to a diagnostic or detectable agent. In a more specific embodiment, the antibody is EA2, (PTA-4380).

Such diagnosis and detection can accomplished by coupling the antibody to detectable substances including, but not limited to various enzymes, such as but not limited to horseradish peroxidase, alkaline phosphatase, beta-galactosidase, or acetylcholinesterase; prosthetic groups, such as but not limited to streptavidin/biotin and avidin/biotin; fluorescent materials, such as but not limited to, umbelliferone, fluorescein, fluorescein isothiocynate, rhodamine, dichlorotriazinylamine fluorescein, dansyl chloride or phycoerythrin; luminescent materials, such as but not limited to, luminol; bioluminescent materials, such as but not limited to, luciferase, luciferin, and aequorin; radioactive materials, such as but not limited to, bismuth (²¹³Bi), carbon (¹⁴C), chromium (⁵¹Cr), cobalt (⁵⁷Co), fluorine (¹⁸F), gadolinium (¹⁵³Gd, ¹⁵⁹Gd), gallium (⁶⁸Ca, ⁶⁷Ga), germanium (⁶⁸Ge), holmium (¹⁶⁶Ho), indium (¹¹⁵In, ¹¹³In, ¹¹²In, ¹¹¹In), iodine (¹³¹I, ¹²⁵I, ¹²³I, ¹²¹I), lanthanium (¹⁴⁰La), lutetium (¹⁷⁷Lu), manganese (⁵⁴Mn), molybdenum (⁹⁹Mo), palladium (¹⁰³Pd), phosphorous (³²P), praseodymium (¹⁴²Pr), promethium (¹⁴⁹Pm), rhenium (¹⁸⁶Re, ¹⁸⁸Re), rhodium (¹⁰⁵Rh), ruthemium (⁹⁷Ru), samarium (¹⁵³Sm), scandium (⁴⁷Sc), selenium (⁷⁵Se), strontium (⁸⁵Sr), sulfur (³⁵S), technetium (⁹⁹Tc), thallium (²⁰¹Ti), tin (¹¹³Sn, ¹¹⁷Sn), tritium (³H), xenon (¹³³Xe), ytterbium (¹¹⁹Yb, ¹⁷⁵Yb), yttrium (⁹⁰Y), zinc (⁶⁵Zn); positron emitting metals using various positron emission tomographies, and nomadioactive paramagnetic metal ions.

The present invention further encompasses uses of antibodies or fragments thereof conjugated to a therapeutic agent

An antibody or fragment thereof may be conjugated to a therapeutic moiety such as a cytotoxin, *e.g*., a cytostatic or cytocidal agent, a therapeutic agent or a radioactive metal ion, e.g., alpha-emitters. A cytotoxin or cytotoxic agent includes any agent that is detrimental to cells. Examples include paclitaxel, cytochalasin B, gramicidin D, ethidium bromide, emetine, mitomycin, etoposide, tonoposide, vincristine, vinblastina, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, epirubicin, and cyclophosphamide and analogs or homologs thereof. Therapeutic agents include, but are not limited to, antimetabolites (*e.g*., methotrexate, 6-mercaptopurine, 6-thioguanine, cytarabine, 5-fluorouracil decarbazine), alkylating agents (*e.g*., mechlorethamine, thioepa chlorambucil, melphalan, carmustine (BCNU) and lomustine (CCNU), cyclothosphamide, busulfan, dibromomannitol, streptozotocin, mitomycin C, and cisdichlorodiamine platinum (II) (DDP) cisplatin), anthracyclines (*e.g*., daunorubicin (formerly daunomycin) and doxorubicin), antibiotics (*e.g*., dactinomycin (formerly actinomycin), bleomycin, mithramycin, and anthramycin (AMC)), and anti-mitotic agents (*e.g*., vincristine and vinblastine).

Further, an antibody or fragment thereof may be conjugated to a therapeutic agent or drug moiety that modifies a given biological response. Therapeutic agents or drug moieties are not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, cholera toxin, or diphtheria toxin; a protein such as tumor necrosis factor, α-interferon, β-interferon, nerve growth factor, platelet derived growth factor, tissue plasminogen activator, an apoptotic agent, *e.g*., TNF-α, TNF-β, AIM I (see, International Publication No. WO 97/33899), AIM II (see, International Publication No. WO 97/34911), Fas Ligand (Takahashi et al., 1994, J. Iminunol., 6:1567), and VEGI (see, International Publication No. WO 99/23105), a thrombotic agent or an anti-angiogenic agent, e.g., angiostatin or endostatin; or, a biological response modifier such as, for example, a lymphokine (*e.g*., interleukin-1 ("IL-1"), interleukin-2 ("IL-2"), interleukin-6 ("IL-6"), granulocyte macrophage colony stimulating factor ("GM-CSF"), and granulocyte colony stimulating factor ("G-CSF")), or a growth factor (*e.g*., growth hormone ("GH")).

Moreover, an antibody can be conjugated to therapeutic moieties such as a radioactive materials or macrocyclic chelators useful for conjugating radiometal ions (see above for examples of radioactive materials). In certain embodiments, the macrocyclic chelator is 1,4,7,10-tetraazacyclododecane-N,N',N",N"-tetraacetic acid (DOTA) which can be attached to the antibody via a linker molecule. Such linker molecules are commonly known in the art and described in Denardo et al., 1998, Clin Cancer Res. 4:2483-90; Peterson et al., 1999, Bioconjug. Chem. 10:553; and Zimmerman et al., 1999, Nucl. Med. Biol. 26:943-50

In a specific embodiment, the conjugated antibody is an EphA2 antibody that preferably binds an EphA2 epitope exposed on cancer cells but not on non-cancar cells (*i.e*., exposed EphA2 epitope antibody). In a more specific embodiment, the conjugated antibody is EA2 (PTA- 4380).

Techniques for conjugating therapeutic moieties to antibodies are well known. Moieties can be conjugated to antibodies by any method known in the art, including, but not limited to aldehyde/Schiff linkage, sulphydryl linkage, acid-labile linkage, cis-aconityl linkage, hydrazone linkage, enzymatically degradable linkage (see generally Garnett, 2002, Adv. Drug Deliv. Rev. 53:171-216). Additional techniques for conjugating therapeutic moieties to antibodies are well known, see, *e.g*., Arnon et al., "Monoclonal Antibodies For Immunotargeting Of Drugs In Cancer Therapy," in Monoclonal Antibodies And Cancer Therapy, Reisfeld at al. (eds.), pp. 243-56 (Alan R. Liss, Inc. 1985); Hellstrom et al., "antibodies For Drug Delivery," in Controlled Drug Delivery (2nd Ed.), Robinson et al. (eds.), pp. 623-53 (Marcel Dekker, Inc. 1987); Thorpe, "Antibody Carriers Of Cytotoxio Agents In Cancer Therapy: A Review," in Monoclonal Antibodies '84: Biological And Clinical Applications, Pinchera et al. (eds.), pp. 475-506 (1985*);* "Analysis, Results, And Future Prospective Of The Therapeutic Use Of Radiolabeled Antibody In Cancer Therapy," in Monoclonal Antibodies For Cancer Detection And Therapy, Baldwin et al. (eds.), pp. 303-16 (Academic Press 1985), and Thorpe et al., 1982, Immunol; Rev. 62:119-58. Methods for fusing or conjugating antibodies to polypeptide moieties are known in the art. See, e.g., U.S. Patent Nos. 5,336,603, 5,622,929, 5,359,046, 5,349,053, 5,447,851, and 5,112,946; EP 307,434; EP 367,166; International Publication Nos. WO 96/04388 and WO 91/06570; Ashkenazi et al., 1991, PNAS 88: 10535-10539; Zheng et al., 1995, J. Immunol. 154:5590-5600; and Vil et al., 1992, PNAS 89:11337-11341. The fusion of an antibody to a moiety does not necessarily need to be direct, but may occur through linker sequences. Such linker molecules are commonly known in the art and described in Denardo et al., 1998, Clin Cancer Res. 4:2483-90; Paterson et al., 1999, Bioconjug. Chem. 10:553; Zimmerman et al., 1999, Nucl. Med. Biol. 26:943-50; Garnett, 2002, Adv. Drug Deliv. Rev. 53:171-216,

Alternatively, an antibody can be conjugated to a second antibody to form an antibody heteroconjugate as described by Segal in U.S. Patent No. 4,676,980, is

Antibodies may also be attached to solid supports, which are particularly useful for immunoassays or purification of the target antigen. Such solid supports include, but are not limited to, glass, cellulose, polyacrylamide, nylon, polystyrene, polyvinyl chloride or polypropylene.

### 5.1.2 Methods Of Producing_Antibodies

The antibodies or fragments thereof can be produced by any method known in the art for the synthesis of antibodies, in particular, by chemical synthesis or preferably, by recombinant expression techniques.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) . The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with EphA2 (either the full length protein or a domain thereof, *e.g*., the extracellular or the ligand binding domain) and once an immune response is detected, *e.g*., antibodies specific for EphA2 are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. Hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, monoclonal antibodies can be generated by culturing a hybridoma cell secreting an antibody of the invention wherein, preferably, the hybridoma is generated by fusing splenocytes isolated from a mouse immunized with EphA2 or fragment thereof with myeloma cells and then screening the hybridomas resulting from the fusion for hybridoma clones that secrete an antibody able to bind EphA2.

Antibody fragments which recognize specific EphA2 epitopes may be generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, the antibodies of the present invention can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (*e.g.,* human or murine cDNA libraries of lymphoid tissues). The DNA encoding the VH and VL domains are recombined together with an scFv linker by PCR and cloned into a phagemid vector (*e.g*., p CANTAB 6 or pComb 3 HSS). The vector is electroporated in *E. coli* and the *E*. *coli* is infected with helper phage. Phage used in these methods are typically filamentous phage including fd and M13 and the VH and VL domains are usually recombinantly fused to either the phage gene III or gene VIII. Phage expressing an antigen binding domain that binds to the EphA2 epitope of interest can be selected or identified with antigen, *e.g*., using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177; Kettleborough et al., 1994, Eur. J. Immunol. 24:952-958; Persic et al., 1997, Gene 187:9; Burton et al., 1994, Advances in Immunology 57:191-280; International Application No. PCT/GB91/01134; International Publication Nos. WO 90/02809, WO 91/10737, WO 92/01047, WO 92/18619, WO 93/1 1236, WO 95/15982, WO 95/20401, and WO97/13844; and U.S. Patent Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108;

Phage may be screened for EphA2 binding, particularly to the extracellular domain of EphA2. Agonizing EphA2 activity (*e.g*., increasing EphA2 phosphorylation, reducing EphA2 levels) may also be screened.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described below. Techniques to recombinantly produce Fab, Fab' and F(ab')2 fragments can also be employed using methods known in the art such as those disclosed in International Publication No. WO 92/22324; Mullinax et al., 1992, BioTechniques 12:864; Sawai et al., 1995, AJRI 34:26*;* and Better et al., 1988, Science 240:1041

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, *e.g*., the human gamma 4 constant region, and the PCR amplified VL domains can be cloned into vectors expressing a VL constant region, e.g., human kappa or lambda constant regions. Preferably, the vectors for expressing the VH or VL domains comprise an EF-1α promoter, a secretion signal, a cloning site for the variable domain, constant domains, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, *e.g*., IgG, using techniques known to those of skill in the art.

For some uses, including *in vivo* use of antibodies in humans and *in vitro* detection assays, it may be preferable to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences. See also U.S. Patent Nos. 4,444,887 and 4,716,111; and International Publication Nos. WO 98/46645, WO 98/50433, WO 98/24893, WO 98/16654, WO 96/34096, WO 96/33735, and WO 91/10741:

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by homologous recombination. In particular, homozygous deletion of the J_{H} region prevents endogenous antibody production. The modified embryonic stem cells are expanded and microinjected into blastocysts to produce chimeric mice. The chimeric mice are then be bred to produce homozygous offspring which express human antibodies. The transgenic mice are immunized in the normal fashion with a selected antigen, *e.g*., all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, *see, e.g.,* International Publication Nos. WO 98/24893, WO 96/34096, and WO 96/33735; and U.S. Patent Nos. 5,413,923, 5,625,126, 5,633,425, 5,569,825, 5,661,016, 5,545,806, 5,814,318, and 5,939,598, . In addition, companies such as Abgenix, Inc. (Freemont, CA) and Medarex (Princeton, NJ) can be engaged to provide human antibodies directed against a selected antigen using technology similar to that described above.

A chimeric antibody is a molecule in which different portions of the antibody are derived from different immunoglobulin molecules such as antibodies having a variable region derived from a non-human antibody and a human immunoglobulin constant region. Methods for producing chimeric antibodies are known in the art. See *e.g*., Morrison, 1985, Science 229:1202; Oi et al., 1986, BioTechniques 4:214; Gillies et al., 1989, J. Immunol. Methods 125:191-202; and U.S. Patent Nos. 6,311,415, 5,807,715, 4,816,567, and 4,816,397. Chimeric antibodies comprising one or more CDRs from a non-human species and framework regions from a human immunoglobulin molecule can be produced using a variety of techniques known in the art including, for example, CDR-grafting (EP 239,400; International Publication No. WO 91/09967; and U.S. Patent Nos. 5,225,539, 5,530,101, and 5,585,089), veneering or resurfacing (EP 592,106; EP 519,596; Radian, 1991, Molecular Immunology 28(4/5):489-498; Studnicka et al, 1994, Protein Engineering 7:805; and Roguska et al.,1994, PNAS 91:969), and chain shuffling (U.S. Patent No. 5,565,332).
in a specific embodiment, a chimeric antibody of the invention immunospecifically binds EphA2 and comprises VL CDRs having the amino acid sequence of SEQ ID NO:2,3, and 4. embodiment, a chimeric antibody of the invention immunospecifically binds EphA2 and comprises a VH CDR having the amino acid sequence of SEQ ID NO:6,7, and 8.
In a specific preferred embodiment, a chimeric antibody of the invention immumospecifically binds EphA2 and comprises a VL CDR having an amino acid sequence of SEQ ID NO: 2,3, and 4 and further comprises a VH CDR having an amino acid sequence of SEQ ID NO:6,7, and 8.

Often, framework residues in the framework regions will be substituted with the corresponding residue from the CDR donor antibody to alter, preferably improve, antigen binding. These framework substitutions are identified by methods well known in the art, *e.g*., by modeling of the interactions of the CDR and framework residues to identify framework residues important for antigen binding and sequence comparison to identify unusual framework residues at particular positions. (See, *e.g*., U.S. Patent No. 5,585,089; and Riechmann et al., 1988, Nature 332:323,

A humanized antibody is an antibody or its variant or fragment thereof which is capable of binding to a predetermined antigen and which comprises a framework region having substantially the amino acid sequence of a human immunoglobulin and a CDR having substantially the amino acid sequence of a non-human immunoglobulin. A humanized antibody comprises substantially all of at least one, and typically two, variable domains in which all or substantially all of the CDR regions correspond to those of a non-human immunoglobulin (*i.e*., donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD, IgA and IgE, and any isotype, including IgG₁, IgG₂, IgG₃ and IgG₄. Usually the constant domain is a complement fixing constant domain where it is desired that the humanized antibody exhibit cytotoxic activity, and the class is typically IgG₁. Where such cytotoxic activity is not desirable, the constant domain may be of the IgG₂ class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, e.g., the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework region (FR) and CDR sequences, more often 90%, and most preferably greater than 95%. Humanized antibodies can be produced using variety of techniques known in the art, including but not limited to, CDR-grafting (European Patent No. EP 239,400; International Publication No. WO 91/09967; and U.S. Patent Nos. 5,225,539, 5,530,101, and 5,585,089), veneering or resurfacing (European Patent Nos. EP 592,106 and EP 519,596; Padlan, 1991, Molecular Immunology 28(4/5):489-498; Studnicka et al, 1994, Protein Engineering 7(6):805-814; and Roguska et al., 1994, PNAS 91:969-973), chain shuffling (U.S. Patent No. 5,565,332), and techniques disclosed in, *e.g*., U.S. Patent Nos. 6,407,213, 5,766,886, 5,585,089, International Publication No. WO 9317105, Tan et al., 2002, J. Immunol. 169:1119-25, Caldas et al., 2000, Protein Eng. 13:353-60, Morea et al., 2000, Methods 20:267-79, Baca et al., 1997, J. Biol. Chem. 272:10678-84, Roguska et al., 1996, Protein Eng. 9:895-904, Couto et al., 1995, Cancer Res. 55 (23 Supp):5973s-5977s, Couto et al., 1995, Cancer Res. 55:1717-22, Sandhu, 1994, Gene 150:409-10, Pedersen et al., 1994, J. Mol. Biol. 235:959-73, Jones et al., 1986, Nature 321:522-525, Riechmann et al., 1988, Nature 332:323, and Presta, 1992, Curr. Op. Struct. Biol. 2:593-596. Often, framework residues in the framework regions will be substituted with the corresponding residue from the CDR donor antibody to alter, preferably improve, antigen binding. These framework substitutions are identified by methods well known in the art, *e.g*., by modeling of the interactions of the CDR and framework residues to identify framework residues important for antigen binding and sequence comparison to identify unusual framework residues at particular positions. (*See, e.g.,* Queen et al., U.S. Patent No. 5,585,089; and Riechmann et al., 1988, Nature 332:323, ies.)

Further, the antibodies of the invention can, in turn, be utilized to generate anti-idiotype antibodies using techniques well known to those skilled in the art. (See, *e.g*., Greenspan & Bona, 1989, FASEB J. 7:437-444; and Nissinoff, 1991, J. Immunol. 147:2429-2438). The invention provides methods employing the use of polynucleotides comprising a nucleotide sequence encoding an antibody of the invention or a fragment thereof.

### 5.1.3 Polynucleotides Encoding An Antibody

The methods of the invention also encompass polynucleotides that hybridize under high stringency, intermediate or lower stringency hybridization conditions, *e.g*., as defined *supra,* to polynucleotides that encode an antibody of the invention.

The polynucleotides may be obtained, and the nucleotide sequence of the polynucleotides determined, by any method known in the art. Since the amino acid sequences of the antibodies are known, nucleotide sequences encoding these antibodies can be determined using methods well known in the art, *i.e*., nucleotide codons known to encode particular amino acids are assembled in such a way to generate a nucleic acid that encodes the antibody or fragment thereof of the invention. Such a polynucleotide encoding the antibody may be assembled from chemically synthesized oligonucleotides (*e.g*., as described in Kutmeier et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR

Alternatively, a polynucleotide encoding an antibody may be generated from nucleic acid from a suitable source. If a clone containing a nucleic acid encoding a particular antibody is not available, but the sequence of the antibody molecule is known, (see *e.g*., FIG. 16), a nucleic acid encoding the immunoglobulin may be chemically synthesized or obtained from a suitable source (*e.g*., an antibody cDNA library, or a cDNA library generated from, or nucleic acid, preferably poly A+ RNA, isolated from, any tissue or cells expressing the antibody, such as hybridoma cells selected to express an antibody of the invention, *e.g*., clone deposited in the ATCC as PTA-4380) by PCR amplification using synthetic primers hybridizable to the 3' and 5' ends of the sequence or by cloning using an oligonucleotide probe specific for the particular gene sequence to identify, *e.g*., a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, *e.g*., recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, NY and Ausubel et al., eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, and preferably human framework regions (see, *e.g.,* Chothia et al., 1998, J. Mol. Biol. 278: 457-479 for a listing of human framework regions). Preferably, the polynucleotide generated by the combination of the framework regions and CDRs encodes an antibody that specifically binds to EphA2. Preferably, as discussed *supra,* one or more amino acid substitutions may be made within the framework regions, and, preferably, the amino acid substitutions improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

### 5.1.4 Recombinant Expression Of An Antibody

Recombinant expression of an antibody of the invention, derivative, analog or fragment thereof, (*e.g*., a heavy or light chain of an antibody of the invention or a portion thereof or a single chain antibody of the invention), requires construction of an expression vector containing a polynucleotide that encodes the antibody. Once a polynucleotide encoding an antibody molecule or a heavy or light chain of an antibody, or portion thereof (preferably, but not necessarily, containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, *e.g*., International Publication Nos. WO 86/05807 and WO 89/01036; and U.S. Patent No. 5,122,464) and the variable domain of the antibody may be cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In preferred embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention (see, *e.g*., U.S. Patent No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention *in situ.* These include but are not limited to microorganisms such as bacteria (*e.g., E. coli* and *B. subtilis*) transformed with recombinant bacteriophage DNA, plasmid DNA or cosmid DNA expression vectors containing antibody coding sequences; yeast (*e.g., Saccharomyces Pichia*) transformed with recombinant yeast expression vectors containing antibody coding sequences; insect cell systems infected with recombinant virus expression vectors (*e.g*., baculovirus) containing antibody coding sequences; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing antibody coding sequences; or mammalian cell systems (*e.g*., COS, CHO, BHK, 293, NS0, and 3T3 cells) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter). Preferably, bacterial cells such as *Escherichia coli,* and more preferably, eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, BioTechnology 8:2). In a specific embodiment, the expression of nucleotide sequences encoding antibodies or fragments thereof which immunospecifically bind to and agonize is regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the *E. coli* expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing the antibody molecule in infected hosts (*e.g.,* see Logan & Shenk, 1984, PNAS 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, *e.g*., Bittner et al., 1987, Methods in Enzymol. 153:516-544).

In addition, a host cell strain may be chosen which modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERO, BHK, HeLa, COS, MDCK, 293, 3T3, W138, BT483, Hs578T, HTB2, BT20, NS1, and T47D, NS0 (a murine myeloma cell line that does not endogenously produce any immunoglobulin chains), CRL7O30 and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (*e.g*., promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), glutamine synthase, hypoxanthine guanine phosphoribosyltransferase (Szybalska & Szybalski, 1992, Proc. Natl. Acad. Sci. USA 48:202), and adenine phosphoribosyltransferase (Lowy et al., 1980, Cell 22:8-17) genes can be employed in tk-, gs-, hgprt- or aprt- cells, respectively. Also, antimetabolite resistance can be used as the basis of selection for the following genes: *dhfr*, which confers resistance to methotrexate (Wigler et al., 1980, PNAS77:357; O'Hare et al., 1981, PNAS 78:1527); *gpt*, which confers resistance to mycophenolic acid (Mulligan & Berg, 1981, PNAS 78:2072); neo, which confers resistance to the aminoglycoside G-418 (Wu and Wu, 1991, Biotherapy 3:87; Tolstoshev, 1993, Ann. Rev. Pharmacol. Toxicol. 32:573; Mulligan, 1993, Science 260:926; and Morgan and Anderson, 1993, Ann. Rev. Biochem. 62: 191; May, 1993, TIB TECH 11:155-); and *hygro,* which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150:1,

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, PNAS 77:2197). The coding sequences for the heavy and light chains may comprise cDNA or genomic DNA.

Once an antibody molecule of the invention has been produced by recombinant expression, it may be purified by any method known in the art for purification of an immunoglobulin molecules, for example, by chromatography (*e.g*., ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### 5.2 Prophylactic/Terapeutic Methods

The present invention encompasses antibodies for treating, preventing, or managing a disorder associated with overexpression of EphA2, preferably cancer, in a subject comprising administering one or more EphA2 agonistic antibodies and optionally exposed BphA2 epitope antibodies, preferably one or more monoclonal (or antibodies from some other source of a single antibody species) EphA2 agonistic antibodies and optionally exposed EphA2 epitope antibodies. In a specific embodiment, the disorder to be treated, prevented, or managed is malignant cancer. In another specific embodiment, the disorder to be treated, prevented, or managed is a pre-cancerous condition associated with cells that overexpress EphA22. In more specific embodiments, the pre-cancerous condition is high-grade prostatic intraepithelial neoplasia (PIN), fibroadenoma of the breast, fibrocystic disease, or compound nevi.

In one embodiment, the antibodies of the invention can be administered in combination with one or more other therapeutic agents useful in the treatment, prevention or management of cancer. In certain embodiments, one or more EphA2 antibodies of the invention are administered to a mammal, preferably a human, concurrently with one or more other therapeutic agents useful for the treatment of cancer. The term "concurrently" is not limited to the administration of prophylactic or therapeutic agents at exactly the same time, but rather it is meant that the EphA2 antibodies of the invention and the other agent are administered to a subject in a sequence and within a time interval such that the antibodies of the invention can act together with the other agent to provide an increased benefit than if they were administered otherwise. For example, each prophylactic or therapeutic agent may be administered at the same time or sequentially in any order at different points in time; however, if not (administered at the same time, they should be administered sufficiently close in time so as to provide the desired therapeutic or prophylactic effect. Each therapeutic agent can be administered separately, in any appropriate form and by any suitable route. In other embodiments, the EphA2 antibodies of the invention are administered before, concurrently or after surgery. Preferably the surgery completely removes localized tumors or reduces the size of large tumors. Surgery can also be done as a preventive measure or to relieve pain.

In various embodiments, the prophylactic or therapeutic agents are administered less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In preferred embodiments, two or more components are administered within the same patient visit.

The dosage amounts and frequencies of administration provided herein are encompassed by the terms therapeutically effective and prophylactically effective. The dosage and frequency further will typically vary according to factors specific for each patient depending on the specific therapeutic or prophylactic agents administered, the soverity and type of cancer, the route of administration, as well as age, body weight, response, and the past medical history of the patient Suitable regimens can be selected by one skilled in the art by considering such factors and by following, for example, dosages reported in the literature and recommended in the Physician's Desk Reference (56th ed, 2002).

### 5.2.1 Patient Population

The invention provides antibodies for treating, preventing, and managing cancer by administrating to a subject a therapeutically or prophylactically effective amount of one or more EphA2 antibodies of the invention. In another embodiment, the EphA22 antibodies of the invention can be administered in combination with one or more other therapeutic agents. The subject is preferably a mammal such as non-primate (*e.g*., cows, pigs, horses, cats, dogs, rats, etc.) and a primate (*e.g*., monkey, such as a cynomolgous monkey and a human). In a preferred embodiment, the subject is a human.

Specific examples of cancers that can be treated by the antibodies encompassed by the invention include, but are not limited to, cancers that over express EphA2. In a further embodiment, the cancer is of an epithelial origin. Examples of such cancers are cancer of the lung, colon, prostate, breast, and skin. Additional cancers are listed by example and not by limitation in the following section 5.2.1.1. In particular embodiments, methods of the invention can be used to treat and/or prevent metastasis from primary tumors.

The antibodies and compositions of the invention comprise the administration of one or more EphA2 antibodies of the invention to subjects/patients suffering from or expected to suffer from cancer, e.g., have a genetic predisposition for a particular type of cancer, have been exposed to a carcinogen, or are in remission from a particular cancer. As used herein, "cancer" refers to primary or metastatic, cancers. Such patients may or may not have been previously treated for cancer. The antibodies and compositions of the invention may be used as a first line or second line cancer treatment. Included m the,invention is also the treatment of patients undergoing other cancer therapies and the antibodies and compositions of the invention can be used before any adverse effects or intolerance of these other cancer therapies occurs. The invention also encompasses antibodies
to treat or ameliorate symptoms in refractory patients. In a certain embodiment, that a cancer is refractory to a therapy means that at least some significant portion of the cancer cells are not killed or their cell division arrested. The determination of whether the cancer cells are refractory can be made either *in vivo* or *in vitro* by any method known in the art for assaying the effectiveness of treatment on cancer cells, using the art-accepted meanings of "refractory" in such a context. In various embodiments, a cancer is refractory where the number of cancer cells has not been significantly reduced, or has increased. The invention also encompasses antibodies to prevent the onset or recurrence of cancer in patients predisposed to having cancer.

In particular embodiments, the EphA2 antibodies of the invention, are administered to reverse resistance or reduced sensitivity of cancer cells to certain hormonal, radiation and chemotherapeutic agents thereby resensitizing the cancer cells to one or more of these agents, which can then be administered (or continue to be administered) to treat or manage cancer, including to prevent metastasis.

In alternate embodiments, the invention provides antibodies for treating patients' cancer in combination with any other treatment or to patients who have proven refractory to other treatments but are no longer on these treatments. In certain embodiments, the patients being treated by the antibodies of the invention are patients already being treated with chemotherapy, radiation therapy, hormonal therapy, or biological therapy/immunotherapy. Among these patients are refractory patients and those with cancer despite treatment with existing cancer therapies. In other embodiments, the patients have been treated and have no disease activity and one or more agonistic antibodies of the invention are administered to prevent the recurrence of cancer.

In preferred embodiments, the existing treatment is chemotherapy. In particular embodiments, the existing treatment includes administration of chemotherapies including, but not limited to, methotrexate, taxol, mercaptopurine, thioguanine, hydroxyurea, cytarabine, cyclophosphamide, ifosfamide, nitrosoureas, cisplatin, carboplatin, mitomycin, dacarbazine, procarbizine, etoposides, campathecins, bleomycin, doxorubicin, idarubicin, daunorubicin, dactinomycin, plicamycin, mitoxantrone, asparaginase, vinblastine, vincristine, vinorelbine, paclitaxel, docetaxel, etc. Among these patients are patients treated with radiation therapy, hormonal therapy and/or biological therapy/immunotherapy. Also among these patients are those who have undergone surgery for the treatment of cancer.

Alternatively, the invention also encompasses antibodies for treating patient undergoing or having undergone radiation therapy. Among these are patients being treated or previously treated with chemotherapy, hormonal therapy and/or biological therapy/immunotherapy. Also among these patients are those who have undergone surgery for the treatment of cancer.

In other embodiments, the invention encompasses antibodies for treating patients undergoing or having undergone hormonal therapy and/or biological therapy/immunotherapy. Among these are patients being treated or having been treated with chemotherapy and/or radiation therapy. Also among these patients are those who have undergone surgery for the treatment of cancer.

Additionally, the invention also provides antibodies for treatment of cancer as an alternative to chemotherapy, radiation therapy, hormonal therapy, and/or biological therapy/immunotherapy where the therapy has proven or may prove too toxic, *i*.*e*., results in unacceptable or unbearable side effects, for the subject being treated. The subject being treated with the antibodies of the invention may, optionally, be treated with other cancer treatments such as surgery, chemotherapy, radiation therapy, hormonal therapy or biological therapy, depending on which treatment was found to be unacceptable or unbearable.

In other embodiments, the invention provides administration of one or more agonistic monoclonal antibodies of the invention without any other cancer therapies for the treatment of cancer, but who have proved refractory to such treatments. In specific embodiments, patients refractory to other cancer therapies are administered one or more agonistic monoclonal antibodies in the absence of cancer therapies.

In other embodiments, patients with a pre-cancerous condition associated with cells that overexpress EphA2 can be administered antibodies of the invention to treat the disorder and decrease the likelihood that it will progress to malignant cancer. In specific embodiments, the pro-cancerous condition is high-grade prostatic intraepithelial neoplasia (PIN), fibroadenoma of the breast, fibrocystic disease, or compound nevi.

### 5.2.1.1. Cancers

Cancers and related disorders that can be treated or prevented by methods and compositions of the present invention include but are not limited to cancers of an epithelial cell origin. Examples of such cancers include the following: leukemias, such as but not limited to, acute leukemia, acute lymphocytic leukemia, acute myelocytic leukemias, such as, myeloblastic, promyelocytic, myelomonocytic, monocytic, and erythroleukemia leukemias and myelodysplastic syndrome; chronic leukemias, such as but not limited to, chronic myelocytic (granulocytic) leukemia, chronic lymphocytic leukemia, hairy cell leukemia; polycythemia vera; lymphomas such as but not limited to Hodgkin's disease, non-Hodgkin's disease; multiple myelomas such as but not limited to smoldering multiple myeloma, nonsecretory myeloma, osteosclerotic myeloma, plasma cell leukemia, solitary plasmacytoma and extramedullary plasmacytoma; Waldenström's macroglobulinemia; monoclonal gammopathy of undetermined significance; benign monoclonal gammopathy; heavy chain disease; bone and connective tissue sarcomas such as but not limited to bone sarcoma, osteosarcoma, chondrosarcoma, Ewing's sarcoma, malignant giant cell tumor, fibrosarcoma of bone, chordoma, periosteal sarcoma, soft-tissue sarcomas, angiosarcoma (hemangiosarcoma), fibrosarcoma, Kaposi's sarcoma, leiomyosarcoma, liposarcoma, lymphangiosarcoma, neurilemmoma, rhabdomyosarcoma, synovial sarcoma; brain tumors such as but not limited to, glioma, astrocytoma, brain stem glioma, ependymoma, oligodendroglioma, nonglial tumor, acoustic neurinoma, craniopharyngioma, medulloblastoma, meningioma, pineocytoma, pineoblastoma, primary brain lymphoma; breast cancer including but not limited to adenocarcinoma, lobular (small cell) carcinoma, intraductal carcinoma, medullary breast cancer, mucinous breast cancer, tubular breast cancer, papillary breast cancer, Paget's disease, and inflammatory breast cancer; adrenal cancer such as but not limited to pheochromocytom and adrenocortical carcinoma; thyroid cancer such as but not limited to papillary or follicular thyroid cancer, medullary thyroid cancer and anaplastic thyroid cancer; pancreatic cancer such as but not limited to, insulinoma, gastrinoma, glucagonoma, vipoma, somatostatin-secreting tumor, and carcinoid or islet cell tumor; pituitary cancers such as but limited to Cushing's disease, prolactin-secreting tumor, acromegaly, and diabetes insipius; eye cancers such as but not limited to ocular melanoma such as iris melanoma, choroidal melanoma, and cilliary body melanoma, and retinoblastoma; vaginal cancers such as squamous cell carcinoma, adenocarcinoma, and melanoma; vulvar cancer such as squamous cell carcinoma, melanoma, adenocarcinoma, basal cell carcinoma, sarcoma, and Paget's disease; cervical cancers such as but not limited to, squamous cell carcinoma, and adenocarcinoma; uterine cancers such as but not limited to endometrial carcinoma and uterine sarcoma; ovarian cancers such as but not limited to, ovarian epithelial carcinoma, borderline tumor, germ cell tumor, and stromal tumor; esophageal cancers such as but not limited to, squamous cancer, adenocarcinoma, adenoid cystic carcinoma, mucoepidermoid carcinoma, adenosquamous carcinoma, sarcoma, melanoma, plasmacytoma, verrucous carcinoma, and oat cell (small cell) carcinoma; stomach cancers such as but not limited to, adenocarcinoma, fungating (polypoid), ulcerating, superficial spreading, diffusely spreading, malignant lymphoma, liposarcoma, fibrosarcoma, and carcinosarcoma; colon cancers; rectal cancers; liver cancers such as but not limited to hepatocellular carcinoma and hepatoblastoma; gallbladder cancers Such as adenocarcinoma; cholangiocarcinomas such as but not limited to pappillary, nodular, and diffuse; lung cancers such as non-small cell lung cancer, squamous cell carcinoma (epidermoid carcinoma), adenocarcinoma, large-cell carcinoma and small-cell lung cancer; testicular cancers such as but not limited to germinal tumor, seminoma, anaplastic, classic (typical), spermatocytic, nonseminoma, embryonal carcinoma, teratoma carcinoma, choriocarcinoma (yolk-sac tumor), prostate cancers such as but not limited to, adenocarcinoma, leiomyosarcoma, and rhabdomyosarcoma; penal cancers; oral cancers such as but not limited to squamous cell carcinoma; basal cancers; salivary gland cancers such as but not limited to adenocarcinoma, mucoepidermoid carcinoma, and adenoidcystic carcinoma; pharynx cancers such as but not limited to squamous cell cancer, and verrucous; skin cancers such as but not limited to, basal cell carcinoma, squamous cell carcinoma and melanoma, superficial spreading melanoma, nodular melanoma, lentigo malignant melanoma, acral lentiginous melanoma; kidney cancers such as but not limited to renal cell carcinoma adenocarcinoma, hypermephroma, fibrosarcoma, transitional cell cancer (renal pelvis and/ or uterer); Wilms' tumor; bladder cancers such as but not limited to transitional cell carcinoma, squamous cell cancer, adenocarcinoma, carcinosarcoma. In addition, cancers include myxosarcoma, osteogenic sarcoma, endotheliosarcoma, lymphangioendotheliosarcoma, mesothelioma, synovioma, hemangioblastoma, epithelial carcinoma, cystadenocarcinoma, bronchogenic carcinoma, sweat gland carcinoma, sebaceous gland carcinoma, papillary carcinoma and papillary adenocarcinomas (for a review of such disorders, see Fishman et al., 1985, Medicine, 2d Ed., J.B. Lippincott Co., Philadelphia and Murphy et aL, 1997, Informed Decisions: The Complete Book of Cancer Diagnosis, Treatment, and Recovery, Viking Penguin, Penguin Books U.S.A., Inc., United States of America).

Accordingly, the antibodies and compositions of the invention are also useful in the treatment or prevention of a variety of cancers or other abnormal proliferative diseases, including (but not limited to) the following: carcinoma, including that of the bladder, breast, colon, kidney, liver, lung, ovary, pancreas, stomach, cervix, thyroid and skin; including squamous cell carcinoma; hematopoietic tumors of lymphoid lineage, including leukemia, acute lymphocytic leukemia, acute lymphoblastic leukemia, B-cell lymphoma, T-cell lymphoma, Burkitt's lymphoma; hematopoietic tumors ofmyeloid lineage, including acute and chronic myelogenous leukemias and promyelocytic leukemia; tumors of mesenchymal origin, including fibrosarcoma and rhabdomyoscarcoma; other tumors, including melanoma, seminoma, tetratocarcinoma, neuroblastoma and glioma; tumors of the central and peripheral nervous system, including astrocytoma, neuroblastoma, glioma, and schwannomas; tumors of mesenchymal origin, including fibrosarcoma, rhabdomyoscarama, and osteosarcoma; and other tumors, including melanoma, xeroderma pigmentosum, keratoactanthoma, seminoma, thyroid follicular cancer and teratocarcinoma. It is also contemplated that cancers caused by aberrations in apoptosis would also be treated by the methods and compositions of the invention. Such cancers may include but not be limited to follicular lymphomas, carcinomas with p53 mutations, hormone dependent tumors of the breast, prostate and ovary, and precancerous lesions such as familial adenomatous polyposis, and myelodysplastic syndromes. In specific embodiments, malignancy or dysproliferative changes (such as metaplasias and dysplasias), or hyperproliferative disorders, are treated or prevented in the skin, lung, colon, breast, prostate, bladder, kidney, pancreas, ovary, or uterus. In other specific embodiments, sarcoma, melanoma, or leukemia is treated or prevented.

In some embodiments, the cancer is malignant and overexpresses EphA2. In other embodiments, the disorder to be treated is a pre-cancerous condition associated with cells that overexpress EphA2. In a specific embodiments, the pre-cancerous condition is high-grade prostatic intraepithelial neoplasia (PIN), fibroadenoma of the breast, fibrocystic disease, or compound nevi.

In preferred embodiments, the antibodies and compositions of the invention are used for the treatment and/or prevention of breast, colon, ovarian, lung, and prostate cancers and melanoma and are provided below by example rather than by limitation.

### 5.2.1.2. Treatment of Breast Cancer

In specific embodiments, patients with breast cancer are administered an effective amount of one or more monoclonal antibodies of the invention. In another embodiment, the antibodies of the invention can be administered in combination with an effective amount of one or more other agents useful for breast cancer therapy including but not limited to: doxorubicin, epirubicin, the combination of doxorubicin and cyclophosphamide (AC), the combination of cyclophosphamide, doxorubicin and 5-fluorouracil (CAF), the combination of cyclophosphamide, epirubicin and 5-fluorouracil (CEF), herceptin, tamoxifen, the combination of tamoxifen and cytotoxic chemotherapy, taxanes (such as docetaxel and paclitaxel). In a further embodiment, antibodies of the invention can be administered with taxanes plus standard doxorubicin and cyclophosphamide for adjuvant treatment of node-positive, localized breast cancer.

In a specific embodiment, patients with pre-cancerous fibroadenoma of the breast or fibrocystic disease are administered an EphA2 antibody of the invention to treat the disorder and decrease the likelihood that it will progress to malignant breast cancer.

### 5.2.1.3. Treatment of Colon Cancer

In specific embodiments, patients with colon cancer are administered an effective amount of one or more monoclonal antibodies of the invention. In another embodiment, the antibodies of the invention can be administered in combination with an effective amount of one or more other agents useful for colon cancer therapy including but not limited to: the combination of 5-FU and leucovorin, the combination of 5-FU and levamisole, irinotecan (CPT-11) or the combination of irinotecan, 5-FU and leucovorin (IFL).

### 5.2.1.4. Treatment of Prostate Cancer

In specific embodiments, patients with prostate cancer are administered an effective amount of one or more monoclonal antibodies of the invention. In another embodiment, the antibodies of the invention can be administered in combination with an effective amount of one or more other agents useful for prostate cancer therapy including but not limited to: external-beam radiation therapy, interstitial implantation of radioisotopes (*i.e*., I¹²⁵, palladium, iridium), leuprolide or other LHRH agonists, non-steroidal antiandrogens (flutamide, nilutamide, bicalutamide), steroidal antiandrogens (cyproterone acetate), the combination of leuprolide and flutamide, estrogens such as DES, chlorotrianisene, ethinyl estradiol, conjugated estrogens U.S.P., DES-diphosphate, radioisotopes, such as strontium-89, the combination of external-beam radiation therapy and strontium-89, second-line hormonal therapies such as aminoglutethimide, hydrocortisone, flutamide withdrawal, progesterone, and ketoconazole, low-dose prednisone, or other chemotherapy regimens reported to produce subjective improvement in symptoms and reduction in PSA level including docetaxel, paclitaxel, estramustine/docetaxel, estramustine/etoposide, estramustine/vinblastine, and estramustine/paclitaxel.

In a specific embodiment, patients with pre-cancerous high-grade prostatic intraepithelial neoplasia (PIN) are administered an EphA2 antibody of the invention to treat the disorder and decrease the likelihood that it will progress to malignant prostate cancer.

### 5.2.1.5. Treatment of Melanoma

In specific embodiments, patients with melanoma are administered an effective amount of one or more monoclonal antibodies of the invention. In another embodiment, the antibodies of the invention can be administered in combination with an effective amount of one or more other agents useful for melanoma cancer therapy including but not limited to: dacarbazine (DTIC), nitrosoureas such as carmustine (BCNU) and lomustine (CCNU), agents with modest single agent activity including vinca alkaloids, platinum compounds, and taxanes, the Dartmouth regimen (cisplatin, BCNU, and DTIC), interferon alpha (IFN-A), and interleukin-2 (IL-2). In a specific embodiment, an effective amount of one or more agonistic monoclonal antibodies of the invention can be administered in combination with isolated hyperthermic limb perfusion (ILP) with melphalan (L-PAM), with or without tumor necrosis factor-alpha (TNF-alpha) to patients with multiple brain metastases, bone metastases, and spinal cord compression to achieve symptom relief and some shrinkage of the tumor with radiation therapy.

In a specific embodiment, patients with pre-cancerous compound nevi are administered an EphA2 antibody of the invention to treat the disorder and decrease the likelihood that it will progress to malignant melanoma.

### 5.2.1.6. Treatment of Ovarian Cancer

In specific embodiments, patients with ovarian cancer are administered an effective amount of one or more monoclonal antibodies of the invention. In another embodiment, the antibodies of the invention can be administered in combination with an effective amount of one or more other agents useful for ovarian cancer therapy including but not limited to: intraperitoneal radiation therapy, such as P³² therapy, total abdominal and pelvic radiation therapy, cisplatin, the combination of paclitaxel (Taxol) or docetaxel (Taxotere) and cisplatin or carboplatin, the combination of cyclophosphamide and cisplatin, the combination of cyclophosphamide and carboplatin, the combination of 5-FU and leucovorin, etoposide, liposomal doxorubicin, gemcitabine or topotecan. It is contemplated that an effective amount of one or more agonistic monoclonal antibodies of the invention is administered in combination with the administration Taxol for patients with platinum-refractory disease. Included is the treatment of patients with refractory ovarian cancer including administration of: ifosfamide in patients with disease that is platinum-refractory, hexamethylmelamine (HMM) as salvage chemotherapy after failure of cisplatin-based combination regimens, and tamoxifen in patients with detectable levels of cytoplasmic estrogen receptor on their tumors.

### 5.2.1.7. Treatment of Lung Cancers

In specific embodiments, patients with small lung cell cancer are administered an effective amount of one or more monoclonal antibodies of the invention. In another embodiment, the antibodies of the invention can be administered in combination with an effective amount of one or more other agents useful for lung cancer therapy including but not limited to: thoracic radiation therapy, cisplatin, vincristine, doxorubicin, and etoposide, alone or in combination, the combination of cyclophosphamide, doxorubicin, vincristine/etoposide, and cisplatin (CAV/EP), local palliation with endobronchial laser therapy, endobronchial stents, and/or brachytherapy.

In other specific embodiments, patients with non-small lung cell cancer are administered an effective amount of one or more monoclonal antibodies of the invention in combination with an effective amount of one or more other agents useful for lung cancer therapy including but not limited to: palliative radiation therapy, the combination of cisplatin, vinblastine and mitomycin, the combination of cisplatin and vinorelbine, paclitaxel, docetaxel or gemcitabine, the combination of carboplatin and paclitaxel, interstitial radiation therapy for endobronchial lesions or stereotactic radiosurgery.

### 5.2.2 Other Prophylactic/Therapeutic Agents

In some embodiments, therapy by administration of one or more monoclonal antibodies is combined with the administration of one or more therapies such as, but not limited to, chemotherapies, radiation therapies, hormonal therapies, and/or biological therapies/immunotherapies. Prophylactic/therapeutic agents include, but are not limited to, proteinaceous molecules, including, but not limited to, peptides, polypeptides, proteins, including post-translationally modified proteins, antibodies etc.; or small molecules (less than 1000 daltons), inorganic or organic compounds; or nucleic acid molecules including, but not limited to, double-stranded or single-stranded DNA, or double-stranded or single-stranded RNA, as well as triple helix nucleic acid molecules. Prophylavtic/therapeutic agents can be derived from any known organism (including, but not limited to, animals, plants, bacteria, fungi, and protista, or viruses) or from a library of synthetic molecules.

In a specific embodiment, the methods of the invention encompass administration of an antibody of the invention in combination with the administration of one or more prophylactic/therapeutic agents that are inhibitors of kinases such as, but not limited to, ABL, ACK, AFK, AKT (*e.g*., AKT-1, AKT-2, and AKT-3), ALK, AMP-PK, ATM, Auroral, Aurora2, bARK1, bArk2, BLK, BMX, BTK, CAK, CaM kinase, CDC2, CDK, CK, COT, CTD, DNA-PK, EGF-R, ErbB-1, ErbB-2, ErbB-3, ErbB-4, ERK (*e.g*., ERK1, ERK2, ERK3, ERK4, ERK5, ERK6, ERK7), ERT-PK. FAK, FOR (*e.g*., FGF1R, FGF2R), FLT (*e.g*., FLT-1, FLT-2, FLT-3, FLT-4), FRK, FYN, GSK (*e.g.,* GSK1, GSK2, GSK3-alpha, GSK3-beta, GSK4, GSK5), G-protein coupled receptor kinases (GRKs), HCK, HER2, HKIL, JAK (*e.g*., JAK1, JAK2, JAK3, JAK4), JNK (*e.g*., JNK1, JNK2, JNK3), KDR, KIT, IGF-1 receptor, IKK-1, IKK-2, INSR (insulin receptor), IRAK1, IRAK2, IRK, ITK, LCK, LOK, LYN, MAPK, MAPKAPK-1, MAPKAPK-2, MEK, MET, MFPK, MHCK, MLCK, MLK3, NEU, NIK, PDGF receptor alpha, PDGF receptor beta, PHK, PI-3 kinase, PKA, PKB, PKC, PKG, PRK1, PYK2, p38 kinases, p135tyk2, p34cdc2, p42cdc2, p42mapk, p44mpk, RAF, RET, RIP, RIP-2, RK, RON, RS kinase, SRC, SYK, S6K, TAK1, TEC, TIE1, TIE2, TRKA, TXK, TYK2, UL13, VEGFR1, VEGFR2, YES, YRK, ZAP-70, and all subtypes of these kinases (see *e.g*., Hardie and Hanks (1995) The Protein Kinase Facts Book, I and II, Academic Press, San Diego, Calif.). In preferred embodiments, an antibody of the invention id administered in combination with the administration of one or more prophylactic/therapeutic agents that are inhibitors of Eph receptor kinases (*e.g*., EphA2, EphA4). In a most preferred embodiment, an antibody of the invention is administered in combination with the administration of one or more prophylactic/therapeutic agents that are inhibitors of EphA2.

In another specific embodiment, the antibodies of the invention encompass administration of the antibody of the invention in combination with the administration of one or more prophylactic/therapeutic agents that are angiogenesis inhibitors such as, but not limited to: Angiostatin (plasminogen fragment); antiangiogenic antithrombin III; Angiozyme; ABT-627; Bay 12-9566; Benefin; Bevacizumab; BMS-275291; cartilage-derived inhibitor (CDI); CAI; CD59 complement fragment; CEP-7055; Col 3; Combretastatin A-4; Endostatin (collagen XVIII fragment); fibronectin fragment; Gro-beta; Halofuginone; Heparinases; Heparin hexasaccharide fragment; HMV833; Human chorionic gonadotropin (hCG); IM-862; Interferon alpha/beta/gamma; Interferon inducible protein (IP-10), Interleukin-12; Kringle 5 (plasminogen fragment); Marimastat; Metalloproteinase inhibitors (TIMPs); 2-Methoxyestradiol; MMI 270 (CGS 27023A); MoAb IMC-1C11; Neovastat; NM-3; Panzem; PI-88; Placental ribonuclease inhibitor; Plasminogen activator inhibitor; Platelet factor-4 (PF4); Prinomastat; Prolactin 16kD fragment; Proliferin-related protein (PRP); PTK 787/ZK 222594; Retinoids; Solimastat; Squalamine; SS 3304; SU 5416; SU6668; SU11248; Tetrahydrocortisol-S; tetrathiomolybdate; thalidomide; Thrombospondin-1 (TSP-1); TNP-470; Transforming growth factor-beta (TGF-β); Vasculostatin; Vasostatin (calreticulin fragment); ZD6126; ZD6474; farnesyl transferase inhibitors (FTI); and bisphosphonates.

In another specific embodiment, the methods of the invention encompass administration of an antibody of the invention in combination with the administration of one or more prophylactic/therapeutic agents that are anti-cancer agents such as, but not limited to: acivicin, aclarubicin, acodazole hydrochloride, acronine, adozelesin, aldesleukin, altretamine, ambomycin, ametantrone acetate, aminoglutethimide, amsacrine, anastrozole, anthramycin, asparaginase, asperlin, azacitidine, azetepa, azotomycin, batimastat, benzodepa, bicalutamide, bisantrene hydrochloride, bisnafide dimesylate, bizelesin, bleomycin sulfate, brequinar sodium, bropirimine, busulfan, cactinomycin, calusterone, caracemide, carbetimer, carboplatin, carmustine, carubicin hydrochloride, carzelesin, cedefingol, chlorambucil, cirolemycin, cisplatin, cladribine, crisnatol mesylate, cyclophosphamide, cytarabine, dacarbazine, dactinomycin, daunorubicin hydrochloride, decarbazine, decitabine, dexormaplatin, dezaguanine, dezaguanine mesylate, diaziquone, docetaxel, doxorubicin, doxorubicin hydrochloride, droloxifene, droloxifene citrate, dromostanolone propionate, duazomycin, edatrexate, eflornithine hydrochloride, elsamitrucin, enloplatin, enpromate, epipropidine, epirubicin hydrochloride, erbulozole, esorubicin hydrochloride, estramustine, estramustine phosphate sodium, etanidazole, etoposide, etoposide phosphate, etoprine, fadrozole hydrochloride, fazarabine, fenretinide, floxuridine, fludarabine phosphate, fluorouracil, flurocitabine, fosquidone, fostriecin sodium, gemcitabine, gemcitabine hydrochloride, hydroxyurea, idarubicin hydrochloride, ifosfamide, ilmofosine, interleukin 2 (including recombinant interleukin 2, or rIL2), interferon alpha-2a, interferon alpha-2b, interferon alpha-n1, interferon alpha-n3, interferon beta-I a, interferon gamma-I b, iproplatin, irinotecan hydrochloride, lanreotide acetate, letrozole, leuprolide acetate, liarozole hydrochloride, lometrexol sodium, lomustine, losoxantrone hydrochloride, masoprocol, maytansine, mechlorethamine hydrochloride, megestrol acetate, melengestrol acetate, melphalan, menogaril, mercaptopurine, methotrexate, methotrexate sodium, metoprine, meturedepa, mitindomide, mitocarcin, mitocromin, mitogillin, mitomalcin, mitomycin, mitosper, mitotane, mitoxantrone hydrochloride, mycophenolic acid, nitrosoureas, nocodazole, nogalamycin, ormaplatin, oxisuran, paclitaxel, pegaspargase, peliomycin, pentamustine, peplomycin sulfate, perfosfamide, pipobroman, piposulfan, piroxantrone hydrochloride, plicamycin, plomestane, porfimer sodium, porfiromycin, prednimustine, procarbazine hydrochloride, puromycin, puromycin hydrochloride, pyrazofurin, riboprine, rogletimide, safingol, safingol hydrochloride, semustine, simtrazene, sparfosate sodium, sparsomycin, spirogermanium hydrochloride, spiromustine, spiroplatin, streptonigrin, streptozocin, sulofenur, talisomycin, tecogalan sodium, tegafur, teloxantrone hydrochloride, temoporfin, teniposide, teroxirone, testolactone, thiamiprine, thioguanine, thiotepa, tiazofurin, tirapazamine, toremifene citrate, trestolone acetate, triciribine phosphate, trimetrexate, trimetrexate glucuronate, triptorelin, tubulozole hydrochloride, uracil mustard, uredepa, vapreotide, verteporfin, vinblastine sulfate, vincristine sulfate, vindesine, vindesine sulfate, vinepidine sulfate, vinglycinate sulfate, vinleurosine sulfate, vinorelbine tartrate, vinrosidine sulfate, vinzolidine sulfate, vorozole, zeniplatin, zinostatin, zorubicin hydrochloride. Other anti-cancer drugs include, but are not limited to: 20-epi-1,25 dihydroxyvitamin D3, 5-ethynyluracil, abiraterone, aclarubicin, acylfulvene, adecypenol, adozelesin, aldesleukin, ALL-TK antagonists, altretamine, ambamustine, amidox, amifostine, aminolevulinic acid, amrubicin, amsacrine, anagrelide, anastrozole, andrographolide, angiogenesis inhibitors, antagonist D, antagonist G, antarelix, anti-dorsalizing morphogenetic protein-1, antiandrogens, antiestrogens, antineoplaston, aphidicolin glycinate, apoptosis gene modulators, apoptosis regulators, apurinic acid, ara-CDP-DL-PTBA, arginine deaminase, asulacrine, atamestane, atrimustine, axinastatin 1, axinastatin 2, axinastatin 3, azasetron, azatoxin, azatyrosine, baccatin III derivatives, balanol, batimastat, BCR/ABL antagonists, benzochlorins, benzoylstaurosporine, beta lactam derivatives, beta-alethine, betaclamycin B, betulinic acid, bFGF inhibitor, bicalutamide, bisantrene, bisaziridinylspermine, bisnafide, bistratene A, bizelesin, breflate, bropirimine, budotitane, buthionine sulfoximine, calcipotriol, calphostin C, camptothecin derivatives, canarypox IL-2, capecitabine, carboxamide-amino-triazole, carboxyamidotriazole, CaRest M3, CARN 700, cartilage derived inhibitor, carzelesin, casein kinase inhibitors (ICOS), castanospermine, cecropin B, cetrorelix, chloroquinoxaline sulfonamide, cicaprost, cis-porphyrin, cladribine, clomifene analogues, clotrimazole, collismycin A, collismycin B, combretastatin A4, combretastatin analogue, conagenin, crambescidin 816, crisnatol, cryptophycin 8, cryptophycin A derivatives, curacin A, cyclopentanthraquinones, cycloplatam, cypemycin, cytarabine ocfosfate, cytolytic factor, cytostatin, dacliximab, decitabine, dehydrodidemnin B, deslorelin, dexamethasone, dexifosfamide, dexrazoxane, dexverapamil, diaziquone, didemnin B, didox, diethylnorspermine, dihydro-5-azacytidine, dihydrotaxol, dioxamycin, diphenyl spiromustine, docetaxel, docosanol, dolasetron, doxifluridine, droloxifene, dronabinol, duocarmycin SA, ebselen, ecomustine, edelfosine, edrecolomab, eflornithine, elemene, emitefur, epirubicin, epristeride, estramustine analogue, estrogen agonists, estrogen antagonists, etanidazole, etoposide phosphate, exemestane, fadrozole, fazarabine, fenretinide, filgrastim, finasteride, flavopiridol, flezelastine, fluasterone, fludarabine, fluorodaunorunicin hydrochloride, forfenimex, formestane, fostriecin, fotemustine, gadolinium texaphyrin, gallium nitrate, galocitabine, ganirelix, gelatinase inhibitors, gemcitabine, glutathione inhibitors, hepsulfam, heregulin, hexamethylene bisacetamide, hypericin, ibandronic acid, idarubicin, idoxifene, idramantone, ilmofosine, ilomastat, imidazoacridones, imiquimod, immunostimulant peptides, insulin-like growth factor-1 receptor inhibitor, interferon agonists, interferons, interleukins, iobenguane, iododoxorubicin, ipomeanol, iroplact, irsogladine, isobengazole, isohomohalicondrin B, itasetron, jasplakinolide, kahalalide F, lamellarin-N triacetate, lanreotide, leinamycin, lenograstim, lentinan sulfate, leptolstatin, letrozole, leukemia inhibiting factor, leukocyte alpha interferon, leuprolide+estrogen+progesterone, leuprorelin, levamisole, liarozole, linear polyamine analogue, lipophilic disaccharide peptide, lipophilic platinum compounds, lissoclinamide 7, lobaplatin, lombricine, lometrexol, lonidamine, losoxantrone, lovastatin, loxoribine, lurtotecan, lutetium texaphyrin, lysofylline, lytic peptides, maitansine, mannostatin A, marimastat, masoprocol, maspin, matrilysin inhibitors, matrix metalloproteinase inhibitors, menogaril, merbarone, meterelin, methioninase, metoclopramide, MIF inhibitor, mifepristone, miltefosine, mirimostim, mismatched double stranded RNA, mitoguazone, mitolactol, mitomycin analogues, mitonafide, mitotoxin fibroblast growth factor-saporin, mitoxantrone, mofarotene, molgramostim, monoclonal antibody, human chorionic gonadotrophin, monophosphoryl lipid A+myobacterium cell wall sk, mopidamol, multiple drug resistance gene inhibitor, multiple tumor suppressor 1-based therapy, mustard anticancer agent, mycaperoxide B, mycobacterial cell wall extract, myriaporone, N-acetyldinaline, N-substituted benzamides, nafarelin, nagrestip, naloxone+pentazocine, napavin, naphterpin, nartograstim, nedaplatin, nemorubicin, neridronic acid, neutral endopeptidase, nilutamide, nisamycin, nitric oxide modulators, nitroxide antioxidant, nitrullyn, O6-benzylguanine octreotide, okicenone, oligonucleotides, onapristone, ondansetron, ondansetron, oracin, oral cytokine inducer, ormaplatin, osaterone, oxaliplatin, oxaunomycin, paclitaxel, paclitaxel analogues, paclitaxel derivatives, palauamine, palmitoylrhizoxin, pamidronic acid, panaxytriol, panomifene, parabactin, pazelliptine, pegaspargase, peldesine, pentosan polysulfate sodium, pentostatin, pentrozole, perflubron, perfosfamide, perillyl alcohol, phenazinomycin, phenylacetate, phosphatase inhibitors, picibanil, pilocarpine hydrochloride, pirarubicin, piritrexim, placetin A, placetin B, plasminogen activator inhibitor, platinum complex, platinum compounds, platinum-triamine complex, porfimer sodium, porfiromycin, prednisone, propyl bis-acridone, prostaglandin J2, proteasome inhibitors, protein A-based immune modulator, protein kinase C inhibitor, protein kinase C inhibitors, microalgal, protein tyrosine phosphatase inhibitors, purine nucleoside phosphorylase inhibitors, purpurins, pyrazoloacridine, pyridoxylated hemoglobin polyoxyethylene conjugate, raf antagonists, raltitrexed, ramosetron, ras farnesyl protein transferase inhibitors, ras inhibitors, ras-GAP inhibitor, retelliptine demethylated, rhenium Re 186 etidronate, rhizoxin, ribozymes, RII retinamide, rogletimide, rohitukine, romurtide, roquinimex, rubiginone B1, ruboxyl, safingol, saintopin, SarCNU, sarcophytol A, sargramostim, Sdi 1 mimetics, semustine, senescence derived inhibitor 1, sense oligonucleotides, signal transduction inhibitors, signal transduction modulators, single chain antigen binding protein, sizofiran, sobuzoxane, sodium borocaptate, sodium phenylacetate, solverol, somatomedin binding protein, sonermin, sparfosic acid, spicamycin D, spiromustine, splenopentin, spongistatin 1, squalamine, stem cell inhibitor, stem-cell division inhibitors, stipiamide, stromelysin inhibitors, sulfinosine, superactive vasoactive intestinal peptide antagonist, suradista, suramin, swainsonine, synthetic glycosaminoglycans, tallimustine, tamoxifen methiodide, tauromustine, taxol, tazarotene, tecogalan sodium, tegafur, tellurapyrylium, telomerase inhibitors, temoporfin, temozolomide, teniposide, tetrachlorodecaoxide, tetrazomine, thaliblastine, thalidomide, thiocoraline, thioguanine, thrombopoietin, thrombopoietin mimetic, thymalfasin, thymopoietin receptor agonist, thymotrinan, thyroid stimulating hormone, tin ethyl etiopurpurin, tirapazamine, titanocene bichloride, topsentin, toremifene, totipotent stem cell factor, translation inhibitors, tretinoin, triacetyluridine, triciribine, trimetrexate, triptorelin, tropisetron, turosteride, tyrosine kinase inhibitors, tyrphostins, UBC inhibitors, ubenimex, urogenital sinus-derived growth inhibitory factor, urokinase receptor antagonists, vapreotide, variolin B, vector system, erythrocyte gene therapy, velaresol, veramine, verdins, verteporfin, vinorelbine, vinxaltine, vitaxin, vorozole, zanoterone, zeniplatin, zilascorb, and zinostatin stimalamer. Preferred additional anti-cancer drugs are 5-fluorouracil and leucovorin.

In more particular embodiments, the present invention also comprises the administration of one or more monoclonal antibodies of the invention in combination with the administration of one or more therapies such as, but not limited to anti-cancer agents such as those disclosed in Table 2, preferably for the treatment of breast, ovary, melanom, prostate, colon and lung cancers as described above.

**TABLE 2**

| Therapeutic Agent | Administration | Dose | Intervals |
|---|---|---|---|
| doxorubicin hydrochloride (Adriamycin RDF® and Adriamycin PFS®) | Intravenous | 60-75 mg/m² on Day 1 | 21 day intervals |
| epirubicin hydrochloride (Ellence™) | Intravenous | 100-120 mg/m² on Day 1 of each cycle or divided equally and given on Days 1-8 of the cycle | 3-4 week cycles |
| fluorousacil | Intravenous 5 | How supplied: 5 ml and 10 ml vials (containing 250 and 500 mg flourouracil respectively) | |
| docetaxel (Taxotere®) | Intravenous | 60- 100 mg/m² over 1 hour | Once every 3 weeks |
| paclitaxel (Taxol® | Intravenous | 175 mg/m² over 3 hours | Every 3 weeks for 4 courses (administered sequentially to doxorubicin-containing combination chemotherapy) |
| tamoxifen citrate (Nolvadex®) | Oral (tablet) | 20-40 mg Dosages greater than 20 mg should be given in divided doses (morning and evening) | Daily |
| leucovorin calcium for injection | Intravenous or intramuscular injection | How supplied: 350 mg vial | Dosage is unclear from text. PDR3610 |
| luprolide acetate (Lupron®) | Single subcutaneous injection | 1 mg (0.2 ml or 20 unit mark) | Once a day |
| flutamide (Eulexin®) | Oral (capsule) | 250 mg (capsules contain 125 mg flutamide each) | 3 times a day at 8 hour intervals (total daily dosage 750 mg) |
| nilutamide (Nilandron®) | Oral (tablet) | 300 mg or 150 mg (tablets contain 50 or 150 mg nilutamide each) | 300 mg once a day for 30 days followed by 150 mg once a day |
| bicalutamide (Casodex®) | Oral (tablet) | 50 mg (tablets contain 50 mg bicalutamide each) | Once a day |
| progesterone | Injection | USP in sesame oil 50 mg/ml | |
| ketoconazole (Nizoral®) | Cream | 2% cream applied once or twice daily depending on symptoms | |
| prednisone | Oral (tablet) | Initial dosage may vary from 5 mg to 60 mg per day | |
| | | depending on the specific disease entity being treated. | |
| estramustine phosphate sodium (Emcyt®) | Oral (capsule) | 14 mg/ kg of body weight (i.e. one 140 mg capsule for each 10 kg or 22 Ib of body weight) | Daily given in 3 or 4 divided doses |
| etoposide or VP-16 | Intravenous | 5 ml of 20 mg/ ml solution (100 mg) | |
| dacarbazine (DTIC-Dome®) | Intravenous | 2-4.5 mg/kg | Once a day for 10 days. May be repeated at 4 week intervals |
| polifeprosan 20 with carmustine implant (BCNU) (nitrosourea) (Gliadel®) | wafer placed in resection cavity | 8 wafers, each containing 7.7 mg of carmustine, for a total of 61.6 mg, if size and shape of resection cavity allows | |
| cisplatin | Injection | [n/a in PDR 861] How supplied: solution of 1 mg/ml in multidose vials of 50mL and 100mL | |
| mitomycin | Injection | supplied in 5 mg and 20 mg vials (containing 5 mg and 20 mg mitomycin) | |
| gemcitabine HCl (Gemzar®) | Intravenous | For NSCLC- 2 schedules have been investigated and the optimum schedule has not been determined 4 week schedule-administration intravenously at 1000 mg/m² over 30 minutes on 3 week schedule-Gemzar administered intravenously at 1250 mg/m² over 30 minutes | 4 week schedule-Days 1,8 and 15 of each 28-day cycle. Cisplatin intravenously at 100 mg/m² on day 1 after the infusion of Gemzar. 3 week schedule-Days 1 and 8 of each 21 day cycle. Cisplatin at dosage of 100 mg/m² administered intravenously after administration of Gemzar on day 1. |
| carboplatin (Paraplatin®) | Intravenous | Single agent therapy: 360 mg/m² I.V. on day 1 (infusion lasting 15 minutes or longer) Other dosage calculations: Combination therapy with cyclophosphamide, Dose adjustment recommendations, Formula dosing, etc. | Every 4 weeks |
| ifosamide (Ifex®) | Intravenous | 1.2 g/m² daily | 5 consecutive days Repeat every 3 weeks or after recovery from hematologic toxicity |
| topotecan hydrochloride (Hycamtin®) | Intravenous | 1.5 mg/m² by intravenous infusion over 30 minutes daily | 5 consecutive days, starting on day 1 of 21 day course |

The invention also encompasses administration of the EphA2 antibodies of the invention in combination with radiation therapy comprising the use of x-rays, gamma rays and other sources of radiation to destroy the cancer cells. In preferred embodiments, the radiation treatment is administered as external beam radiation or teletherapy wherein the radiation is directed from a remote source. In other preferred embodiments, the radiation treatment is administered as internal therapy or brachytherapy wherein a radioactive source is placed inside the body close to cancer cells or a tumor mass.

Cancer therapies and their dosages, routes of administration and recommended usage are known in the art and have been described in such literature as the Physician's Desk Reference (56th ed., 2002).

### 5.3 Identification of Antibodies of the Invention

### 5.3.1 Agonistic Antibodies

Antibodies of the invention agonize (*i.e*., elicit EphA2 phosphorylation) as well as immunospecifically bind to the EphA2 receptor. When agonized, EphA2 becomes phosphorylated and then subsequently degraded. Any method known in the art to assay either the level of EphA2 phosphorylation, activity, or expression can be used to assay candidate EphA2 antibodies to determine their agonistic activity (see, *e.g*., Section 6.2.1 *infra*).

The invention teaches methods of assaying and screening for EphA2 antibodies of the invention by incubating antibodies that specifically bind EphA2, particularly that bind the extracellular domain of BphA2, with cells that express EphA2, particularly cancer cells, preferably metastatic cancer cells, that overexpress EphA2 (relative to non-cancer cells of the same cell type) and then assaying for an increase in EphA2 phosphorylation and/or EphA2 degradation, thereby identifying an BphA2 antibody of the invention.

### 5.3.2 Antibodies That Preferentially Bind EphA2 Epitopes Exposed on Cancer Cells

Antibodies of the invention may preferably bind to EphA2 epitopes exposed on cancer cells (*e.g.*, cells overexpressing EphA2 and/or cells with substantial EphA2 that is not bound to ligand) but not non-cancer cells or cell where EphA2 is bound to ligand. In this embodiment, antibodies of the invention are antibodies directed to an EphA2 epitope not exposed on non-cancer cells but exposed on cancer cells (see, *e*,*g*., Section 6.6 *infra*). Differences in EphA2 membrane distribution between non-cancer cells and cancer cells expose certain epitopes on cancer cells that are not exposed on non-cancer cells. For example, normally EphA2 is bound to its ligand, EphrinA1, and localizes at areas of cell-cell contacts. However, cancer cells generally display decreased cell-cell contacts as well as overexpress EphA2 in excess of its ligand. Thus, in cancer cells, there is an increased amount of unbound EphA2 that is not localized to cell-cell contacts. As such, in one embodiment, an antibody that preferentially binds unbound, unlocalized EphA2 is an antibody of the invention.

Any method known in the art to determine candidate EphA2 antibody binding/localization on a cell can be used to screen candidate antibodies for desirable binding properties. In a one embodiment, immunofluorescence microscopy is used to determine the binding characteristics of an antibody. Standard techniques can be used to compare the binding of an antibody binding to cells grown *in vitro.* In a specific embodiment, antibody binding to cancer cells is compared to antibody binding to non-cancer cells. An exposed EphA2 epitope antibody binds poorly to non-cancer cells but binds well to cancer cells. In another specific embodiment, antibody binding to non-cancer dissociated cells (*e.g*., treated with a calcium chelator such as EGTA) is compared to antibody binding to non-cancer cells that have not been dissociated. An exposed EphA2 epitope antibody binds poorly non-cancer cells that have not been dissociated but binds well to dissociated non-cancer cells.

In another embodiment, flow cytometry is used to determine the binding characteristics of an antibody. In this embodiment, EphA2 may or may not be crosslinked to its ligand, Ephrin A1. An exposed EphA2 epitope antibody binds poorly crosslinked EphA2 but binds well to uncrosslinked EphA2.

In another embodiment, cell-based or immunoassays are used to determine the binding characteristics of an antibody. In this embodiment, antibodies that can compete with an EphA2 ligand (*e.g*., Ephrin A1) for binding to EphA2 displace Ephrin A1 from EphA2. The EphA2 ligand used in this assay can be soluble protein (*e.g*., recombinantly expressed) or expressed on a cell so that it is anchored to the cell.

### 5.4 Characterization And Demonstration Of Therapeutic Or Prophylactic Utility

Toxicity and efficacy of the prophylactic and/or therapeutic protocols of the instant invention can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Prophylactic and/or therapeutic agents that exhibit large therapeutic indices are preferred. While prophylactic and/or therapeutic agents that exhibit toxic side effects may be used, care should be taken to design a delivery system that targets such agents to the site of affected tissue in order to minimize potential damage to uninfected cells and, thereby, reduce side effects.

The data obtained from the cell culture assays and animal studies can be used in formulating a range of dosage of the prophylactic and/or therapeutic agents for use in humans. The dosage of such agents lies preferably within a range of circulating concentrations that include the ED₅₀ with little or no toxicity. The dosage may vary within this range depending upon the dosage form employed and the route of administration utilized. For any agent used in the method of the invention, the therapeutically effective dose can be estimated initially from cell culture assays. A dose may be formulated in animal models to achieve a circulating plasma concentration range that includes the IC₅₀ (*i.e*., the concentration of the test compound that achieves a half-maximal inhibition of symptoms) as determined in cell culture. Such information can be used to more accurately determine useful doses in humans. Levels in plasma may be measured, for example, by high performance liquid chromatography.

The anti-cancer activity of the therapies used in accordance with the present invention also can be determined by using various experimental animal models for the study of cancer such as the SCID mouse model or transgenic mice where a mouse EphA2 is replaced with the human EphA2, nude mice with human xenografts, animal models described in Section 6 *infra,* or any animal model (including hamsters, rabbits, etc.) known in the art and described in Relevance of Tumor Models for Anticancer Drug Development (1999, eds. Fiebig and Burger); Contributions to Oncology (1999, Karger); The Nude Mouse in Oncology Research (1991, eds. Boven and Winograd); and Anticancer Drug Development Guide (1997 ed. Teicher),

### 5.4.1 Demonstration of Therapeutic Utility

The protocols and compositions of the invention are preferably tested *in vitro,* and then *in vivo,* for the desired therapeutic or prophylactic activity, prior to use in humans. For example, *in vitro* assays which can be used to determine whether administration of a specific therapeutic protocol is indicated, include *in vitro* cell culture assays in which a patient tissue sample is grown in culture, and exposed to or otherwise administered a protocol, and the effect of such protocol upon the tissue sample is observed, *e.g*., increased phosphorylation/degradation of EphA2. A lower level of proliferation or survival of the contacted cells indicates that the therapeutic agent is effective to treat the condition in the patient. Alternatively, instead of culturing cells from a patient, therapeutic agents and methods may be screened using cells of a tumor or malignant cell line. Many assays standard in the art can be used to assess such survival and/or growth; for example, cell proliferation can be assayed by measuring ³H-thymidine incorporation, by direct cell count, by detecting changes in transcriptional activity of known genes such as proto-oncogenes (*e.g*., fos, myc) or cell cycle markers; cell viability can be assessed by trypan blue staining, differentiation can be assessed visually based on changes in morphology, increased phosphorylation/degradation of EphA2, etc.

Compounds for use in therapy can be tested in suitable animal model systems prior to testing in humans, including but not limited to in rats, mice, chicken, cows, monkeys, rabbits, hamsters, etc., for example, the animal models described above. The compounds can then be used in the appropriate clinical trials.

Further, any assays known to those skilled in the art can be used to evaluate the prophylactic and/or therapeutic utility of the combinatorial therapies disclosed herein for treatment or prevention of cancer.

### 5.5 Pharmaceutical Compositions

The compositions of the invention include bulk drug compositions useful in the manufacture of pharmaceutical compositions (*e.g*., impure or non-sterile compositions) and pharmaceutical compositions (*i.e.,* compositions that are suitable for administration to a subject or patient) which can be used in the preparation of unit dosage forms. Such compositions comprise a prophylactically or therapeutically effective amount of a prophylactic and/or therapeutic agent disclosed herein or a combination of those agents and a pharmaceutically acceptable carrier. Preferably, compositions of the invention comprise a prophylactically or therapeutically effective amount of one or more EphA2 antibodies of the invention and a pharmaceutically acceptable carrier. In a further embodiment, the composition of the invention further comprises an additional anti-cancer agent.

In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant (*e.g*., Freund's adjuvant (complete and incomplete) or, more preferably, MF59C.1 adjuvant available from Chiron, Emeryville, CA), excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like.

Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

Various delivery systems are known and can be used to administer an agonistic monoclonal antibody of the invention or the combination of an agonistic monoclonal antibody of the invention and a prophylactic agent or therapeutic agent useful for preventing or treating cancer, *e.g*., encapsulation in liposomes, microparticles, microcapsules, recombinant cells capable of expressing the antibody or antibody fragment, receptor-mediated endocytosis (see, *e.g*., Wu and Wu, 1987, J. Biol. Chem. 262:4429-4432), construction of a nucleic acid as part of a retroviral or other vector, etc. Methods of administering a prophylactic or therapeutic agent of the invention include, but are not limited to, parenteral administration (*e.g*., intradermal, intramuscular, intraperitoneal, intravenous and subcutaneous), epidural, and mucosal (*e.g*., intranasal, inhaled, and oral routes). In a specific embodiment, prophylactic or therapeutic agents of the invention are administered intramuscularly, intravenously, or subcutaneously. The prophylactic or therapeutic agents may be administered by any convenient route, for example by infusion or bolus injection, by absorption through epithelial or mucocutaneous linings (*e.g*., oral mucosa, rectal and intestinal mucosa, etc.) and may be administered together with other biologically active agents. Administration can be systemic or local.

In a specific embodiment, it may be desirable to administer the prophylactic or therapeutic agents of the invention locally to the area in need of treatment; this may be achieved by, for example, and not by way of limitation, local infusion, by injection, or by means of an implant, said implant being of a porous, non-porous, or gelatinous material, including membranes, such as sialastic membranes, or fibers.

In yet another embodiment, the prophylactic or therapeutic agent can be delivered in a controlled release or sustained release system. In one embodiment, a pump may be used to achieve controlled or sustained release (see Langer, *supra;* Sefton, 1987, CRC Crit. Ref. Biomed. Eng. 14:20; Buchwald et al., 1980, Surgery 88:507; Saudek et al., 1989, N. Engl. J. Med. 321:574). In another embodiment, polymeric materials can be used to achieve controlled or sustained release of the antibodies of the invention or fragments thereof (see *e.g*., Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, 1983, J. Macromol. Sci. Rev. Macromol. Chem. 23:61; see also Levy et al., 1985, Science 228:190; During et al., 1989, Ann. Neurol. 25:351; Howard et al., 1989, J. Neurosurg. 7 1:105); U.S. Patent Nos. 5,679,377; 5,916,597; 5,912,015; 5,989,463; 5,128,326; International Publication Nos. WO 99/15154 and WO 99/20253. Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. In a preferred embodiment, the polymer used in a sustained release formulation is inert, free of leachable impurities, stable on storage, sterile, and biodegradable. In yet another embodiment, a controlled or sustained release system can be placed in proximity of the prophylactic or therapeutic target, thus requiring only a fraction of the systemic dose (see, *e.g*., Goodson, in Medical Applications of Controlled Release, supra, vol. 2, pp. 115-138 (1984)).

Controlled release systems are discussed in the review by Langer (1990, Science 249:1527-1533). Any technique known to one of skill in the art can be used to produce sustained release formulations comprising one or more therapeutic agents of the invention. See, *e.g*., U.S. Patent No. 4,526,938; International Publication Nos. WO 91/05548 and WO 96/20698; Ning et al., 1996, Radiotherapy & Oncology 39:179-189; Song et al., 1995, PDA Journal of pharmaceutical Science & Technology 50:372-397; Cleek et al., 1997, Pro. Int'l. Symp. Control. Rel. Bioact. Mater. 24:853-854; and Lam et al., 1997, Proc. Int'l. Symp. Control Rel. Bioact. Mater. 24:759-760,

### 5.5.1 Formulations

Pharmaceutical compositions for use in accordance with the present invention may be formulated in conventional manner using one or more physiologically acceptable carriers or excipients.

Thus, the EphA2 antibodies of the invention and their physiologically acceptable salts and solvates may be formulated for administration by inhalation or insufflation (either through the mouth or the nose) or oral, parenteral or mucosal (such as buccal, vaginal, rectal, sublingual) administration. In a preferred embodiment, local or systemic parenteral administration is used.

For oral administration, the pharmaceutical compositions may take the form of, for example, tablets or capsules prepared by conventional means with pharmaceutically acceptable excipients such as binding agents (*e.g*., pregelatinised maize starch, polyvinylpyrrolidone or hydroxypropyl methylcellulose); fillers (*e.g*., lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active compound.

For buccal administration the compositions may take the form of tablets or lozenges formulated in conventional manner.

For administration by inhalation, the prophylactic or therapeutic agents for use according to the present invention are conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g*., gelatin for use in an inhaler or insufflator may be formulated containing a powder mix of the compound and a suitable powder base such as lactose or starch.

The prophylactic or therapeutic agents may be formulated for parenteral administration by injection, *e.g*., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, *e.g*., sterile pyrogen-free water, before use.

The prophylactic or therapeutic agents may also be formulated in rectal compositions such as suppositories or retention enemas, *e.g*., containing conventional suppository bases such as cocoa butter or other glycerides.

In addition to the formulations described previously, the prophylactic or therapeutic agents may also be formulated as a depot preparation. Such long acting formulations may be administered by implantation (for example subcutaneously or intramuscularly) or by intramuscular injection. Thus, for example, the prophylactic or therapeutic agents may be formulated with suitable polymeric or hydrophobic materials (for example as an emulsion in an acceptable oil) or ion exchange resins, or as sparingly soluble derivatives, for example, as a sparingly soluble salt.

The invention also provides that a prophylactic or therapeutic agent is packaged in a hermetically sealed container such as an ampoule or sachette indicating the quantity. In one embodiment, the prophylactic or therapeutic agent is supplied as a dry sterilized lyophilized powder or water free concentrate in a hermetically sealed container and can be reconstituted, *e.g.*, with water or saline to the appropriate concentration for administration to a subject.

In a preferred embodiment of the invention, the formulation and administration of various chemotherapeutic, biological/immunotherapeutic and hormonal therapeutic agents are known in the art and often described in the Physician's Desk Reference, 56th ed. (2002). For instance, in certain specific embodiments of the invention, the therapeutic agents of the invention can be formulated and supplied as provided in Table 2.

In other embodiments of the invention, radiation therapy agents such as radioactive isotopes can be given orally as liquids in capsules or as a drink. Radioactive isotopes can also be formulated for intravenous injections. The skilled oncologist can determine the preferred formulation and route of administration.

In certain embodiments the agonistic monoclonal antibodies of the invention, are formulated at 1 mg/ml, 5 mg/ml, 10 mg/ml, and 25 mg/ml for intravenous injections and at 5 mg/ml, 10 mg/ml, and 80 mg/ml for repeated subcutaneous administration and intramuscular injection.

The compositions may, if desired, be presented in a pack or dispenser device that may contain one or more unit dosage forms containing the active ingredient. The pack may for example comprise metal or plastic foil, such as a blister pack. The pack or dispenser device may be accompanied by instructions for administration.

### 5.5.2 Dosages

The amount of the composition of the invention which will be effective in the treatment, prevention or management of cancer can be determined by standard research techniques. For example, the dosage of the composition which will be effective in the treatment, prevention or management of cancer can be determined by administering the composition to an animal model such as, *e.g*., the animal models disclosed herein or known to those skilled in the art. In addition, *in vitro* assays may optionally be employed to help identify optimal dosage ranges.

Selection of the preferred effective dose can be determined (*e.g*., via clinical trials) by a skilled artisan based upon the consideration of several factors which will be known to one of ordinary skill in the art. Such factors include the disease to be treated or prevented, the symptoms involved, the patient's body mass, the patient's immune status and other factors known by the skilled artisan to reflect the accuracy of administered pharmaceutical compositions.

The precise dose to be employed in the formulation will also depend on the route of administration, and the seriousness of the cancer, and should be decided according to the judgment of the practitioner and each patient's circumstances. Effective doses may be extrapolated from dose-response curves derived from *in vitro* or animal model test systems.

For antibodies, the dosage administered to a patient is typically 0.1 mg/kg to 100 mg/kg of the patient's body weight. Preferably, the dosage administered to a patient is between 0.1 mg/kg and 20 mg/kg of the patient's body weight, more preferably 1 mg/kg to 10 mg/kg of the patient's body weight. Generally, human and humanized antibodies have a longer half-life within the human body than antibodies from other species due to the immune response to the foreign polypeptides. Thus, lower dosages of human antibodies and less frequent administration is often possible.

For other cancer therapeutic agents administered to a patient, the typical doses of various cancer therapeutics known in the art are provided in Table 2. Given the invention, certain preferred embodiments will encompass the administration of lower dosages in combination treatment regimens than dosages recommended for the administration of single agents.

The invention provides for any method of administrating lower doses of known prophylactic or therapeutic agents than previously thought to be effective for the prevention, treatment, management or amelioration of cancer. Preferably, lower doses of known anti-cancer therapies are administered in combination with lower doses of agonistic monoclonal antibodies of the invention.

### 5.6 Kits

The invention provides a pharmaceutical pack or kit comprising one or more containers filled with an EphA2 antibody of the invention. Additionally, one or more other prophylactic or therapeutic agents useful for the treatment of a cancer can also be included in the pharmaceutical pack or kit. The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The present invention provides kits that can be used in the above methods. In one embodiment, a kit comprises one or more EphA2 antibodies of the invention. In another embodiment, a kit further comprises one or more other prophylactic or therapeutic agents useful for the treatment of cancer, in one or more containers.
In certain embodiments, the other prophylactic or therapeutic agent is a chemotherapeutic. In other embodiments, the prophylactic or therapeutic agent is a biological or hormonal therapeutic.

### 6. EXAMPLES

### 6.1 Preparation of Monoclonal Antibodies

### Antigen Preparation

Ras-transformed MCF-10A cells were extracted in RIPA buffer. Tyrosine phosphorylated proteins were partially purified using immobilized PY20 antibodies (Kanner et al., 1989, J. Immunol. Meth. 120:115-124). Bound proteins were competitively eluted with 25 mM phenylphosphate. Fractions containing PY20-reactive proteins were confirmed by western blot analysis using phosphotyrosine-specific antibodies.

### Antibody Screening

As a preliminary screen for EphA2-immunoreactivity, supernatants from bulk culture hybridomas were screened for immunoreactivity against EphA2. Immunization strategy was designed to identify extracellular EphA2 epitopes on viable tumor cells. Thus, a fluorescence-based ELISA protocol (FluorELISA), which selects for antibody reactivity against live cells, was utilized. This screening approach was preferable to western blot analyses, which might have biased against antibodies that recognize conformation-restricted epitopes.

Cell surface binding by anti-EphA2 antibodies to the EphA2 receptor was monitored using modifications to a reported assay (Kilpatrick et al., 1998, Hybridoma 17:576). 96 well, flat-bottom tissue culture treated plates (Costar, Cambridge, MA) were treated with 100µl of poly-L-lysine hydrobromide (Sigma, St. Louis, MO) diluted to 10 µg/ml in 0.1M sodium phosphate (pH 8.0) for 1 hour. Poly-L-lysine was removed from the wells before the addition of 100µl of a cell suspension of MDA-MB-23 1 (positive for EphA2) or BT474 cells (negative controls) at a concentration of 3 x 10⁴ cells per well. After incubation overnight at 37°C, 5% C0₂, the culture media was gently removed, and 100µl of supernatants from hybridomas were incubated on cells at room temperature for 1 hour. The samples were washed three times with 1X Dulbecco's phosphate buffered saline (pH 7.1) (GIBCO, Grand Island, NY). Goat anti-mouse Alexa Fluor 488 antibody (100µl; Molecular Probes, Eugene, OR), diluted to 2µg/ml in PBS, was added for one hour at room temperature. After washing cells with PBS, 50 µl of PBS containing 2% FCS was added to each well before observation using an inverted fluorescence microscope (Model DM-IRB, Leica, Deerfield, IL).

FluorELISA identified 44 bulk hybridoma populations that stained EphA2-overexpressing tumor cells (MDA-MB-231), but not EphA2-deficient cells (BT474) (data not shown). Immunoreactivity was confirmed using fluorescence microscopy, which revealed a pattern of diffuse membrane staining that was consistent with our previous studies (*e.g*., Zelinski et al., 2001, Cancer Res. 61:2301 and Zantek, et al., 1999, Cell Growth Diff. 10:629) of EphA2 subcellular localization. Hybridoma bulk cultures were initially selected for subcloning by flow cytometry based on strong immunostaining of target-positive, but not target deficient, cells. Bulk culture populations of hybridomas were then subcloned by flow cytometry and FluorELISA was repeated with supernatants from the subcloned hybridomas.

### 6.2 EphA2 Monoclonal Antibodies Decrease Metastatic Properties of Tumor Cells

### 6.2.1 EphA2 Phosphorylation and Degradation

EphA2 antibodies promoted tyrosine phosphorylation and degradation of EphA2 in MDA-MB-231 (FIGS. 1A-1C) cells. Monolayers of cells were incubated in the presence of EA2 (FIGS. 1A-1B, lanes 2, 3) or EA5 (FIGS. 1A-1B, lanes 4, 5) or control (FIGS. 1A-1B, lane 1) for 8 minutes at 37°C. Cell lysates were then immunoprecipitated with an EphA2-specific antibody (D7, purchased from Upstate Biologicals, Inc., Lake Placid, NY and deposited with the American Type Tissue Collection on December 8, 2000, and assigned ATCC number PTA 2755), resolved by SDS-PAGE and subjected to western blot analysis with a phosphotyrosine-specific antibody (4G10, purchased from Upstate Biologicals, Inc., Lake Placid, NY) (FIG. 1A). The membranes were stripped and re-probed with the EphA2-specific antibody used in the immunoprecipitation (D7) as a loading control.

Western blot analyses and immunoprecipitations were performed as described previously (Zantek et al., 1999, Cell Growth Diff. 10:629-38). Briefly, detergent extracts of cell monolayers were extracted in Tris-buffered saline containing 1% Triton X-100 (Sigma, St. Louis, MO). After measuring protein concentrations (BioRad, Hercules, CA), 1.5 mg of cell lysate was immunoprecipitated, resolved by SDS-PAGE and transferred to nitrocellulose (Protran, Schleicher and Schuell, Keene, NH). Antibody binding was detected by enhanced chemiluminescence (Pierce, Rockford, IL) and autoradiography (Kodak X-OMAT; Rochester, NY).

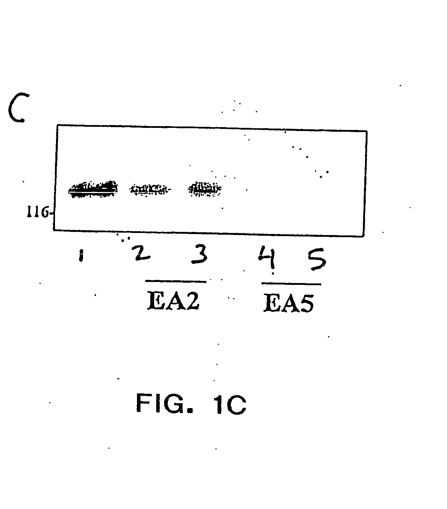

Levels of EphA2 phosphorylation were found to increase with EphA2 agonistic EA2 and EA5 antibody incubation (FIG. 1B). Monolayers of MDA-MB-231 cells were incubated in the presence of 30 µg/ml EA2 (FIG. 1C, lanes 2, 3) or EA5 (FIG. 1C, lanes 4, 5) or a control (FIG. 1C, lane 1) for 24 hours at 37°C. Cell lysates were then resolved by SDSPAGE and subjected to western blot analysis with an EphA2-specific antibody (D7). EphA2 protein levels decrease with antibody incubation.

Similar experiments were conducted with A549 cells. Monolayers of A549 cells were incubated at 37°C in the presence of EA2 or EA5 or control (PBS) for either 10 minutes (FIGS. 2A-2B) or 5 hours (FIGS. 2C-2D). Cell lysates were then immunoprecipitated with an EphA2-specific antibody (D7), resolved by SDS-PAGE and subjected to western blot analysis with a phosphotyrosine-specific antibody (4G10, purchased from Upstate Biologicals, Inc., Lake Placid, NY) (FIGS. 2A, 2C). The membranes were stripped and re-probed with the EphA2-specific antibody used in the immunoprecipitation (D7) as a loading control (FIGS. 2B, 2D). At 10 minutes, antibody incubation caused an increase in phosphorylation (FIG. 2A). With continued incubation of 5 hours, these antibodies caused degradation of the EphA2 protein. (FIG. 2D).

### 6.2.2 Growth in Soft Agar

Tumor cells were suspended in soft agar. Colony formation in soft agar was assayed as described in Zelinski et al. (2001, Cancer Res. 61:2301-6). Antibodies or a control solution (PBS) was included in bottom and top agar solutions. Cells were suspended in soft agar for 7 days at 37°C in the presence of purified antibody or control solution (PBS), administered at the time of suspension. Colony formation was scored microscopically using an Olympus CK-3 inverted phase-contrast microscope outfitted with a 40x objective. Clusters containing at least three cells were scored as a positive. The average number of colonies per high-powered field is shown. Ten separate high-power microscopic fields were averaged in each experiment, and the results shown are representative of at least three separate experiments.

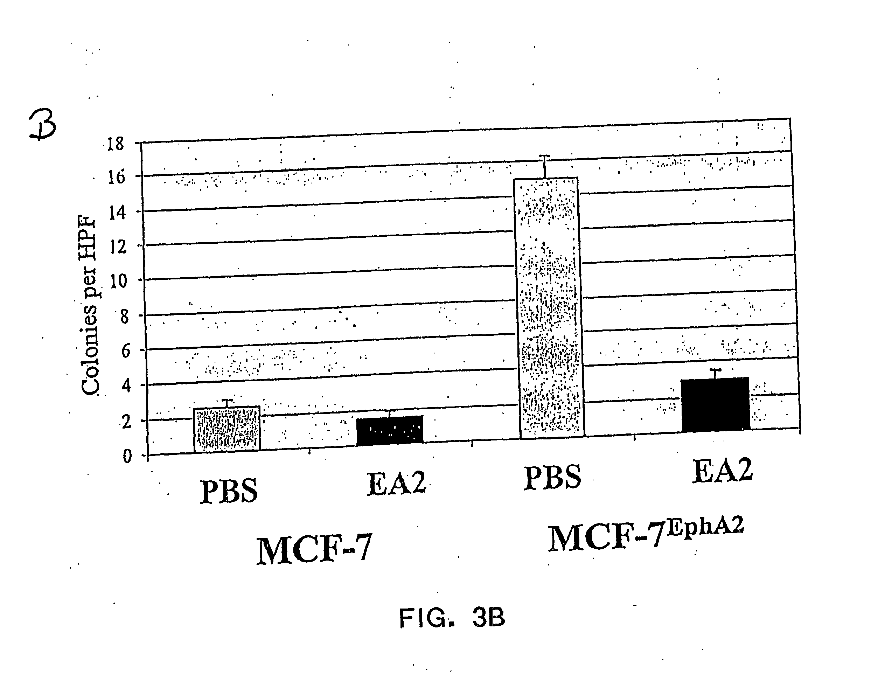

A549 malignant lung cancer cells were incubated with either 10 µg/ml or 2.5 µg/ml of EA2 or EA5 monoclonal antibodies or a control (PBS). All amounts of antibodies used inhibited cell growth in soft agar (FIG. 3A). Benign MCF-7 breast epithelial tumor cells were converted to malignant cells by the overexpression of EphA2 (MCF-7^{EphA2}). Both tumor cell types were incubated with either EA2 monoclonal antibodies or a control (PBS). EA2 inhibits the ability of MCF-7^{EphA2} cells to grow in soft agar. Benign MCF-7 did not form colonies in soft agar either with or without antibody incubation (FIG. 3B). Results are reported as colonies per high-powered field (HPF). Control experiments confirmed that neither isotype-matched (IgG₁) controls (*e.g*., anti-paxillin) nor antibodies against intracellular epitopes on EphA2 (*e.g*., D7) decreased soft agar colonization (data not shown).

### 6.2.3 Tubular Network Formation in MATRIGEL™

Tumor cell behavior within a three-dimensional microenvironment, such as MATRIGEL™, can reliably predict the differentiation state and aggressiveness of breast epithelial cells. Monolayer cultures of benign (MCF-10A) or malignant (MDA-MB-231) breast epithelial cells are incubated on MATRIGEL™ in the presence of EphA2 antibodies (10 µg/ml) or control solution (PBS). The behavior of cells on MATRIGEL™ is analyzed as described in Zelinski et al. (2001, Cancer Res. 61:2301-6). Briefly, tissue culture dishes are coated with MATRIGEL™ (Collaborative Biomedical Products, Bedford, MA) at 37°C before adding 1 x 10⁵ MDA-MB-231 or MCF-10A cells that had been incubated on ice for 1 hour with an EphA2 agonistic antibody or control solution (PBS). Cells are incubated on MATRIGEL™ for 24 hours at 37°C, and cell behavior is assessed using an Olympus IX-70 inverted light microscope. All images are recorded onto 35 mm film (T-Max-400. Kodak, Rochester, NY).

Within 24 hours, non-transformed MCF-10A epithelial cells organize into acinus-like spheres on MATRIGEL™ while MDA-MB-231 cells quickly assemble into tubular networks. These networks progressively invade all throughout the MATRIGEL™. With the addition of EphA2 agonistic antibodies, the formation of tubular networks is prevented.

### 6.2.4 Growth in vivo

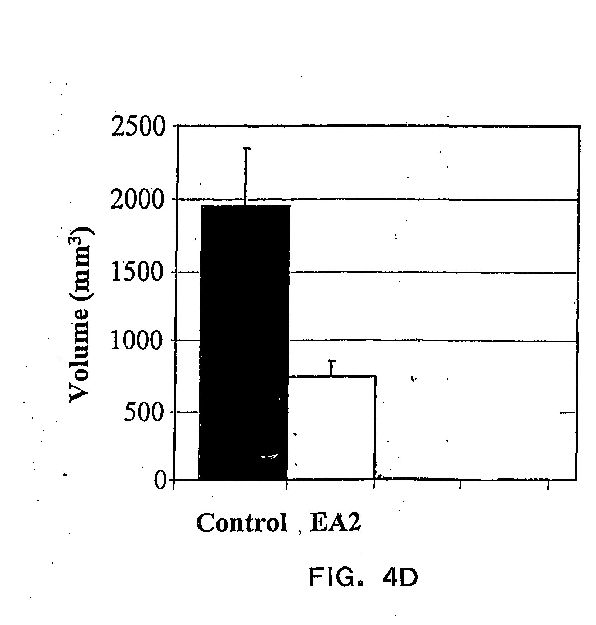

EA2 can inhibit tumor cell growth *in vivo.* 5x10⁶ MDA-MB-231 breast cancer cells were implanted orthotopically or subcutaneously and 5x10⁶ A549 lung cancer cells were implanted subcutaneously into athymic mice. After the tumors had grown to an average volume of 100mm³, mice were administered 6mg/kg of an EA2 or negative control (PBS or 1A7 antibody) intraperitoneally twice a week for 3 weeks. Animals were generally sacrificed at least two weeks after the last treatment or when tumors exceeded 2000 mm³. Tumor growth was assessed and expressed either as a ratio of the tumor volume divided by initial tumor volume (100 mm³) or as total tumor volume. EA2 inhibited growth of MDA-MB-231 cells implanted orthotopically (FIG. 4A) or subcutaneously (FIG. 4B, D). Growth of A549 cells implanted subcutaneously was also inhibited by EA2 (FIG. 4C).

### 6.3 Estrogen Dependence in Breast Cancer Cells

Estrogen-sensitive breast cancer cells, MCF-7 cells, were transfected with and stably overexpressed human EphA2 (MCF-7^{EPhA2}) (pNeoMSV-EphA2 provided by Dr. T. Hunter, Scripps Institute). Western blot analyses confirmed the ectopic overexpression of EphA2 in transfected cells relative to matched controls (data not shown).

EphA2 overexpression increased malignant growth (FIGS. 5A-5B). Growth assays were conducted as follows. MCF-7^{neo} (control cells) or MCF7^{EphA2} cells were seeded in 96-well plates. Cell growth was measured with Alamar blue (Biosource International, Camarillo, CA) following the manufacture's suggestion. Colony formation in soft agar was performed as previously described (Zelinski et al., 2001, Cancer Res. 61:2301-6) and scored microscopically, defining clusters of at least three cells as a positive. The data represent the average of ten separate high-power microscopic fields from each sample and representative of at least three separate experiments. Error bars represent the standard error of the mean of at least three different experiments as determined using Microsoft Excel software.

Although MCF-7 control cells were largely unable to colonize soft agar (an average of 0.1 colony/field), MCF-7^{EphA2} cells formed larger and more numerous colonies (4.7 colonies/field; P < 0.01) that persisted for at least three weeks (FIG. 5A and data not shown). Despite increased colonization of soft agar, the growth of MCF-7^{EphA2} cells in monolayer culture did not differ from matched controls (FIG. 5B), thus indicating that the growth promoting activities of EphA2 were most apparent using experimental conditions that model anchorage-independent (malignant) cell growth.

Consistent with increased soft agar colonization, orthotopically implanted MCF-7^{EphA2} cells formed larger, more rapidly growing tumors *in vivo*. Six to eight week-old athymic (*nu*/*nu*) mice were purchased from Harlan Sprague Dawley (Indianapolis, IN). When indicated, a controlled release estradiol pellet (0.72 mg 17β-estradiol, 60-day formulation) was injected subcutaneously via a sterile 14-gauge trocar 24 hours prior to tumor implantation and pellets were replaced every 60 days for those experiments spanning > 60 days in duration. 1x10⁶ MCF-7^{neo} or MCF7^{EphA2} cells were injected into the mammary fat pad under direct visualization. When indicated, tamoxifen (1 mg) was administered by oral gavage 6 days per week.

In the presence of supplemental estrogen (17β-estradiol purchased from Sigma), the MCF-7^{EphA2} cells demonstrated a two-fold increase in tumor volume relative to matched controls (FIG. 6A). EphA2-overexpressing tumors differed phenotypically from control tumors in that they were more vascular and locally invasive at the time of resection (data not shown). To confirm that these tumors expressed EphA2, whole cell lysates of resected tumors were subjected to western blot analyses with EphA2-specific antibodies (FIG. 6B). The membranes were then stripped and reprobed with β-catenin antibodies to verify equal sample loading. The relative amount of EphA2 was higher in tumor samples than in the input cells (prior to implantation), suggesting that tumors arose from cells with high levels of EphA2. Comparable findings with *in vitro* and *in vivo* models indicate that EphA2 overexpression results in a more aggressive phenotype.

Parallel studies were performed in the absence of exogenous estrogen. Experimental deprivation of estrogen amplified differences between the cellular behaviors of control and MCF-7^{EPhA2} cells. While MCF-7^{EphA2} cells continued to colonize soft agar more efficiently than matched controls (FIG. 7A), these cells did grow in the absence of exogenous estrogen (FIG. 7B). In contrast, supplemental estrogen was required for monolayer growth of control cells (FIG. 7B). Additionally, MCF-7^{EphA2} cells retained tumorigenic potential in the absence of supplemental estrogen. While control MCF-7 cells rarely formed palpable tumors, the MCF-7^{EphA2} cells formed tumors that persisted for over 12 weeks (FIG. 7C and data not shown). Thus, both *in vitro* and *in vivo* assay systems confirm that EphA2 overexpression decreases the need for exogenous estrogen.

Sensitivity of MCF-7^{EphA2} cells to tamoxifen was measured. Tamoxifen (4-hydroxy tamoxifen purchased from Sigma) reduced soft agar colonization of control MCF-7 cells by at least 60%. The inhibitory actions of tamoxifen on MCF-7^{EPhA2} cells were less pronounced (25% inhibition, FIG. 8A). Notably, excess estradiol overcame the inhibitory effects of tamoxifen, which provided additional evidence for the specificity of this finding (FIG. 8A). Similarly, the tumorigenic potential of MCF-7^{EphA2} cells was less sensitive to tamoxifen as compared with control (MCF-7^{neo}) cells (FIG. 8B).

Since tamoxifen sensitivity often relates to estrogen receptor expression, estrogen receptor expression and activity was assayed in MCF-7^{EphA2}. Western blot analyses revealed comparable levels of ERα and ERβ in control and MCF-7^{EphA2} cells (FIGS. 9A-9B) (ERα and ERβ antibodies were purchased from Chemicon, Temecula, CA). Moreover, comparable levels of estrogen receptor activity were detected in control and MCF-7^{EPhA2} cells and this enzymatic activity remained sensitive to tamoxifen (FIGS. 9E- 9F). Estrogen receptor activity was measured using ERE-TK-CAT vector (which encodes a single ERE; a generous gift from Dr. Nakshatri, Indiana University School of Medicine) in the unstimulated state, after estradiol (10⁻⁸ M) stimulation and tamoxifen (10⁻⁶ M) inhibition. Cells were plated in phenol red free, charcoal stripped sera for 2 days and transfected with ERE-TK-CAT (5 µg) using calcium phosphate method. The β-galactosidase expression vector RSV/β-galactosidase (2 µg, Dr. Nakshatri's gift) was cotransfected as a control. Fresh media including the appropriate selection drugs were added 24 hours after transfection. Cells were harvested after 24 hours and CAT activity was evaluated as described (Nakshatri et al., 1997, Mol. Cell. Biol. 17:3629-39). These results indicate that the estrogen receptor in MCF-7^{EphA2} cells is expressed and remains sensitive to tamoxifen, thus suggesting that the defect which renders MCF-7^{EphA2} less dependent on estrogen lies downstream of estrogen signaling.

Growth MCF-7^{EphA2} cells which had decreased EphA2 expression levels was assayed in soft agar. The EphA2 monoclonal antibody EA2 induced EphA2 activation and subsequent degradation. Decreased levels of EphA2 expression were observed within two hours of EA2 treatment and EphA2 remained undetectable for at least the following 24 hours (FIG. 10A). The soft agar colonization of control MCF-7 cells was sensitive to tamoxifen (FIG. 10C) and EA2 did not further alter this response (since these cells lack of endogenous EphA2). The MCF-7^{EphA2} cells were less sensitive to tamoxifen (25% inhibition by tamoxifen) as compared to the matched controls (75% inhibition by tamoxifen). Whereas EA2 decreased soft agar colonization (by 19%), the combination of EA2 and tamoxifen caused a much more dramatic (>80%) decrease in soft agar colonization. Thus, EA2 treatment restored a phenotype that was comparable to control MCF-7 cells. These findings suggest that antibody targeting of EphA2 can serve to re-sensitize the breast tumor cells to tamoxifen.

All statistical analyses were performed using Student's t-test using Microsoft Excel (Seattle, WA), defining P<0.05 as significant. *In vivo* tumor growth analyses were performed using GraphPad Software (San Diego, CA).

### 6.4 Expression of EphA2 in Prostatic Intraepithelial Neoplasia

EphA2 immunoreactivity distinguished neoplastic prostatic epithelial cells from their non-neoplastic counterparts. Ninety-three cases of radical retropubic prostatectomy were obtained from the surgical pathology files of Indiana University Medical Center. Patients ranged in age from 44 to 77 years (mean = 63 years). Grading of the primary tumor from radical prostatectomy specimens was performed according to the Gleason system (Bostwick "Neoplasms of the prostate" in Bostwick and Eble, eds., 1997, Urologic Surgical Pathology St. Louis:Mosby page 343-422; Gleson and Mellinger, 1974, J. Urol. 111:58-64). The Gleason grade ranged from 4 to 10. Pathological stage was evaluated according to the 1997 TNM (tumor, lymph nodes, and metastasis) standard (Fleming et al., 1997, AJCC Cancer Staging Manual. Philadelphia:Raven and Lippincott). Pathological stages were T2a (n= 9 patients), T2b (n= 43), T3a (n= 27), T3b (n=14). Thirteen patients had lymph node metastasis at the time of surgery.

Serial 5 µm-thick sections of formalin-fixed slices of radical prostatectomy specimens were used for immunofluorescent staining. Tissue blocks that contained the maximum amount of tumor and highest Gleason grade were selected. One representative slide from each case was analyzed. Slides were deparaffinized in xylene twice for 5 minutes and rehydrated through graded ethanols to distilled water. Antigen retrieval was carried out by heating sections in EDTA (pH 8.0) for 30 minutes. Endogenous peroxidase activity was inactivated by incubation in 3% H₂O₂ for 15 minutes. Non-specific binding sites were blocked using Protein Block (DAKO) for 20 minutes. Tissue sections were then incubated with a mouse monoclonal antibody against human EphA2 (IgG1, 1:100 dilution) overnight at room temperature, followed by biotinylated secondary antibody (DAKO corporation, Carpintera, CA) and peroxidase-labeled streptavidin, and 3,3-diaminobenzidine was used as the chromogen in the presence of hydrogen peroxide. Positive and negative controls were run in parallel with each batch.

The extent and intensity of staining were evaluated in benign epithelium, high-grade prostatic intraepithelial neoplasia (PIN) and adenocarcinoma from the same slide for each case. Microscopic fields with highest degree of immunoreactivity were chosen for analysis. At least 1000 cells were analyzed in each case. The percentage of cells exhibiting staining in each case was evaluated semiquantitatively on a 5% incremental scale ranging from 0 to 95%. A numeric intensity score is set from 0 to 3 (0, no staining; 1 weak staining; 2 moderate staining; and 3, strong staining) (Jiang et al., 2002, Am. J. Pathol. 160:667-71; Cheng et al., 1996, Am J. Pathol. 148:1375-80).

The mean percentage of immunoreactive cells in benign epithelium, high-grade PIN and adenocarcinoma were compared using the Wilcoxon paired signed rank test. The intensity of staining for EphA2 in benign epithelium, high-grade PIN, and adenocarcinoma was compared using Cochran-Mantel-Haenszel tests for correlated ordered categorical data. Pairwise comparisons were made if the ANOVA revealed significant differences. A p-value<0.05 was considered significant, and all p-values were two-sided.

EphA2 immunoreactivity was observed in all cases of high-grade prostatic intraepithelial neoplasia (PIN) and cancers but not in benign epithelial cells. For example, EphA2 expression (both the mean percentage of immunoreactive cells and staining intensity) was increased in both high-grade PIN and cancers relative to benign epithelial cells (Tables 3 and 4). Similarly, EphA2 immunoreactivity (both the mean percentage of immunoreactive cells and staining intensity) was increased in prostatic carcinomas compared with high-grade PIN (Tables 3 and 4). This immunoreactivity was evident at the membrane and cytoplasm of the neoplastic epithelial cells (data not shown). In contrast, no EphA2 immunoreactivity was observed in tumor-proximal stromal cells. In the high-grade PIN group, 22% showed grade 1 staining intensity, 73% showed grade 2 staining intensity, and 5% showed grade 3 staining intensity (Table 3). In the adenocarcinoma group, 13% of cases showed grade 1 staining intensity, 50% showed grade 2 staining intensity, and 37% showed grade 3 staining intensity. In contrast, the normal epithelium group showed grade 1 stain in 66% of the cases, the remaining cases showed no immunoreactivity for EphA2 protein (grade 0 staining intensity) (Table3). The mean percentage of EphA2 immunoreactive cells was 12% in the normal epithelial cells, 67% in the high-grade PIN, and 85% in the prostatic adenocarcinoma (Table 4).

Although high levels of EphA2 could distinguish neoplastic from benign prostatic epithelial cells, EphA2 did not correlate with other histologic and pathologic parameters of disease severity. For example, high levels of EphA2 were observed in most prostatic carcinomas and did not relate to Gleason grade, pathologic stage, lymph node metastasis, extraprostatic extension, surgical margins, vascular invasion, perineural invasion, or the presence of other areas of the prostate with high-grade PIN (Table 5).]

**TABLE 3**

| Cell Type | Staining Intensity Grade | | | |
|---|---|---|---|---|
| | 0 | 1 | 2 | 3 |
| Benign epithelium | 31(33%) | 61(66%) | 1(1%) | 0(0%) |
| High-grade PIN^{a} | 0 (0%) | 20 (22%) | 68 (73%) | 5(5%) |
| Adenocarcinoma^{a,b} | 0(0%) | 12 (13%) | 47(50%). | 34 (37%) |

| | | | | |
|---|---|---|---|---|
| ^{a}Indicates percentage of staining intensity was statistically lower compared to that of the normal cells with a P-value = 0.0001 using a Wilcoxon paired signed rank test. ^{b}The staining intensity was significantly higher compared to high-grade PIN (P<0.01, Cochran-Mantel-Henszel test). | | | | |

**TABLE 4**

| Cell Type | Mean % of Cells Staining ± SD | Range (%) |
|---|---|---|
| Normal Cells | 12±17 | 0-90 |
| High-grade PIN | 67±18^{a} | 5-95 |
| Adenocarcinoma | 85±12^{a,b} | 30-95 |

| | | |
|---|---|---|
| ^{a}Indicates percentage of staining statistically lower compared to that of the normal cells with a P-value = 0.0001 using a Wilcoxon paired signed rank test. ^{b}The percentage of staining was statistically higher compared to high-grade PIN (P<0.01, ANOVA). | | |

**TABLE 5**

| Patient Characteristic | | % of Total Patients (n=93) | Mean % of Cells Staining w/EphA2 Antibody (±SD) | Mean EphA2 Antibody Staining Intensity (±SD) |
|---|---|---|---|---|
| Primary Gleason Grade | | | | |
| | 2 | 12 | 83±2 | 2.0±0.6 |
| | 3 | 43 | 86±10 | 2.3±0.7 |
| | 4 | 23 | 84±16 | 2.3±0.7 |
| | 5 | 15 | 86±11 | 2.3±0.6 |
| Secondary Gleason Grade | | | | |
| | 2 | 15 | 82±16 | 2.3±0.5 |
| | 3 | 29 | 85±15 | 2.1±0.6 |
| | 4 | 35 | 85±9 | 2.3±0.7 |
| | 5 | 14 | 88±8 | 2.4±0.8 |
| Gleason Sum | <7 | 28 | 83±12 | 2.2±0.6 |
| | 7 | 35 | 85±14 | 2.2±0.7 |
| | >7 | 30 | 87±10 | 2.4±0.7 |
| T Classification | T2a | 9 | 89±6 | 2.3±0.5 |
| | T2b | 43 | 84±12 | 2.2±0.7 |
| | T3a | 27 | 84±15 | 2.2±0.7 |
| | T3b | 14 | 63±10 | 2.4±0.6 |
| Lymph Node Metastasis | | | | |
| | Positive | 13 | 88±9 | 2.3±0.6 |
| | Negative | 80 | 84±13 | 2.2±0.7 |
| Extraprostatic Extension | | | | |
| | Positive | 53 | 86±11 | 2.3±0.7 |
| | Negative | 40 | 84±14 | 2.2±0.7 |
| Surgical Margin | Positive | 50 | 86±11 | 2.1±0.6 |
| | Negative | 43 | 84±13 | 2.4±0.7 |
| Vascular Invasion | Positive | 30 | 85±11 | 2.1±0.8 |
| | Negative | 63 | 86±13 | 2.3±0.6 |
| Perineural Invasion | Positive | 82 | 82±15 | 2.4±0.5 |
| | Negative | 11 | 85±12 | 2.2±0.7 |
| High-grade PIN | Positive | 89 | 85±12 | 2.3±0.7 |
| | Negative | 4 | 85±9 | 2.0±0.8 |

### 6.5 Treatment Of Patients With Metastatic Cancer

A study is designed to assess pharmacokinetics and safety of agonistic monoclonal antibodies of the invention in patients with metastatic breast cancer. Cancer patients currently receive Taxol or Taxotere. Patients currently receiving treatment are permitted to continue these medications.

Patients are administered a single IV dose of a monoclonal antibody of the invention and then, beginning 4 weeks later, are analyzed following administration of repeated weekly IV doses at the same dose over a period of 12 weeks. The safety of treatment with the agonistic monoclonal antibody of the invention is assessed as well as potential changes in disease activity over 26 weeks of IV dosing. Different groups of patients are treated and evaluated similarly but receive doses of 1 mg/kg, 2 mg/kg, 4 mg/kg, or 8 mg/kg.

Antibodies of the invention are formulated at 5 mg/ml and 10 mg/ml for IV injection. A formulation of 80 mg/ml is required for repeated subcutaneous administration. The antibodies of the invention are also formulated at 100 mg/ml for administration for the purposes of the study.

Changes are measured or determined by the progression of tumor growth.

### 6.6 Epitope Analysis of EphA2 Antibodies

The epitope of the EphA2 antibodies were characterized. EA2 and EA5 selectively bind to malignant cells. The anti-EphA2 monoclonal antibodies EA2 and EA5 bind malignant MDA-MB-231 breast epithelial tumor cells (FIGS. 11A-11B) more strongly than benign MCF-10A breast epithelial tumor cells (FIGS. 11C-11D) as shown by immunofluorescent staining. Furthermore, EA2 was immunoreactive against malignant prostate cells. The anti-EphA2 monoclonal antibody EA2 identified malignant prostate cancer cells in formalin-fixed, paraffin-embedded archival clinical specimens (FIG. 12).

EA2 preferentially binds an EphA2 epitope exposed on cancer cells but not non-cancer cells. Non-transformed MCF-10A cells or transformed MDA-MB-231 cells were incubated with 10 µg/ml EA2 at 4°C for 30 min. prior to fixation in a 3% formalin solution and immunolabeling with fluorophore-conjugated anti-mouse IgG. EA2 preferentially binds EphA2 on transformed cells (FIG. 13D). In contrast, another EphA2 antibody Eph099B-233.152 (ATCC deposit no. _; see co-pending US Patent Application Serial No._, entitled "EphA2 Monoclonal Antibodies and Methods of Use Thereof" filed May 12, 2003 as Attorney Docket No. 10271-097-999) binds EphA2 expressed on both transformed and non-transformed cells (FIGS. 13A-13B). Treatment of non-transformed MCF-10A cells with 4mM EGTA for 20 min. dissociated the cells. EA2 bound EphA2 on the EGTA dissociated cells but not the untreated cells (FIGS. 14A-14B).

An equivalent experiment was performed using MCF-10A or MDA-MB-231 cells. The amount of EA2 binding to EphA2 was measured using flow cytometry (FIGS. 17C-17D). Cells were either treated by incubation in 4 mM EGTA for 10-15 minutes on ice (top panel) or were not treated with EGTA (middle panel) before incubation with 10 µg/ml EA2. Cells were then fixed with 3% formalin and labeled with fluorophore-labeled donkey anti-mouse IgG. Control cells were incubated only with secondary antibody (fluorophore-labeled donkey anti-mouse IgG) in the absence of primary antibody (EA2) (bottom panel). The samples were then evaluated using flow cytometry (Becton Dickinson FACStar Plus). EGTA treatment did not affect EA2 binding to transformed cells (FIG. 17D, top and middle panels). In contrast, EA2 binding to non-transformed cells was increased by incubation in EGTA (FIG. 17C, top and middle panels).

EA2 does not bind the same epitope as the EphA2 ligand Ephrin A1. A microtiter plate was coated with 10 mg/ml Ephrin A1-F_{c} overnight at 4°C. A fusion protein consisting of the extracellular domain of EphA2 linked to human IgG₁ constant region (EphA2-F_{c}) was incubated with and bound to the immobilized Ephrin A1-F_{c}. Biotinylated Ephrin A1-F_{c} or EA2 was incubated with the EphA2-Ephrin A1-F_{c} complex and amount of binding was measured. Very little additional Ephrin A1-F_{c} bound the EphA2-Ephrin A1-F_{c} complex while, in contrast, considerable levels of EA2 bound the EphA2-phrinA1- F_{c} complex (FIG. 15A).

The EphA2-Ephrin A1-F_{c} complex was prepared as described above. Biotinylated EA2 (10 µg/ml) was then incubated with the complex for 30 min. Unlabeled competitor was incubated with EphA2-Ephrin A1-F_{c}-EA2 complex in the indicated amount. Unlabeled EA2 could displace the labeled EA2 at concentrations of 100 ng/ml or greater. Unlabeled Ephrin A1-Fc did not significantly displace labeled EA2 (FIG 15B).

### 7. EQUIVALENT

Those skilled in the art will recognize, or be able to ascertain using no more than routine experimentation, many equivalents to the specific embodiments of the invention described herein.

## Claims

1. An isolated antibody that specifically binds to EphA2 and induces phosphorylation of EphA2, wherein:
a. the antibody competes for binding to EphA2 with the antibody produced by the hybridoma deposited with American Type Culture Collection (ATCC) and assigned accession no. PTA-4380;
b. the antibody competes for binding to EphA2 with the antibody produced by the hybridoma deposited with ATCC and assigned accession no. PTA-4381;
c. the antibody is produced by the hybridoma deposited with the ATCC and assigned accession no. PTA-4380;
d. the antibody is produced by the hybridoma deposited with the ATCC and assigned accession no. PTA-4381;
e. the antibody comprises a VH chain comprising the amino acid sequence of SEQ ID NO:5 and a VL chain comprising the amino acid sequence of SEQ ID NO:1;
f. the antibody comprises a VL CDR1 having the amino acid sequence of SEQ ID NO:2, a VL CDR2 having the amino acid sequence of SEQ ID NO:3, a VL CDR3 having the amino acid sequence of SEQ ID NO:4, a VH CDR1 having the amino acid of SEQ ID NO: 6, a VH CDR2 having the amino acid sequence of SEQ ID NO:7, and a VH CDR3 having the amino acid sequence of SEQ ID NO:8; or
g. the antibody comprises a VL CDR1 having the amino acid sequence of SEQ ID NO:2, a VL CDR2 having the amino acid sequence of SEQ ID NO:3, a VL CDR3 having the amino acid sequence of SEQ ID NO:4, a VH CDR1 having the amino acid of SEQ ID NO:6, a VH CDR2 having the amino acid sequence of SEQ ID NO:7, and a VH CDR3 having the amino acid sequence of SEQ ID NO:8, wherein one or more of the CDRs has one, two, or three mutations.

2. The antibody of claim 1, wherein the antibody is produced by the hybridoma deposited with the ATCC and assigned accession no. PTA-4380.

3. The antibody of claim 1, wherein the antibody is produced by the hybridoma deposited with the ATCC and assigned accession no. PTA-4381.

4. The antibody of claim 1, wherein the antibody comprises a VH chain comprising the amino acid sequence of SEQ ID NO:5.

5. The antibody of claim 1, wherein the antibody comprises a VL chain comprising the amino acid sequence of SEQ ID NO: 1.

6. The antibody of claim 1, wherein the antibody comprises a VH chain comprising the amino acid sequence of SEQ ID NO:5 and a VL chain comprising the amino acid sequence of SEQ ID NO:1.

7. The antibody of claim 1, wherein the antibody comprises a VL CDR1 having the amino acid sequence of SEQ ID NO:2, a VL CDR2 having the amino acid sequence of SEQ ID NO:3, a VL CDR3 having the amino acid sequence of SEQ ID NO:4, a VH CDR1 having the amino acid of SEQ ID NO: 6, a VH CDR2 having the amino acid sequence of SEQ ID NO:7, and a VH CDR3 having the amino acid sequence of SEQ ID NO:8.

8. The antibody of claim 4, 5 or 6, wherein the antibody is a monoclonal antibody, a chimeric antibody, a single chain Fv (scFv), a single chain antibody, a Fab fragment (Fab), or a F(ab')₂.

9. The antibody of claim 7, wherein the antibody is a monoclonal antibody, a humanized antibody, a chimeric antibody, a single chain Fv (scFv), a single chain antibody, a Fab fragment (Fab), or a F(ab')₂.

10. An antibody conjugate comprising the antibody of any one of the preceding claims conjugated or fused to a heterologous polypeptide.

11. An antibody conjugate comprising the antibody of any one of claims 1 to 9 conjugated or fused to a diagnostic or detectable agent.

12. An antibody conjugate comprising the antibody of any one of claims 1 to 9 conjugated or fused to a therapeutic agent.

13. The antibody conjugate of claim 12, wherein the therapeutic agent is a cytotoxin.

14. The antibody conjugate of claim 13, wherein the cytotoxin is paclitaxel, chytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1 -dehydrotestosterone, glucocorticoids, procaine, tetracaine, lidocaine, propanolol, puromycin, epirubicin, or cyclophosphamide.

15. The antibody conjugate of claim 12, wherein the therapeutic agent is abrin, ricin A, Pseudomonas exotoxin, cholera toxin, diphtheria toxin, tumor necrosis factor alpha, tumor necrosis factor beta, interferon alpha, interferon beta, or platelet derived growth factor.

16. The antibody conjugate of claim 12, wherein the therapeutic agent is a radioactive material or macrocyclic chetalor.

17. An isolated nucleic acid comprising a nucleotide sequence encoding the antibody of claim 2, 3 or 6.

18. A vector comprising the nucleic acid of claim 17.

19. The vector of claim 18 further comprising a nucleotide sequence which regulates the expression of the nucleotide sequence.

20. A host cell genetically engineered to contain or express the nucleic acid of claim 17.

21. A host cell genetically engineered to contain the vector of claim 18 or 19.

22. A pharmaceutical composition comprising the antibody of any one of claims 1 to 9 and a pharmaceutically acceptable carrier.

23. Use of the pharmaceutical composition of claim 22 in the manufacture of a medicament for the treatment of cancer, wherein the cancer is associated with the overexpression of EphA2.

24. The pharmaceutical composition of claim 22, for use in treating cancer, wherein the cancer is associated with the overexpression of EphA2.

25. A pharmaceutical composition comprising the antibody conjugate of any one of claims 10 to 16 and a pharmaceutically acceptable carrier.

26. Use of the pharmaceutical composition of claim 24 in the manufacture of a medicament for the treatment of cancer, wherein the cancer is associated with the overexpression of EphA2.

27. The pharmaceutical composition of claim 24, for use in treating cancer, wherein the cancer is associated with the overexpression of EphA2.

28. The antibody of any one of claims 1 to 9 for use in the treatment of cancer in a patient, wherein the cancer is associated with the overexpression of EphA2.

29. Use of the antibody of any one of claims 1 to 9 in the preparation of a medicament for the treatment of cancer in a patient, wherein the cancer is associated with the overexpression of EphA2.

30. The antibody conjugate of any one of claims 10 to 16 for use in the treatment of cancer in a patient, wherein the cancer is associated with the overexpression of EphA2.

31. Use of the antibody conjugate of any one of claims 10 to 16 in the preparation of a medicament for the treatment of cancer in a patient, wherein the cancer is associated with the overexpression of EphA2.

32. Use of claim 29 or 31, wherein the medicament further comprises an additional therapy.

33. A method for diagnosing cancer in a patient, comprising:
a. contacting cells from said patient with the antibody of any one of claims 1 to 9 under conditions appropriate for antibody-EphA2 binding; and
b. measuring EphA2 antibody binding to said cells, wherein a higher EphA2 antibody binding level than a control indicates that the patient has cancer.

34. Use of the antibody of any one of claims 1 to 9 in the preparation of a medicament for diagnosing, prognosing or monitoring the efficacy of therapy for cancer in a patient, wherein the cancer is associated with the overexpression of EphA2.

35. Use of the antibody conjugate of any one of claims 10 to 16 in the preparation of a medicament for diagnosing, prognosing or monitoring the efficacy of therapy for cancer in a patient, wherein the cancer is associated with the overexpression of EphA2.

36. Use of claim 23, 29 or 34 or composition of claim 24, wherein the cancer is of an epithelial origin.

37. Use of claim 26, 31 or 35 or composition of claim 27, wherein the cancer is of an epithelial origin.

38. Use of claim 23, 29 or 34 or composition of claim 24, wherein the cancer is a renal cell carcinoma, melanoma, a metastatic cancer, or a cancer of the skin, lung, colon, breast, prostate, bladder, kidney, or pancreas.

39. Use of claim 26, 31 or 35 or composition of claim 27, wherein the cancer is a renal cell carcinoma, melanoma, a metastatic cancer, or a cancer of the skin, lung, colon, breast, prostate, bladder, kidney, or pancreas.

40. Use of claim 23, 26, 29, 31, 34 or 35, wherein the patient is a human.

41. The composition of claims 24 and 27, wherein the patient is human.

42. Use of claim 40, wherein the human is refractory to a previous therapy.

43. The composition of claim 41, wherein the human is refractory to a previous therapy.

44. The method of claim 33, wherein the cancer is of an epithelial origin.

45. The method of claim 33, wherein the cancer is a renal cell carcinoma, melanoma, a metastatic cancer, or a cancer of the skin, lung, colon, breast, prostate, bladder, kidney, or pancreas.

46. The method of claim 33, wherein the cells are from whole blood, sputum, urine. serum, or fine needle aspirates of tumor cell tissue.

47. The method of claim 33, wherein the patient is a human.

48. A hybridoma deposited with the ATCC and assigned accession no. PTA-4380.

49. A hybridoma deposited with the ATCC and assigned accession no. PTA-4381.

50. A method for producing an antibody, comprising culturing the host cell of any one of claims 20 or 21 under conditions in which the nucleic acid is expressed.

51. A kit comprising the antibody of any one of claims 1 to 9, in a container, and instructions for use.

52. A kit comprising the antibody conjugate of any one of claims 1 to 16, in a container, and instructions for use.

53. The antibody of claim 7, wherein the antibody is a humanized antibody or a chimeric antibody.

## Patentansprüche

1. Isolierter Antikörper, der spezifisch an EphA2 bindet und die Phosphorylierung von EphA2 induziert, wobei:
a. der Antikörper um die Bindung an EphA2 mit dem Antikörper konkurriert, der durch das Hybridom gebildet ist, das bei der American Type Culture Collection (ATCC) hinterlegt und dem die Beitrittsnummer PTA-4380 zugewiesen worden ist;
b. der Antikörper um die Bindung an EphA2 mit dem Antikörper konkurriert, der durch das Hybridom gebildet ist, das bei ATCC hinterlegt und dem die Beitrittsnummer PTA-4381 zugewiesen worden ist;
c. der Antikörper durch das Hybridom gebildet ist, das bei der ATCC hinterlegt und dem die Beitrittsnummer PTA-4380 zugewiesen worden ist;
d. der Antikörper durch das Hybridom gebildet ist, das bei der ATCC hinterlegt und dem die Beitrittsnummer PTA-4381 zugewiesen worden ist;
e. der Antikörper eine VH-Kette, die die Aminosäuresequenz von SEQ ID NO: 5 umfasst, und eine VL-Kette umfasst, die die Aminosäuresequenz von SEQ ID NO: 1 umfasst;
f. der Antikörper eine VL CDR1, die die Aminosäuresequenz von SEQ ID NO: 2 aufweist, eine VL CDR2, die die Aminosäuresequenz von SEQ ID NO: 3 aufweist, eine VL CDR3, die die Aminosäuresequenz von SEQ ID NO: 4 aufweist, eine VH CDR1, die die Aminosäure von SEQ ID NO: 6 aufweist, eine VH CDR2, die die Aminosäuresequenz von SEQ ID NO: 7 aufweist und eine VH CDR3, die die Aminosäuresequenz von SEQ ID NO: 8 aufweist, umfasst; oder
g. der Antikörper eine VL CDR1, die die Aminosäuresequenz von SEQ ID NO: 2 aufweist, eine VL CDR2, die die Aminosäuresequenz von SEQ ID NO: 3 aufweist, eine VL CDR3, die die Aminosäuresequenz von SEQ ID NO: 4 aufweist, eine VH CDR1, die die Aminosäure von SEQ ID NO: 6 aufweist, eine VH CDR2, die die Aminosäuresequenz von SEQ ID NO: 7 aufweist und eine VH CDR3, die die Aminosäuresequenz von SEQ ID NO: 8 aufweist, umfasst, wobei eine oder mehrere der CDR eine, zwei oder drei Mutationen aufweist/aufweisen.

2. Antikörper nach Anspruch 1, wobei der Antikörper durch das Hybridom gebildet ist, das bei der ATCC hinterlegt worden und dem die Beitrittsnummer PTA-4380 zugewiesen worden ist.

3. Antikörper nach Anspruch 1, wobei der Antikörper durch das Hybridom gebildet ist, das bei der ATCC hinterlegt worden und dem die Beitrittsnummer PTA-4381 zugewiesen worden ist.

4. Antikörper nach Anspruch 1, wobei der Antikörper eine VH-Kette umfasst, die die Aminosäuresequenz von SEQ ID NO: 5 umfasst.

5. Antikörper nach Anspruch 1, wobei der Antikörper eine VL-Kette, die die Aminosäuresequenz von SEQ ID NO: 1 umfasst, umfasst

6. Antikörper nach Anspruch 1, wobei der Antikörper eine VH-Kette, die die Aminosäuresequenz von SEQ ID NO: 5 umfasst, und eine VL-Kette umfasst, die die Aminosäuresequenz von SEQ ID NO: 1 umfasst.

7. Antikörper nach Anspruch 1, wobei der Antikörper eine VL CDR1, die die Aminosäuresequenz von SEQ ID NO: 2 aufweist, eine VL CDR2, die die Aminosäuresequenz von SEQ ID NO: 3 aufweist, eine VL CDR3, die die Aminosäuresequenz von SEQ ID NO: 4 aufweist, eine VH CDR1, die die Aminosäure von SEQ ID NO: 6 aufweist, eine VH CDR2, die die Aminosäuresequenz von SEQ ID NO: 7 aufweist und eine VH CDR3, die die Aminosäuresequenz von SEQ ID NO: 8 aufweist, umfasst.

8. Antikörper nach Anspruch 4, 5 oder 6, wobei der Antikörper ein monoklonaler Antikörper, ein chimärer Antikörper, ein Einketten-Fv (scFv), ein Einkettenantikörper, ein Fab-Fragment (Fab) oder ein F(ab')₂ ist.

9. Antikörper nach Anspruch 7, wobei der Antikörper ein monoklonaler Antikörper, ein humanisierter Antikörper, ein chimärer Antikörper, ein Einketten-Fv (scFv), ein Einkettenantikörper, ein Fab-Fragment (Fab) oder ein F(ab')₂ ist.

10. Antikörperkonjugat umfassend den Antikörper nach einem der vorhergehenden Ansprüche, der an ein heterologes Polypeptid konjugiert oder fusioniert ist.

11. Antikörperkonjugat umfassend den Antikörper nach einem der Ansprüche 1 bis 9, der an ein diagnostisches oder erfassbares Mittel konjugiert oder fusioniert ist.

12. Antikörperkonjugat umfassend den Antikörper nach einem der Anspruche 1 bis 9, der an ein therapeutisches Mittel konjugiert oder fusioniert ist.

13. Antikörperkonjugat nach Anspruch 12, wobei das therapeutische Mittel ein Zytotoxin ist.

14. Antikörperkonjugat nach Anspruch 13, wobei das Zytotoxin Paclitaxel, Chytochalasin B, Gramicidin D, Ethidiumbromid, Emetin, Mitomycin, Etoposid, Tenoposid, Vinkristin, Vinblastin, Colchicin, Doxorubicin, Daunorubicin, Dihydroxyanthracindion, Mitoxantron, Mithramycin, Actinomycin D, 1-Dehydrotestosteron, Glucocorticoide, Procain, Tetracain, Lidocain, Propanolol, Puromycin, Epirubicin oder Cyclophosphamid ist.

15. Antikörperkonjugat nach Anspruch 12, wobei das therapeutische Mittel Abrin, Rizin A, Pseudomonas exotoxin, Choleratoxin, Diptherietoxin, Tumornekrosefaktor-Alpha, Tumornekrosefaktor-Beta, Interferon-Alpha, Interferon-Beta oder von Plättchen abgeleiteter Wachstumsfaktor ist.

16. Antikörperkonjugat nach Anspruch 12, wobei das therapeutische Mittel ein radioaktives Material oder makrocyclischer Chetalor ist.

17. Isolierte Nucleinsäure umfassend eine Nucleotidsequenz, die für den Antikörper nach Anspruch 2, 3 oder 6 kodiert.

18. Vektor umfassend die Nucleinsäure nach Anspruch 17.

19. Vektor nach Anspruch 18, des Weiteren eine Nucleotidsequenz umfassend, die die Expression der Nucleotidsequenz reguliert.

20. Wirtszelle, die genmanipuliert ist, um die Nucleinsäure nach Anspruch 17 zu enthalten oder zu exprimieren.

21. Wirtszelle, die genmodifiziert ist, um den Vektor nach Anspruch 18 oder 19 zu enthalten.

22. Pharmazeutische Zusammensetzung umfassend den Antikörper nach einem der Ansprüche 1 bis 9 und einen pharmazeutisch akzeptablen Träger.

23. Verwendung der pharmazeutischen Zusammensetzung nach Anspruch 22 bei der Herstellung eines Medikaments für die Behandlung von Krebs, wobei der Krebs mit der Überexpression von EphA2 verbunden ist.

24. Pharmazeutische Zusammensetzung nach Anspruch 22 zur Verwendung bei der Behandlung von Krebs, wobei der Krebs mit der Überexpression von EphA2 verbunden ist.

25. Pharmazeutische Zusammensetzung umfassend das Antikörperkonjugat nach einem der Ansprüche 10 bis 16 und einen pharmazeutisch akzeptablen Träger.

26. Verwendung einer pharmazeutischen Zusammensetzung nach Anspruch 24 bei der Herstellung eines Medikaments für die Behandlung von Krebs, wobei der Krebs mit der Überexpression von EphA2 verbunden ist.

27. Pharmazeutische Zusammensetzung nach Anspruch 24 zur Verwendung bei der Behandlung von Krebs, wobei der Krebs mit der Überexpression von EphA2 verbunden ist.

28. Antikörper nach einem der Ansprüche 1 bis 9 zur Verwendung bei der Behandlung von Krebs bei einem Patienten, wobei der Krebs mit der Überexpression von EphA2 verbunden ist.

29. Verwendung des Antikörpers nach einem der Ansprüche 1 bis 9 bei der Herstellung eines Medikaments für die Behandlung von Krebs bei einem Patienten, wobei der Krebs mit der Überexpression von EphA2 verbunden ist.

30. Antikörperkonjugat nach einem der Ansprüche 10 bis 16 zur Verwendung bei der Behandlung von Krebs bei einem Patienten, wobei der Krebs mit der Überexpression von EphA2 verbunden ist.

31. Verwendung des Antikörperkonjugats nach einem der Ansprüche 10 bis 16 bei der Herstellung eines Medikaments für die Behandlung von Krebs bei einem Patienten, wobei der Krebs mit der Überexpression von EphA2 verbunden ist.

32. Verwendung nach Anspruch 29 oder 31, wobei das Medikament des Weiteren eine zusätzliche Therapie umfasst.

33. Verfahren zum Diagnostizieren von Krebs bei einem Patienten, umfassend:
a. das Kontaktieren von Zellen des Patienten mit einem Antikörper nach einem der Ansprüche 1 bis 9 unter Bedingungen, die für die Antikörper-EphA2-Bindung geeignet sind: und
b. das Messen der EphA2-Antikörper-Bindung an die Zellen, wobei ein höheres EphA2-Antikörper-Bindungsniveau als eine Kontrolle anzeigt, dass der Patient an Krebs leidet.

34. Verwendung des Antikörpers nach einem der Ansprüche 1 bis 9 bei der Herstellung eines Medikaments zum Diagnostizieren, Prognostizieren oder Überwachen der Wirksamkeit von Therapie gegen Krebs bei einem Patienten, wobei der Krebs mit der Überexpression von EphA2 verbunden ist

35. Verwendung des Antikörperkonjugats nach einem der Ansprüche 10 bis 16 bei der Herstellung eines Medikaments zum Diagnostizieren, Prognostizieren oder Überwachen der Wirksamkeit von Therapie gegen Krebs bei einem Patienten, wobei der Krebs mit der Überexpression von EphA2 verbunden ist

36. Verwendung nach Anspruch 23, 29 oder 34 oder Zusammensetzung nach Anspruch 24, wobei der Krebs epithelialen Ursprungs ist.

37. Verwendung nach Anspruch 26, 31 oder 35 oder Zusammensetzung nach Anspruch 27, wobei der Krebs epithelialen Ursprungs ist.

38. Verwendung nach Anspruch 23, 29 oder 34 oder Zusammensetzung nach Anspruch 24, wobei der Krebs ein Nierenzellenkarzinom, Melanom, metastatischer Krebs oder Krebs der Haut, Lunge, des Kolon, der Brust, Prostata, Blase, Niere oder Bauchspeicheldrüse ist.

39. Verwendung nach Anspruch 26, 31 oder 35 oder Zusammensetzung nach Anspruch 27, wobei der Krebs ein Nierenzellenkarzinom, Melanom, metastatischer Krebs oder Krebs der Haut, Lunge, des Kolon, der Brust, Prostata, Blase, Niere oder Bauchspeicheldrüse ist

40. Verwendung nach Anspruch 23, 26, 29, 31, 34 oder 35, wobei der Patient ein Mensch ist.

41. Zusammensetzung nach den Ansprüchen 24 und 27, wobei der Patient menschlich ist.

42. Verwendung nach Anspruch 40, wobei der Mensch gegen vorherige Therapie refraktär ist.

43. Zusammensetzung nach Anspruch 41, wobei der Mensch gegen vorherige Therapie refraktär ist.

44. Verfahren nach Anspruch 33, wobei der Krebs epithelialen Ursprungs ist.

45. Verfahren nach Anspruch 33, wobei der Krebs ein Nierenzellenkarzinom, Melanom, ein metastatischer Krebs oder ein Krebs der Haut, Lunge, des Kolon, der Brust, Prostata, Blase, Niere oder Bauchspeicheldrüse ist.

46. Verfahren nach Anspruch 33, wobei die Zellen aus Ganzblut, Speichel, Urin, Serum oder Feinnadelaspiraten von Tumorzellgewebe stammen.

47. Verfahren nach Anspruch 33, wobei der Patient ein Mensch ist.

48. Hybridom, das bei der ATCC hinterlegt und dem die Beitrittsnummer PTA-4380 zugewiesen worden ist.

49. Hybridom, das bei der ATCC hinterlegt und dem die Beitrittsnummer PTA-4381 zugewiesen worden ist.

50. Verfahren zum Herstellen eines Antikörpers, umfassend das Züchten der Wirtszelle nach einem der Ansprüche 20 oder 21 unter Bedingungen, unter denen die Nucleinsäure exprimiert wird.

51. Kit umfassend den Antikörper nach einem der Ansprüche 1 bis 9 in einem Behälter und Gebrauchsanleitungen.

52. Kit umfassend das Antikörperkonjugat nach einem der Ansprüche 11 bis 16 in einem Behälter und Gebrauchsanleitungen.

53. Antikörper nach Anspruch 7, wobei der Antikörper ein humanisierter Antikörper oder ein chimärer Antikörper ist.

## Revendications

1. Anticorps isolé qui se lie spécifiquement à EphA2 et induit une phosphorylation de EphA2, dans lequel:
a. l'anticorps est en concurrence pour la liaison à EphA2 avec l'anticorps produit par l'hybridome déposé à la collection de cultures types américaine (ATCC) et avec le no. d'entrée attribué PTA-4380;
b. l'anticorps est en concurrence pour la liaison à EphA2 avec l'anticorps produit par l'hybridome déposé à l'ATCC et avec le no. d'entrée attribué PTA-4381;
c. l'anticorps est produit par l'hybridome déposé à l'ATCC et avec le no.
d'entrée attribué PTA-4380;
d. l'anticorps est produit par l'hybridome déposé à l'ATCC et avec le no.
d'entrée attribué PTA-4381;
e. l'anticorps comprend une chaîne VH comprenant la séquence d'acides aminés de la SEQ ID NO:5 et une chaîne VL comprenant la séquence d'acides aminés de la SEQ ID NO:1;
f. l'anticorps comprend une CDR1 de VL possédant la séquence d'acides aminés de la SEQ ID NO:2, une CDR2 de VL possédant la séquence d'acides aminés de la SEQ ID NO:3, une CDR3 de VL possédant la séquence d'acides aminés de la SEQ ID NO:4, une CDR1 de VH possédant la séquence d'acides aminés de la SEQ ID NO:6, une CDR2 de VH possédant la séquence d'acides aminés de la SEQ ID NO:7 et une CDR3 de VH possédant la séquence d'acides aminés de la SEQ ID NO:8; ou
g. l'anticorps comprend une CDR1 de VL possédant la séquence d'acides aminés de la SEQ ID NO:2, une CDR2 de VL possédant la séquence d'acides aminés de la SEQ ID NO:3, une CDR3 de VL possédant la séquence d'acides aminés de la SEQ ID NO:4, une CDR1 de VH possédant la séquence d'acides aminés de la SEQ ID NO:6, une CDR2 de VH possédant la séquence d'acides aminés de la SEQ ID NO:7 et une CDR3 de VH possédant la séquence d'acides aminés de la SEQ ID NO:8, où une ou plusieurs des CDRs présentent une, deux ou trois mutations.

2. Anticorps selon la revendication 1, où l'anticorps est produit par l'hybridome déposé à l'ATCC et avec le no. d'entrée attribué PTA-4380.

3. Anticorps selon la revendication 1, où l'anticorps est produit par l'hybridome déposé à l'ATCC et avec le no. d'entrée attribué PTA-4381.

4. Anticorps selon la revendication 1, où l'anticorps comprend une chaîne VH comprenant la séquence d'acides aminés de la SEQ ID NO:5.

5. Anticorps selon la revendication 1, où l'anticorps comprend une chaîne VL comprenant la séquence d'acides aminés de la SEQ ID NO:1.

6. Anticorps selon la revendication 1, où l'anticorps comprend une chaîne VH comprenant la séquence d'acides aminés de la SEQ ID NO:5 et une chaîne VL comprenant la séquence d'acides aminés de la SEQ ID NO:1.

7. Anticorps selon la revendication 1, où l'anticorps comprend une CDR1 de VL possédant la séquence d'acides aminés de la SEQ ID NO:2, une CDR2 de VL possédant la séquence d'acides aminés de la SEQ ID NO:3, une CDR3 de VL possédant la séquence d'acides aminés de la SEQ ID NO:4, une CDR1 de VH possédant la séquence d'acides aminés de la SEQ ID NO:6, une CDR2 de VH possédant la séquence d'acides aminés de la SEQ ID NO:7 et une CDR3 de VH possédant la séquence d'acides aminés de la SEQ ID NO:8.

8. Anticorps selon la revendication 4, 5 ou 6, où l'anticorps est un anticorps monoclonal, un anticorps chimérique, une seule chaîne Fv (scFv), un anticorps à une seule chaîne, un fragment Fab (Fab) ou un F(ab')₂.

9. Anticorps selon la revendication 7, où l'anticorps est un anticorps monoclonal, un anticorps humanisé, un anticorps chimérique, une seule chaîne Fv (scFv), un anticorps à une seule chaîne, un fragment Fab (Fab) ou un F(ab')₂.

10. Conjugué d'anticorps comprenant l'anticorps selon l'une quelconque des revendications précédentes conjugué ou fusionné à un polypeptide hétérologue.

11. Conjugué d'anticorps comprenant l'anticorps selon l'une quelconque des revendications 1 à 9 conjugué ou fusionné à un agent diagnostique ou détectable.

12. Conjugué d'anticorps comprenant l'anticorps selon l'une quelconque des revendications 1 à 9 conjugué ou fusionné à un agent thérapeutique.

13. Conjugué d'anticorps selon la revendication 12, dans lequel l'agent thérapeutique est une cytotoxine.

14. Conjugué d'anticorps selon la revendication 13, dans lequel la cytotoxine est le paclitaxel, la chytochalasine B, la gramicidine D, le bromure d'éthidium, l'émétine, la mitomycine, l'étoposide, le ténoposide, la vincristine, la vinblastine, la colchicine, la doxorubicine, la daunorubicine, la dihydroxyanthracinedione, la mitoxantrone, la mithramycine, l'actinomycine D, la 1-déshydrotestostérone, des glucocorticoïdes, la procaïne, la tétracaïne, la lidocaïne, le propanolol, la puromycine, l'épirubicine ou le cyclophosphamide.

15. Conjugué d'anticorps selon la revendication 12, dans lequel l'agent thérapeutique est l'abrine, le ricin A, l'exotoxine de Pseudomonas, la toxine de choléra, la toxine de diphtérie, le facteur de nécrose tumorale alpha, le facteur de nécrose tumorale bêta, l'interféron alpha, l'interféron bêta ou le facteur de croissance dérivé des plaquettes.

16. Conjugué d'anticorps selon la revendication 12, dans lequel l'agent thérapeutique est un matériau radioactif ou un chélateur macrocyclique.

17. Acide nucléique isolé comprenant une séquence de nucléotides codant pour l'anticorps selon la revendication 2, 3 ou 6.

18. Vecteur comprenant l'acide nucléique selon la revendication 17.

19. Vecteur selon la revendication 18 comprenant en outre une séquence de nucléotides qui régule l'expression de la séquence de nucléotides.

20. Cellule hôte fabriquée génétiquement pour contenir ou exprimer l'acide nucléique selon la revendication 17.

21. Cellule hôte fabriquée génétiquement pour contenir le vecteur selon la revendication 18 ou 19.

22. Composition pharmaceutique comprenant l'anticorps selon l'une quelconque des revendications 1 à 9 et un support pharmaceutiquement acceptable.

23. Utilisation de la composition pharmaceutique selon la revendication 22 dans la fabrication d'un médicament pour le traitement d'un cancer, dans laquelle le cancer est associé à la surexpression de EphA2.

24. Composition pharmaceutique selon la revendication 22, pour une utilisation dans le traitement d'un cancer, dans laquelle le cancer est associé à la surexpression de EphA2.

25. Composition pharmaceutique comprenant le conjugué d'anticorps selon l'une quelconque des revendications 10 à 16 et un support pharmaceutiquement acceptable.

26. Utilisation de la composition pharmaceutique selon la revendication 24 dans la fabrication d'un médicament pour le traitement d'un cancer, dans laquelle le cancer est associé à la surexpression de EphA2.

27. Composition pharmaceutique selon la revendication 24, pour une utilisation dans le traitement d'un cancer, dans laquelle le cancer est associé à la surexpression de EphA2.

28. Anticorps selon l'une quelconque des revendications 1 à 9 pour une utilisation dans le traitement d'un cancer, dans laquelle le cancer est associé à la surexpression de EphA2.

29. Utilisation de l'anticorps selon l'une quelconque des revendications 1 à 9 dans la préparation d'un médicament pour le traitement d'un cancer, dans laquelle le cancer est associé à la surexpression de EphA2.

30. Conjugué d'anticorps selon l'une quelconque des revendications 10 à 16 pour une utilisation dans le traitement d'un cancer chez un patient, dans laquelle le cancer est associé à la surexpression de EphA2.

31. Utilisation du conjugué d'anticorps selon l'une quelconque des revendications 10 à 16 dans la préparation d'un médicament pour le traitement d'un cancer chez un patient, dans laquelle le cancer est associé à la surexpression de EphA2.

32. Utilisation selon la revendication 29 ou 31, dans laquelle le médicament comprend en outre une thérapie supplémentaire.

33. Méthode pour le diagnostic d'un cancer chez un patient, comprenant:
a. la mise en contact de cellules provenant dudit patient avec l'anticorps selon l'une quelconque des revendications 1 à 9 dans des conditions appropriées pour une liaison anticorps-EphA2; et
b. la mesure de la liaison de l'anticorps contre EphA2 avec lesdites cellules, dans laquelle un niveau de liaison d'anticorps contre EphA2 supérieur à un témoin indique que le patient a un cancer.

34. Utilisation de l'anticorps selon l'une quelconque des revendications 1 à 9 dans la préparation d'un médicament pour le diagnostic, le pronostic ou la surveillance de l'efficacité d'une thérapie pour un cancer chez un patient, dans laquelle le cancer est associé à la surexpression de EphA2.

35. Utilisation du conjugué d'anticorps selon l'une quelconque des revendications 10 à 16 dans la préparation d'un médicament pour le diagnostic, le pronostic ou la surveillance de l'efficacité d'une thérapie pour un cancer chez un patient, dans laquelle le cancer est associé à la surexpression de EphA2.

36. Utilisation selon la revendication 23, 29 ou 34 ou composition selon la revendication 24, dans laquelle le cancer est d'une origine épithéliale.

37. Utilisation selon la revendication 26, 31 ou 35 ou composition selon la revendication 27, dans laquelle le cancer est d'une origine épithéliale.

38. Utilisation selon la revendication 23, 29 ou 34 ou composition selon la revendication 24, dans laquelle le cancer est un carcinome des cellules rénales, un mélanome, un cancer métastatique ou un cancer de la peau, du poumon, du côlon, du sein, de la prostate, de la vessie, du rein ou du pancréas.

39. Utilisation selon la revendication 26, 31 ou 35 ou composition selon la revendication 27, dans laquelle le cancer est un carcinome des cellules rénales, un mélanome, un cancer métastatique ou un cancer de la peau, du poumon, du côlon, du sein, de la prostate, de la vessie, du rein ou du pancréas.

40. Utilisation selon la revendication 23, 26, 29, 31, 34 ou 35, dans laquelle le patient est un humain.

41. Composition selon les revendications 24 et 27, dans laquelle le patient est un humain.

42. Utilisation selon la revendication 40, dans laquelle l'humain est réfractaire à une thérapie précédente.

43. Composition selon la revendication 41, dans laquelle l'humain est réfractaire à une thérapie précédente.

44. Méthode selon la revendication 33, dans laquelle le cancer est d'une origine épithéliale.

45. Méthode selon la revendication 33, dans laquelle le cancer est un carcinome des cellules rénales, un mélanome, un cancer métastatique ou un cancer de la peau, du poumon, du côlon, du sein, de la prostate, de la vessie, du rein ou du pancréas.

46. Méthode selon la revendication 33, dans laquelle les cellules proviennent de sang entier, d'expectoration, d'urine, de sérum ou d'aspirations à l'aiguille fine d'un tissu de cellules tumorales.

47. Méthode selon la revendication 33, dans laquelle le patient est un humain.

48. Hybridome déposé à l'ATCC et avec le no. d'entrée attribué PTA-4380.

49. Hybridome déposé à l'ATCC et avec le no. d'entrée attribué PTA-4381.

50. Méthode pour la production d'un anticorps, comprenant la culture de la cellule hôte selon l'une quelconque des revendications 20 et 21 dans des conditions dans lesquelles l'acide nucléique est exprimé.

51. Kit comprenant l'anticorps selon l'une quelconque des revendications 1 à 9, dans un récipient, et des instructions pour l'utilisation.

52. Kit comprenant le conjugué d'anticorps selon l'une quelconque des revendications 11 à 16, dans un récipient, et des instructions pour l'utilisation.

53. Anticorps selon la revendication 7, où l'anticorps est un anticorps humanisé ou un anticorps chimérique.


### SEQUENCE LISTING

<110> Purdue Research Foundation Kinch, Michael Carles-Kinch, Kelly
<120> EphA2 Agonistic Monoclonal Antibodies and Methods of Use Thereof
<130> 10271-107-228
<140>
   <141>
<150> 60/379,368
   <151> 2002-05-10
<150> 60/418,204
   <151> 2002-10-14
<150> 60/460,358
   <151> 2003-04-03
<160> 16
<170> PatentIn version 3.2
<210> 1
   <211> 107
   <212> PRT
   <213> Homo Sapiens
<400> 1
   Asp Ile Lys Met Thr Gln Ser Pro Ser Ser Met Tyr Ala Ser Leu Gly
   1 5 10 15
   Glu Arg Val Thr Ile Thr Cys Lys Ala Ser Gln Asp Ile Asn Asn Tyr
   20 25 30
   Leu Ser Trp Phe Gln Gln Lys Pro Gly Lys Ser Pro Lys Thr Leu Ile
   35 40 45
   Tyr Arg Ala Asn Arg Leu Val Asp Gly Val Pro Ser Arg Phe Ser Gly
   50 55 60
   Ser Gly Ser Gly Gln Asp Tyr Ser Leu Thr Ile Ser Ser Leu Glu Tyr
   65 70 75 80
   Glu Asp Met Gly Ile Tyr Tyr Cys Leu Lys Tyr Asp Glu Phe Pro Tyr
   85 90 95
   Thr Phe Gly Gly Gly Thr Lys Leu Glu Ile Lys
   100 105
<210> 2
   <211> 11
   <212> PRT
   <213> Homo Sapiens
<400> 2
   Lys Ala Ser Gln Asp Ile Asn Asn Tyr Leu Ser
   1 5 10
<210> 3
   <211> 7
   <212> PRT
   <213> Homo Sapiens
<400> 3
   Arg Ala Asn Arg Leu Val Asp
   1 5
<210> 4
   <211> 9
   <212> PRT
   <213> Homo Sapiens
<400> 4
   Leu Lys Tyr Asp Glu Phe Pro Tyr Thr
   1               5
<210> 5
   <211> 115
   <212> PRT
   <213> Homo Sapiens
<400> 5
   Asp Val Lys Leu Val Glu Ser Gly Gly Gly Leu Val Lys Pro Gly Gly
   1               5                   10                  15
   Ser Leu Lys Leu Ser Cys Ala Ala Ser Gly Phe Thr Phe Ser Ser Tyr
               20                  25                  30
   Thr Met Ser Trp Val Arg Gln Thr Pro Glu Lys Arg Leu Glu Trp Val
           35                  40                  45
   Ala Thr Ile Ser Ser Gly Gly Thr Tyr Thr Tyr Tyr Pro Asp Ser Val
       50                  55                  60
   Lys Gly Arg Phe Thr Ile Ser Arg Asp Asn Ala Lys Asn Thr Leu Tyr
   65                  70                  75                  80
   Leu Gln Met Ser Ser Leu Lys Ser Glu Asp Thr Ala Met Tyr Tyr Cys
   Thr Arg Glu Ala Ile Phe Thr Tyr Trp Gly Gln Gly Thr Leu Val Thr
               100                 105                 110
   Val Ser Ala
           115
<210> 6
   <211> 10
   <212> PRT
   <213> Homo Sapiens
<400> 6
   Gly Phe Thr Phe Ser Ser Tyr Thr Met Ser
   1               5                   10
<210> 7
   <211> 17
   <212> PRT
   <213> Homo Sapiens
<400> 7
   Thr Ile Ser Ser Gly Gly Thr Tyr Thr Tyr Tyr Pro Asp Ser Val Lys
   1               5                   10                  15
   Gly
<210> 8
   <211> 6
   <212> PRT
   <213> Homo Sapiens
<400> 8
   Glu Ala Ile Phe Thr Tyr
   1               5
<210> 9
   <211> 321
   <212> DNA
   <213> Homo sapiens
<400> 9
   gacatcaaga tgacccagtc tccatcttcc atgtatgcat ctctaggaga gagagtcact 60
   atcacttgca aggcgagtca ggacattaat aactatttaa gctggttcca gcagaaacca 120
   gggaaatctc ctaagaccct gatctatcgt gcaaacagat tggtagatgg ggtcccatca 180
   aggttcagtg gcagtggatc tgggcaagat tattctctca ccatcagcag cctggagtat 240
   gaagatatgg gaatttatta ttgtctgaaa tatgatgagt ttccgtacac gttcggaggg 300
   gggaccaagc tggaaataaa a 321
<210> 10
   <211> 30
   <212> DNA
   <213> Homo sapiens
<400> 10
   gcgagtcagg acattaataa ctatttaagc 30
<210> 11
   <211> 21
   <212> DNA
   <213> Homo sapiens
<400> 11
   cgtgcaaaca gattggtaga t 21
<210> 12
   <211> 21
   <212> DNA
   <213> Homo sapiens
<400> 12
   aaatatgatg agtttccgta c 21
<210> 13
   <211> 345
   <212> DNA
   <213> Homo sapiens
<400> 13
   gacgtgaagc tggtggagtc tgggggaggc ttagtgaagc ctggagggtc cctgaaactc 60
   tcctgtgcag cctctggatt cactttcagt agctatacca tgtcttgggt tcgccagact 120
   ccggagaaga ggctggagtg ggtcgcaacc attagtagtg gtggtactta cacctactat 180
   ccagacagtg tgaagggccg attcaccatc tccagagaca atgccaagaa caccctgtac 240
   ctgcaaatga gcagtctgaa gtctgaggac acagccatgt attactgtac aagagaagct 300
   atctttactt actggggcca agggactctg gtcactgtct ctgca 345
<210> 14
   <211> 30
   <212> DNA
   <213> Homo sapiens
<400> 14
   ggattcactt tcagtagcta taccatgtct 30
<210> 15
   <211> 51
   <212> DNA
   <213> Homo sapiens
<400> 15
   accattagta gtggtggtac ttacacctac tatccagaca gtgtgaaggg c 51
<210> 16
   <211> 21
   <212> DNA
   <213> Homo sapiens
<400> 16
   agagaagcta tctttactta c 21

<110> Purdue Research Foundation Kinch, Michael Carles-Kinch, Kelly
<120> EphA2 Agonistic Monoclonal Antibodies and Methods of use Thereof
<130> 10271-107-228
<140>
   <141>
<150> 60/379,368
   <151> 2002-05-10
<150> 60/418,204
   <151> 2002-10-14
<150> 60/460,358
   <151> 2003-04-03
<160> 16
<170> PatentIn version 3.2
<210> 1
   <211> 107
   <212> PRT
   <213> Homo Sapiens
<400> 1
   Asp Ile Lys Met Thr Gln Ser Pro Ser Ser Met Tyr Ala Ser Leu Gly
   1               5                   10                  15
   Glu Arg Val Thr Ile Thr Cys Lys Ala Ser Gln Asp Ile Asn Asn Tyr
               20                  25                  30
   Leu Ser Trp Phe Gln Gln Lys Pro Gly Lys Ser Pro Lys Thr Leu Ile
           35                  40                  45
   Tyr Arg Ala Asn Arg Leu Val Asp Gly Val Pro Ser Arg Phe Ser Gly
       50                  55                  60
   Ser Gly Ser Gly Gln Asp Tyr Ser Leu Thr Ile Ser Ser Leu Glu Tyr
   65                  70                  75                  80
   Glu Asp Met Gly Ile Tyr Tyr Cys Leu Lys Tyr Asp Glu Phe Pro Tyr
                   85                  90                  95
   Thr Phe Gly Gly Gly Thr Lys Leu Glu Ile Lys
               100                 105
<210> 2
   <211> 11
   <212> PRT
   <213> Homo Sapiens
<400> 2
   Lys Ala Ser Gln Asp Ile Asn Asn Tyr Leu Ser
   1 5 10
<210> 3
   <211> 7
   <212> PRT
   <213> Homo Sapiens
<400> 3
   Arg Ala Asn Arg Leu Val Asp
   1 5
<210> 4
   <211> 9
   <212> PRT
   <213> Homo Sapiens
<400> 4
   Leu Lys Tyr Asp Glu Phe Pro Tyr Thr
   1 5
<210> 5
   <211> 115
   <212> PRT
   <213> Homo Sapiens
<400> 5
   Asp Val Lys Leu Val Glu Ser Gly Gly Gly Leu Val Lys Pro Gly Gly
   1 5 10 15
   Ser Leu Lys Leu Ser Cys Ala Ala Ser Gly Phe Thr Phe Ser Ser Tyr
   20 25 30
   Thr Met Ser Trp Val Arg Gln Thr Pro Glu Lys Arg Leu Glu Trp Val
   35 40 45
   Ala Thr Ile Ser Ser Gly Gly Thr Tyr Thr Tyr Tyr Pro Asp Ser Val
   50 55 60
   Lys Gly Arg Phe Thr Ile Ser Arg Asp Asn Ala Lys Asn Thr Leu Tyr
   65 70 75 80
   Leu Gln Met Ser Ser Leu Lys Ser Glu Asp Thr Ala Met Tyr Tyr Cys
   85 90 95
   Thr Arg Glu Ala Ile Phe Thr Tyr Trp Gly Gln Gly Thr Leu Val Thr
   100 105 110
   Val Ser Ala
   115
<210> 6
   <211> 10
   <212> PRT
   <213> Homo Sapiens
<400> 6
   Gly Phe Thr Phe Ser Ser Tyr Thr Met Ser
   1 5 10
<210> 7
   <211> 17
   <212> PRT
   <213> Homo Sapiens
<400> 7
   Thr Ile Ser Ser Gly Gly Thr Tyr Thr Tyr Tyr Pro Asp Ser Val Lys
   Gly
<210> 8
   <211> 6
   <212> PRT
   <213> Homo Sapiens
<400> 8
   Glu Ala Ile Phe Thr Tyr
   1 5
<210> 9
   <211> 321
   <212> DNA
   <213> Homo sapiens
<400> 9
   gacatcaaga tgacccagtc tccatcttcc atgtatgcat ctctaggaga gagagtcact 60
   atcacttgca aggcgagtca ggacattaat aactatttaa gctggttcca gcagaaacca 120
   gggaaatctc ctaagaccct gatctatcgt gcaaacagat tggtagatgg ggtcccatca 180
   aggttcagtg gcagtggatc tgggcaagat tattctctca ccatcagcag cctggagtat 240
   gaagatatgg gaatttatta ttgtctgaaa tatgatgagt ttccgtacac gttcggaggg 300
   gggaccaagc tggaaataaa a 321
<210> 10
   <211> 30
   <212> DNA
   <213> Homo sapiens
<400> 10
   gcgagtcagg acattaataa ctatttaagc 30
<210> 11
   <211> 21
   <212> DNA
   <213> Homo sapiens
<400> 11
   cgtgcaaaca gattggtaga t 21
<210> 12
   <211> 21
   <212> DNA
   <213> Homo sapiens
<400> 12
   aaatatgatg agtttccgta c 21
<210> 13
   <211> 345
   <212> DNA
   <213> Homo sapiens
<400> 13
   gacgtgaagc tggtggagtc tgggggaggc ttagtgaagc ctggagggtc cctgaaactc 60
   tcctgtgcag cctctggatt cactttcagt agctatacca tgtcttgggt tcgccagact 120
   ccggagaaga ggctggagtg ggtcgcaacc attagtagtg gtggtactta cacctactat 180
   ccagacagtg tgaagggccg attcaccatc tccagagaca atgccaagaa caccctgtac    240
   ctgcaaatga gcagtctgaa gtctgaggac acagccatgt attactgtac aagagaagct    300
   atctttactt actggggcca agggactctg gtcactgtct ctgca                    345
<210> 14
   <211> 30
   <212> DNA
   <213> Homo sapiens
<400> 14
   ggattcactt tcagtagcta taccatgtct 30
<210> 15
   <211> 51
   <212> DNA
   <213> Homo sapiens
<400> 15
   accattagta gtggtggtac ttacacctac tatccagaca gtgtgaaggg c 51
<210> 16
   <211> 21
   <212> DNA
   <213> Homo sapiens
<400> 16
   agagaagcta tctttactta c 21